# EUROPÄISCHE PATENTANMELDUNG

(11) **EP 0 273 242 A2**
(43) Veröffentlichungstag der Anmeldung: **06.07.1988**
(21) Anmeldenummer: 87118111.1
(22) Anmeldetag: 08.12.1987
(51) Int. Cl.: C07D 239/38, C07D 213/70, A01N 43/40, A01N 43/54, C07F 7/18, C07F 7/08, C07C 149/28

(54) **2-Pyri(mi)dylthio-aryläther**

(30) Priorität: 22.12.1986 DE 3643851
(71) Anmelder: BAYER AG, 51368 Leverkusen (DE)
(72) Erfinder: Sasse, Klaus, Dr., D-5060 Bergisch-Gladbach 2 (DE); Fischer, Reiner, Dr., D-4019 Monheim (DE); Hagemann, Hermann, Dr., D-5090 Leverkusen 1 (DE); Krebs, Andreas, Dr., D-5068 Odenthal (DE); Santel, Hans-Joachim, Dr., D-5090 Leverkusen 1 (DE); Schmidt, Robert R., Dr., D-5060 Bergisch Gladbach 2 (DE); Lürssen, Klaus, Dr., D-5060 Bergisch Gladbach 2 (DE); Strang, Harry, Dr., D-4000 Düsseldorf 31 (DE)

(57) **Zusammenfassung**

Die vorliegende Erfindung betrifft neue 2-Pyri(mi)dylthio-arylether der Formel (I) 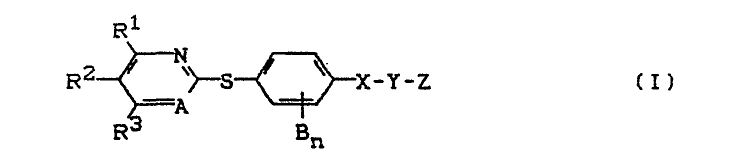 in welcher
R¹, R² und R³ unabhängig voneinander jeweils für Wasserstoff oder Alkyl stehen,
A für eine -CH-Gruppe oder ein Stickstoffatom steht,
X für Sauerstoff, Schwefel, Sulfoxy oder Sulfonyl steht,
Y für eine 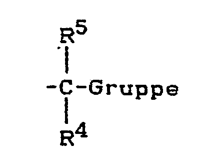 oder eine 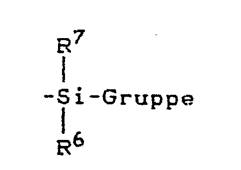 steht,
   und
Z für einen Rest 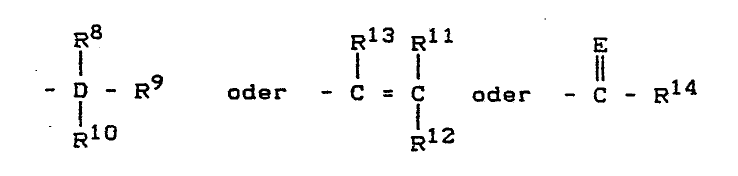
steht, wobei die restlichen Substituenten die in der Beschreibung angegebene Bedeutung haben, mehrere Verfahren sowie neue Zwischenprodukte zu ihrer Herstellung und ihre Verwendung als Herbizide und Pflanzenwachstumsregulatoren.

## Beschreibung

Die vorliegende Erfindung betrifft neue 2-Pyri(mi)dyithioaryiether, meherere Verfahren sowie neue Zwischenprodukte zu ihrer Herstellung und ihre Verwendung als Herbizide und Pflanzenwachstumsregulatoren.

Es ist bereits bekannt, daß bestimmte 2-Phenoxypyrimidine herbizide Eigenschaften besitzen (vgl. EP 1187 und DE-OS 2 820 032). Die Wirkung dieser Verbindung ist gut, jedoch werden bei geringen Aufwandmengen einige Unkräuter nicht immer voll erfaßt, auch ist die Selektivität nicht immer ausreichend.

Ferner ist bekannt, daß zahlreiche Pyrimidyl-2-ether und -thioether als Herbizide geeignet sind (vgl. z.B. JP 9474/1967 (Anmeldenummer 12771/65), US-PS 3 126 271, US-PS 3 250 775 und EP-A 218 321). Die herbizide Wirksamkeit dieser vorbekannten Verbindungen gegenüber Schadpflanzen ist jedoch ebenso wie ihre Verträglichkeit gegenüber wichtigen Kulturpflanzen nich immer in allen Anwendungsgebieten zufriedenstellend.

Es wurden nun neue 2-Pyri(mi)dylthio-arylether der Formel (I) gefunden, 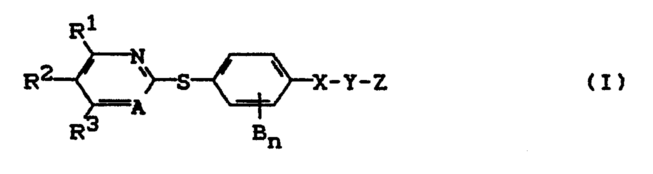 in welcher
R¹, R² und R³ unabhängig voneinander jeweils für Wasserstoff oder Alkyl stehen,
A für eine -CH-Gruppe oder ein Stickstoffatom steht,
B für Halogen, Nitro, Cyano, Amino, Alkyl, Alkoxy, Halogenalkyl oder Halogenalkoxy steht, wobei B gleich oder verschieden sein kann,
n für eine Zahl 0, 1, 2, 3 oder 4 steht,
X für Sauerstoff, Schwefel, Sulfoxy oder Sulfonyl steht,
Y für eine 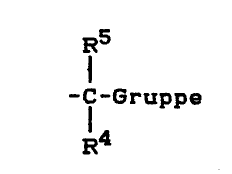
oder eine 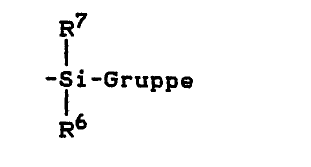
steht,
wobei
R⁴ und R⁵ unabhängig voneinander jeweils für Wasserstoff, Alkyl, Alkoxyalkyl oder Halogenalkyl steht,
R⁶ und R⁷ jeweils für Alkyl stehen,
Z für einen Rest 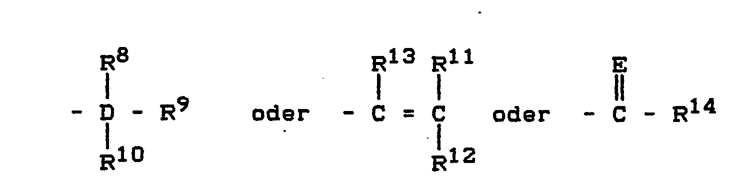 steht, wobei
D für Kohlenstoff oder Silicium steht,
R⁸, R¹³ und R¹⁴ unabhängig voneinander für Wasserstoff, Halogen, Alkyl oder Halogenalkyl stehen,
R⁹ für Wasserstoff, Halogen, Alkyl, Alkoxy, Alkoxyalkyl, Alkylthio oder Halogenalkyl steht,
R¹⁰ für Wasserstoff, Halogen, Hydroxy, Alkyl, Alkoxy, Alkoxyalkyl, Alkylthio, Alkylthioalkyl, Nitriloalkyl, Alkylcarbonyl, Alkoxycarbonyl, Aminocarbonyl, Formyl, Alkenyl, Alkinyl, Halogenalkyl, Halogenalkenyl, Halogenalkinyl, Amino, Alkylamino, Dialkylamino, durch Amino oder Alkylamino substituiertes Alkyl, gegebenenfalls durch Alkyl und/oder Halogen substi tuiertes Cycloalkyl, offenkettiges oder cyclisches Acetal oder offenkettiges oder cyclisches Thioacetal steht oder
R⁹ und R¹⁰ gemeinsam mit dem Kohlenstoffatom, an das sie gebunden sind, einen 3-8 gliedrigen Ring bilden, der außer Kohlenstoff auch Sauerstoff und/oder Schwefel und/oder Stickstoff als Ringglieder und welcher eine Oxo-Gruppe enthalten kann und der durch Halogen, Alkyl, Alkoxy und/oder Alkoxyalkyl substituiert sein kann und welcher eine oder mehrere Doppelbindungen enthalten kann oder
R⁸, R⁹ und R¹⁰ gemeinsam mit dem Kohlenstoffatom, an das sie gebunden sind, für einen gesättigten oder ungesättigten Rest der Struktur 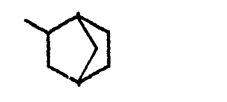 stehen,
E für Sauerstoff oder Schwefel steht und
R¹¹ und R¹² unabhängig voneinander für Wasserstoff, Halogen, Alkyl oder Halogenalkyl stehen,
mit der Maßgabe daß, wenn R¹ und R³ für Methyl, R² für Wasserstoff, A für Stickstoff, X für Sauerstoff und n für O stehen, Y und Z zusammen nicht für die Reste:
-C₂H₅, n-C₃H₇, iso-C₃H₇, n-C₄H₉, iso-C₄H₉, n-C₅H₁₁, 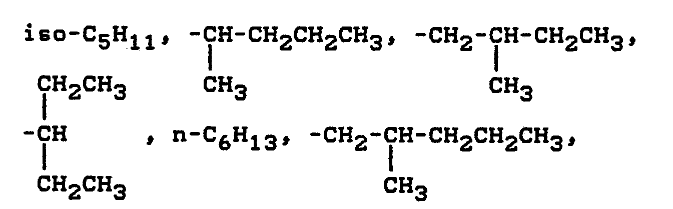 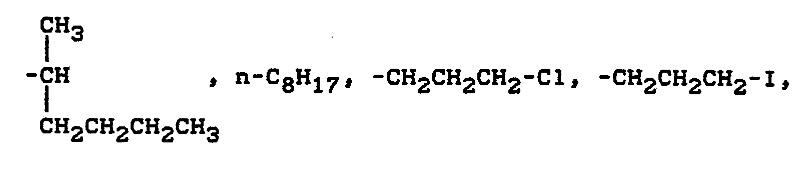 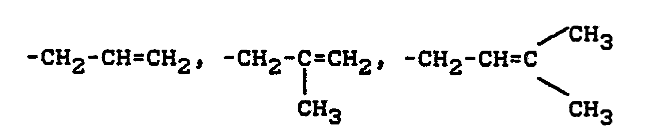 und
-(CH₂)₄-CH₌CH₂ stehen.

Enthalten Verbindungen der Formel (I) ein asymmetrisches Kohlenstoffatom, so können sie auch in verschiedenen enantiomeren Formen vorliegen. Die Erfindung betrifft sowohl die möglichen einzelnen Isomeren als auch Gemische dieser Isomeren.

Ein asymmetrisches Zentrum liegt vor, wenn Y für die 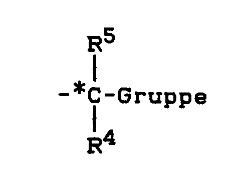 steht und R⁴ und R⁵ unterschiedliche Bedeutung haben, wobei das asymmetrische C-Atom, durch * gekennzeichnet, die R, S oder R/S (Racemat) Konfiguration aufweisen kann.

Das zweite asymmetrische Zentrum liegt vor, wenn Z für 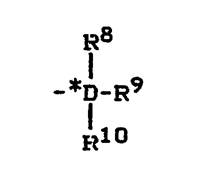 und D für Kohlenstoff steht, und R⁸, R⁹ und R¹⁰ unterschiedliche Bedeutung haben, wobei das 2. asymmetrische C-Atom die R, S oder R/S (Racemat)-Konfiguration aufweisen kann.

Für den Fall, daß R⁸, R⁹ und R¹⁰ gemeinsam mit dem Kohlenstoffatom, an das sie gebunden sind, für einen gesättigten oder ungesättigten Rest der Struktur 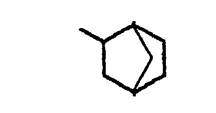 stehen, können die Verbindungen der Formel (I) in verschiedenen geometrischen Formen (cis/trans-Isomerie) vorliegen. Die Erfindung betrifft sowohl die möglichen einzelnen Isomeren als auch Gemische dieser Isomere.

Weiterhin wurde gefunden, daß man 2-Pyri(mi)dyl-thioarylether der Formel (I) erhält, wenn man A) Thiophenol-Derivate der Formel (II), 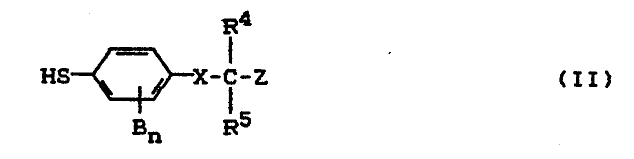 in welcher
R⁴, R⁵, B, X, Z und n die oben angegebene Bedeutung haben,
mit Pyri(mi)dyl-Derivaten der Formel (III), 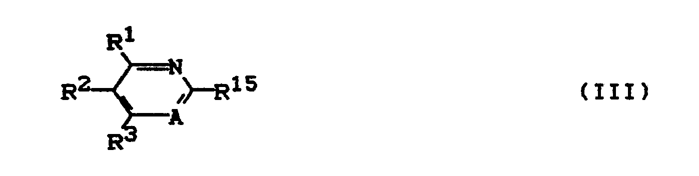 in welcher
R¹, R², R³ und A die oben angegebene Bedeutung haben und
R¹⁵ für Halogen, C₁-C₄-Alkylsulfonyl, Phenylsulfonyl oder substituiertes Phenylsulfonyl steht,
in Gegenwart eines Verdünnungsmittels und gegebenenfalls in Gegenwart eines Säurebindemittels umsetzt, oder wenn man

### B) Pyri(mi)dylthio-(thio)phenole der Formel (IV),

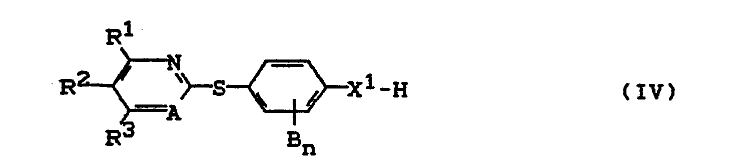

in welcher
A, B, R¹, R², R³ und n die oben angegebene Bedeutung haben und
X¹ für Sauerstoff oder Schwefel steht,
mit Verbindungen der Formel (V),
G-Y-Z (V)
in welcher
Y und Z die oben angegebene Bedeutung haben und
G für Halogen, C₁-C₄-Alkylsulfonyloxy, Halogen-C₁-C₄-alkylsulfonyloxy, Phenylsulfonyloxy oder substituiertes Phenylsulfonyloxy steht,
in Gegenwart eines Verdünnungsmittels und gegebenenfalls in Gegenwart eines Säurebindemittels umsetzt, oder wenn man
C) Verbindungen der Formel (VI) 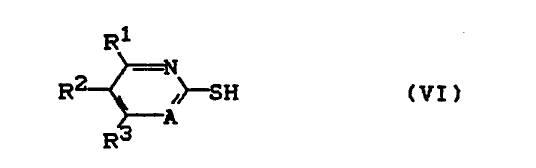 in welcher
R¹, R², R³ und A die oben angegebene Bedeutung haben,
mit Verbindungen der Formel (VIII) 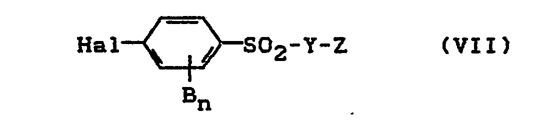 in welcher
B, Y, Z und n die oben angegebene Bedeutung haben,
Hai für Halogen steht,
in Gegenwart eines Verdünnungsmittels und gegebenenfalls in Gegenwart eines Säurebindemittels umsetzt.

Schließlich wurde gefunden, daß sich die neuen 2-Pyri(mi)dylthio-arylether der Formel (I) durch eine sehr gute herbizide Wirksamkeit auszeichnen. Darüberhinaus besitzen die erfindungsgemäßen Verbindungen der Formel (I) eine gute wuchsregulierende Wirkung.

Überraschenderweise besitzen die erfindungsgemäßen 2-Pyri(mi)dylthio-arylether der Formel (I) wesentlich bessere herbizide Eigenschaften als konstitutionell ähnliche vorbekannte Stoffe.

Die Kohlenstoffkette ist in den Definitionen jeweils geradkettig oder verzweigt. Halogen steht jeweils für Fluor, Chlor, Brom oder lod.

Die erfindungsgemäßen 2-Pyri(mi)dylthio-arylether sind durch die Formel (I) allgemein definiert. Bevorzugt sind Verbindungen der Formel (I), bei welchen
R¹, R² und R³ unabhängig voneinander jeweils für Wasserstoff oder C₁-C₄-Alkyl stehen,
A für eine -CH-Gruppe oder ein Stickstoffatom steht,
B für Halogen, Nitro, Cyano, Amino, Ci-C₄-Alkyl, C₁-C₄-Alkoxy, Halogen-C₁-C₄-alkyl oder Halogen-C₁-C₄ alkoxy steht, wobei B gleich oder verschieden sein kann,
n für eine Zahl 0, 1, 2, 3 oder 4 steht,
X für Sauerstoff, Schwefel, Sulfoxy oder Sulfonyl steht,
Y für eine 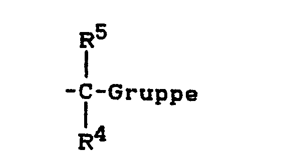 oder eine 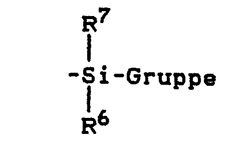 steht, wobei
R⁴ und R⁵ unabhängig voneinander jeweils für Wasserstoff, C₁-C₄-Alkyl; C₁-C₄-Alkoxy-C₁-C₄-alkyl oder Halogen-C₁-C₄-alkyl stehen und
R⁶ und R⁷ unabhängig voneinander jeweils für Methyl oder Ethyl stehen,
Z für eine Rest 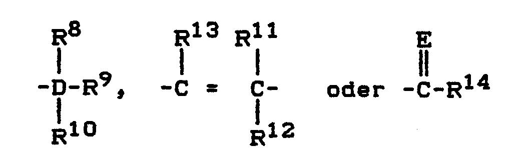 steht, wobei D für Kohlenstoff oder Silicium steht,
R⁸, R¹³ und R¹⁴ unabhängig voneinander für Wasserstoff, Halogen, C₁-C₆-Alkyl oder Halogen-C₁-C₆-alkyl stehen,
R⁹ für Wasserstoff, Halogen, C₁-C₆-Alkyl, C₁-C₆-Alkoxy, C₁-C₆-Alkoxy-C₁-C₆-alkyl, C₁-C₆-Alkylthio oder Halogen-C₁-C₆-alkyl steht,
R¹⁰ für Wasserstoff, Halogen, Hydroxy, C₁-C₆-Alkyl, C₁-C₆-Alkoxy, C₁-C₆-Alkoxy-C₁-C₆-alkyl, C₁-C₆-Alkyl-thio, C₁-C₆-Alkylthio-C₁-C₆-alkyl, Nitriol-C₁-C₆-alkyl, C₁-C₆-Alkylcarbonyl, C₁-C₆-Alkoxycarbonyl, Aminocarbonyl, Formyl, C₂-C₆-Alkenyl, C₂-C₆-Alkinyl, Halogen-C₁-C₆-alkyl, Halogen-C₂-C₆-alkenyl, Halogen-C₂-C₆-alkinyl, Amino, C₁-C₄-Alkylamino, Di-C₁-C₄-alkylamino, durch Amino oder C₁-C₄-Alkylamino substituiertes C₁-C₄-Alkyl, gegebenenfalls durch Methyl, Ethyl, und/oder Halogen substituiertes Cycloalkyl mit 3 bis 6 Kohlenstoffatomen, offenkettiges Acetal, cyclisches Acetal mit 5 ofer 6 Ringgliedem, offenkettiges Thioacetal oder cyclisches Thioacetal mit 5 oder 6 Ringgliedern steht oder
R⁹ und R¹⁰ gemeinsam mit dem Kohlenstoffatom an das sie gebunden sind, einen 5 oder 6-gliedrigen Ring bilden, der außer Kohlenstoff auch 1 oder 2 Sauerstoffatome und/oder Schwefelatome und/oder Stickstoffatome als Ringglieder und welcher eine Oxo-Gruppe enthalten kann und der durch Fluor, Chlor, Brom, lod, C₁-C₄-Alkyl, Ci-C₄-Alkoxy und/oder C₁-C₄-Alkoxy-C₁-C₄-alkyl substituiert sein kann und welcher eine oder mehrere, insbesondere eine bis zwei, Doppelbindungen enthalten kann oder
R⁸, R⁹ und R¹⁰ gemeinsam mit dem Kohlenstoffatom, an das sie gebunden sind, für einen gesättigten oder einfach ungesättigten Rest der Struktur 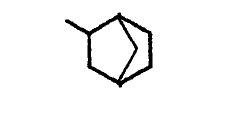
stehen,
E für Sauerstoff oder Schwefel steht und
R¹¹ und R¹² unabhängig voneinander für Wasserstoff, Halogen, C₁-C₆-Alkyl oder Halogen-C₁-C₆-alkyl stehen, mit der Maßgabe daß, wenn R¹ und R³ für Methyl, R² für Wasserstoff, A für Stickstoff, X für Sauerstoff und n für 0 stehen, Y und Z zusammen nicht für die Reste: 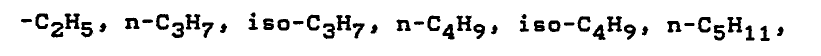 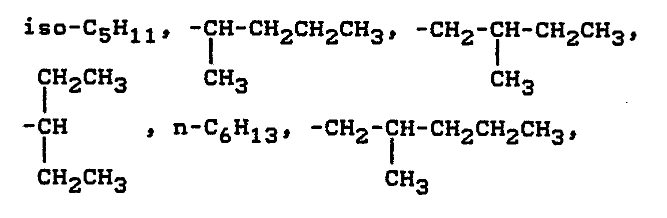 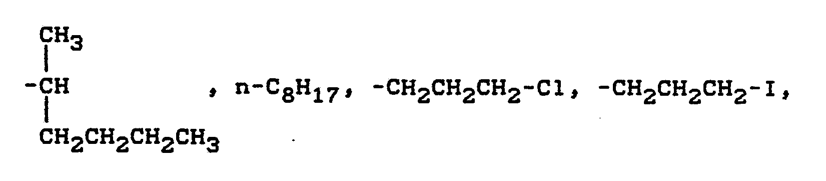 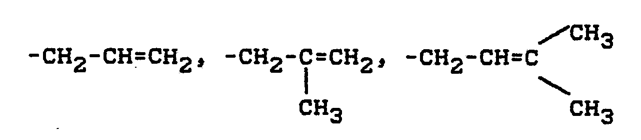 und 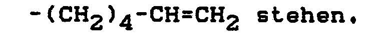 Bevorzugt sind auch die optisch aktiven Verbindungen der Formel (I). Besonders bevorzugt ist die Gruppe von Verbindungen der Formel (I), bei welchen
R¹, R² und R³ unabhängig voneinander jeweils für Wasserstoff, Methyl oder Ethyl stehen, wobei jedoch R¹ und R³ nicht gleichzeitig für Methyl stehen, wenn R² für Wasserstoff, A für Stickstoff, X für Sauerstoff und n für 0 stehen,
A für eine -CH-Gruppe oder ein Stickstoffatom steht,
B für Halogen, Nitro, Cyano, Amino, Methyl, Ethyl, Methoxy, Ethoxy, Halogen-C₁-C₂-alkyl oder Halogen-C₁-C₂-alkoxy steht, wobei B gleich oder verschieden sein kann,
n für eine Zahl 0, 1 oder 2 steht,
X für Sauerstoff, Schwefel, Sulfoxy oder Sulfonyl steht,
y für eine 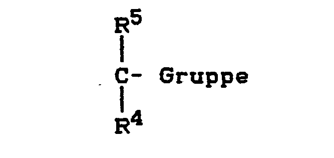 oder 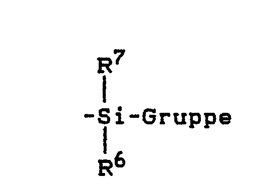 steht, wobei
R⁴ und R⁵ unabhängig voneinander jeweils für Wasserstoff, Methyl, Ethyl, Methoxymethyl, Methoxyethyl, Ethoxymethyl, Ethoxyethyl oder Halogen-C₁-C₂-alkyl stehen,
R⁶ und R⁷ jeweils für Methyl stehten,
Z für einen Rest 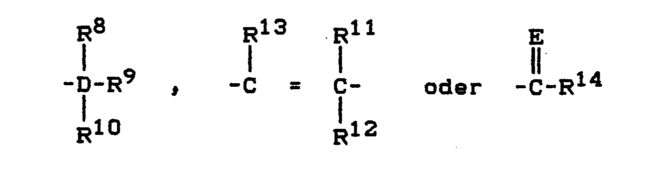 steht, wobei
D für Kohlenstoff oder Silicium steht,
R⁸, R¹³ und R¹⁴ unabhängig voneinander für Wasserstoff, Fluor, Chlor, Brom, lod, Cₗ-C₄-Alkyl oder Halogen-C₁-C₄-alkyl steht,
R⁹ für Wasserstoff, Fluor, Chlor, Brom, lod, C₁-C₄-Alkyl, C₁-C₄-Alkoxy, C₁-C₄-Alkoxy-C₁-C₄-alkyl, C₁-C₄-Alkylthio oder Halogen-Cₗ-C₄-alkyl steht,
R¹⁰ für Wassersoff, Fluor, Chlor, Brom, lod, Hydroxy, C₁-C₆-Alkyl, C₁-C₅-Alkoxy, C₁-C₄-Alkoxy-C₁-C₄-alkyl, C₁-C₄-Alkylthio-C₁-C₄-alkyl, C₁-C₄-Alkythio, Nitrilo-C₁-C₄-alkyl, C₁-C₄-Alkylcarbonyl, C₁-C₄-Alkoxycarbonyl, Aminocarbonyl, Formyl, C₂-C₅-Alkenyl, C₂-C₄-Alkinyl, Halogen-Ci-C₄-alkyl, Halogen-C₂-C₄-alkenyl, Halogen-C₂-C₄- alkinyl, Amino, C₁-C₂-Alkylamino, Di-C₁-C₂-alkylamino, jeweils gegebenenfalls durch Methyl, Fluor und/oder Chlor substituiertes Cyclopropyl, Cyclopentyl oder Cyclohexyl, offenkettiges Acetal, cyclisches Acetal mit 5 oder 6 Ringgliedern, offenkettiges Thioacetal oder cyclisches Thioacetal mit 5 oder 6 Ringgliedern steht oder
R⁹ und R¹⁰ gemeinsam mit dem Kohlenstoffatom, an das sie gebunden sind, einen 5 oder 6-gliedrigen Ring bilden, der außer Kohlenstoff auch 1 oder 2 Sauerstoffatome und/oder Schwefelatome und/oder Stickstoffatome als Ringglieder und der eine Oxo-Gruppe enthalten kann und der durch Fluor, Chlor, Brom, lod, Methyl, Ethyl, Methoxy, Ethoxy, Methoxymethyl, Methoxyethyl, Ethoxymethyl oder Ethoxyethyl substituiert sein kann und welcher eine oder zwei Doppelbindungen enthalten kann oder
R⁸, R⁹ und R¹⁰ gemeinsam mit dem Kohlenstoffatom, an das sie gebunden sind, für einen Rest der Struktur 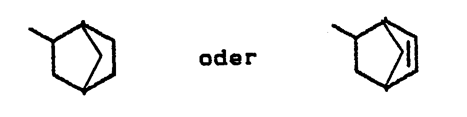 stehen,
E für Sauerstoff oder Schwefel steht und
R¹¹ und R¹² unabhängig voneinander für Wasserstoff, Halogen, C₁-C₂-Alkyl oder Halogen-C₁-C₂-alkyl stehen, sowie deren optisch aktiven Verbindungen.

Eine andere Gruppe von besonders bevorzugten Verbindungen der Formel (I) sind diejenigen, in welchen
R¹ und R³ für Methyl stehen,
R² für Wasserstoff steht,
A für Stickstoff steht,
n für 0 steht,
X für Sauerstoff steht,
Y für die Gruppe- 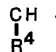 -steht, wobei
R⁴ für Wasserstoff, Methyl, Ethyl, n-oder iso-Propyl, n-, iso-, sec-oder tert.-Butyl, Methoxymethyl, Methoxyethyl, Ethoxymethyl oder Ethoxethyl steht,
Z für die Gruppe 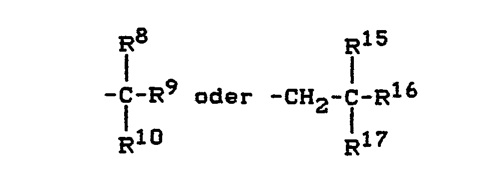 steht, wobei
R⁸ und R⁹ unabhängig voneinander für Methyl, Ethyl, n-oder iso-Propyl, n-, sec-oder tert.-Butyl stehen,
R¹⁰ für Methyl, Ethyl, n-oder iso-Propyl, n-, iso-, sec-oder tert.-Butyt, Methoxy, Ethoxy, n-oder iso-Propoxy, n-, iso-, sec-oder tert.-Butoxy, Halogen-C₁-C₄-alkyl, C₁-C₄-Alkoxy-C₁-C₄-alkyl oder C₁-C₄-Alkylthio-C₁-C₄-alkyl steht und
(A) R¹⁵, R¹⁶ und R¹⁷ unabhängig voneinander für Methyl, Ethyl, n-oder iso-Propyl, n-, iso-, sec-oder tert.-Butyl, Methoxy, Ethoxy, n-Propoxy oder iso-Propoxy stehen oder
(B) R¹⁵, R¹⁶ und R¹⁷ unabhängig voneinander für Wasserstoff, Methyl, Ethyl, n-oder iso-Propyl, n-, iso-, sec.-oder tert.-Butyl, Methoxy, Ethoxy, n-oder iso-Propoxy, n-, iso-, sec-oder tert.-Butoxy, Methylthio, Ethylthio, n-oder iso-Propylthio, n-, iso-, sec.-oder tert.-Butylthio stehen und
R¹⁷ für Methoxy, Ethoxy, n-oder iso-Propoxy, n-, iso-, sec-oder tert.-Butoxy, Methylthio, Ethylthio, n-oder iso-Propylthio, n-, iso-, sec-oder tert.-Butylthio steht oder
(C) R'⁵ für Wasserstoff, Methyl, Ethyl, n-oder iso-Propyl, n-, iso-, sec-oder tert.-Butyl, Methoxy, Ethoxy, n-oder iso-Propoxy, Methylthio, Ethylthio, n-oder iso-Propylthio steht und
R¹⁶ und R¹⁷ zusammen mit dem Kohlenstoffatom, an das sie gebunden sind, für jeweils gegebenenfalls durch Methyl, Fluor und/oder Chlor substituiertes Cyclopropyl, Cyclopentyl oder Cyclohexyl stehen.

Ganz besonders bevorzugt ist die Gruppe von Verbindungen der Formel (I), in denen
R¹, R² und R³ unabhängig voneinander jeweils für Wasserstoff, Methyl oder Ethyl stehen, wobei R¹ und R³ nicht gleichzeitig für Methyl stehen, wenn R² für Wasserstoff, A für Stickstoff, X für Sauerstoff und n für 0 stehen,
A für eine -CH-Gruppe oder ein Stickstoffatom steht,
B für Fluor, Chlor, Brom, Methyl, Ethyl, Methoxy, Ethoxy, Trifluormethyl oder Trifluormethoxy steht, wobei B gleich oder verschieden sein kann,
n für eine Zahl 0, 1 oder 2 steht,
X für Sauerstoff oder Schwefel steht,
Y für die 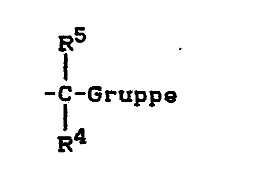 steht, wobei
R⁴ und R⁵ unabhängig voneinander jeweils für Wasserstoff, Methyl, Ethyl, Methoxymethyl, Methoxyethyl, Ethoxymethyl, Ethoxyethyl oder Halogen-C₁-C₂-alkyl stehen,
Z für einen Rest 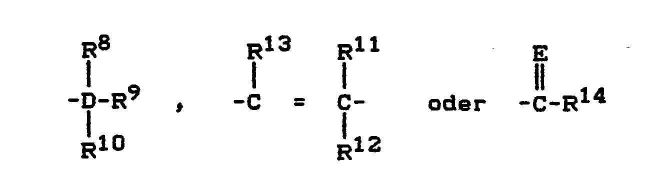 steht, wobei
D für Kohlenstoff oder Silicium steht,
R⁸, R¹³ und R¹⁴ unabhängig voneinander für Wasserstoff, Fluor, Chlor, Methyl, Ethyl, Ethyl, n-Propyl, iso-Propyl, n-, iso-, sec. oder tert. Butyl oder Halogen-C₁-C₂-alkyl steht,
R⁹ für Wasserstoff, Fluor, Chlor, Brom, lod, Methyl, Ethyl, n-Propyl, iso-Propyl, n-, iso-, sec.-oder tert.-Butyl, Methoxy, Ethoxy, n-Propoxy, iso-Propoxy, n-, iso-, sec.-oder tert. Butoxy, Methoxymethyl, Methoxyethyl, Methoxy-n-propyl, Methoxy-iso-propyl, Ethoxymethyl, Ethoxyethyl, Ethoxy-n-propyl, Ethoxy-iso-propyl, n-Propoxymethyl, n-Propoxyethyl, n-Propoxy-n-propyl, n-Propoxy-iso-propyl, Methylthio, Ethylthio, n-Propylthio, iso-Propylthio, oder Halogen-C₁-C₂-alkyl steht,
R¹⁰ für Wasserstoff, Fluor, Chlor, C₁-C₄-Alkyl, Ci-C₄-Alkoxy, C₁-C₄-Alkoxy-C₁-C₄-alkyl, Ci-C₄-Alkylthio, C₁-C₄-Alkylthio-Ci-C₄-alkyl, Nitrilo-C₁-C₄-alkyl, Ci-C₄-Alkylcarbonyl, Ci-C₄-Alkoxycarbonyl, Aminocarbonyl, Formyl, C₂-C₄-Alkenyl, C₂-C₄-Alkinyl, Halogen-C₁-C₄-alkyl, Halogen-C₂-C₄-alkenyl, Halogen-C₂-C₄-alkinyl, Amino, C₁-C₂-Alkylamino, Di-C₁-C₂alkylamino, offenkettiges Acetal, cyclisches Acetal mit 5 oder 6 Ringgliedern, offenkettiges Thioactal oder cyclisches Thioacetal mit 5 oder 6 Ringgliedern steht oder
R⁹ und R¹⁰ gemeinsam mit dem Kohlenstoff, an das sie gebunden sind, einen 5-oder 6-gliedrigen Ring bilden, der außer Kohlenstoff auch 1 oder 2 Sauerstoffatome und/oder Schwefelatome und/oder Stickstoffatome als Ringglieder enthalten kann und der durch Fluor, Chlor, Brom, Methyl, Ethyl, Methoxy, Ethoxy, Methoxymethyl, Ethoxymethyl oder Ethoxyethyl substituiert sein kann und welcher eine oder zwei Doppelbindungen enthalten kann oder
R⁸, R⁹ und R¹⁰ gemeinsam mit dem Kohlenstoffatom, an das sie gebunden sind, für einen Rest der Struktur 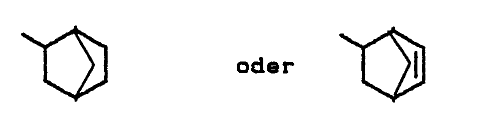 stehen,
E für Sauerstoff oder Schwefel steht und
R¹¹ und R¹² unabhängig voneinander für Wasserstoff, Halogen, C₁-C₂-Alkyl oder Halogen-C₁-C₂-alkyl stehen.

Eine andere ganz besonders bevorzugte Gruppe von Verbindungen der Formel (I) sind diejenigen, in welchen
R¹ und R³ für Methyl stehen,
R² für Wasserstoff steht,
A für Stickstoff steht,
n für 0 steht,
X für Sauerstoff steht,
Y für die Gruppe 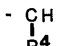 -steht, wobei
R⁴ für Wasserstoff, Methyl, Ethyl, Methoxymethyl oder Ethoxymethyl steht, Z für die Gruppe 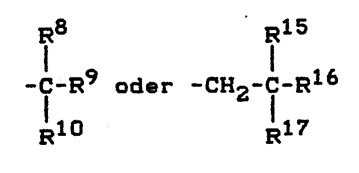 steht,
wobei
R⁸ und R⁹ für Methyl stehen,
R¹⁰ für Methyl, Ethyl, n-Propyl, iso-Propyl, Methoxy, Ethoxy, n-Propoxy, iso-Propoxy, 3-Methyl-1-butoxy, Methoxymethyl, Ethoxymethyl, iso-Propoxymethyl, Methylthiomethyl oder Ethylthiomethyl steht oder für Halogenmethyl oder Halogenethyl steht, wobei Halogen insbesondere für Fluor und/oder Chlor steht, und
(A) R¹⁵, R¹⁶ und R¹⁷ unabhängig voneinander für Methyl, Ethyl, n-oder iso-Propyl, Methoxy, Ethoxy, n-oder iso-Propoxy stehen,
(B) R¹⁵ und R¹⁶ unabhängig voneinander für Wasserstoff, Methyl, Methoxy, Ethoxy oder Methylthio steht und
R¹⁷ für Methoxy, Ethoxy oder Methylthio steht oder
(C) R¹⁵ für Wasserstoff, Methyl, Methoxy, Ethoxy oder Methylthio steht und
R¹⁵, R¹⁷ zusammen mit dem Kohlenstoffatom, an das sie gebunden sind, für jeweils gegebenenfalls durch Fluor und/oder Chlor substituiertes Cyclopropyl, Cyclopentyl oder Cyclohexyl stehen.

Verwendet man beispielsweise 4,6-Dimethyl-2-chlorpyrimidin und 4-(Neopentylthio)-thiophenol als Ausgangsstoffe, so läßt sich der Reaktionsablauf des erfindungsgemäßen Verfahrens (A) durch das folgende Formelschema darstellen:

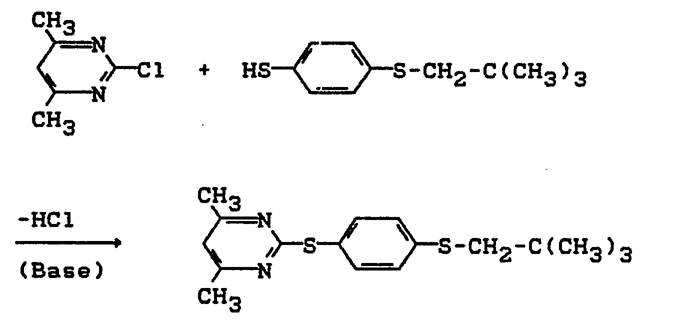

Verwendet man beispielsweise 4-(4,6-Dimethyl-2-pyrimidylthio)-phenol und Dimethyl-tert.-butylsilylch- lorid als Augsgangsstoffe, so läßt sich der Reaktionsablauf des erfindungsgemäßen Verfahrens (B) durch das folgende Formelschema darstellen:

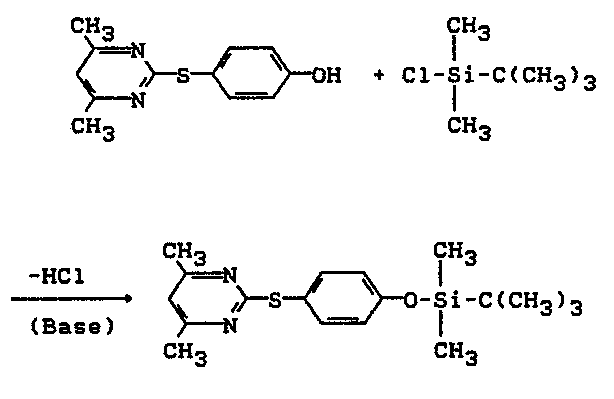

Verwendet man beispielsweise 4,6-Dimethyl-2-mercaptopyrimidinhydrochlorid und 4-(Neopentylsulfonyl)-chlorphenyl als Ausgangsstoffe, so läßt sich der Reaktionsablauf des erfindungsgemäßen Verfahrens (C) durch das folgende Formelschema darstellen:

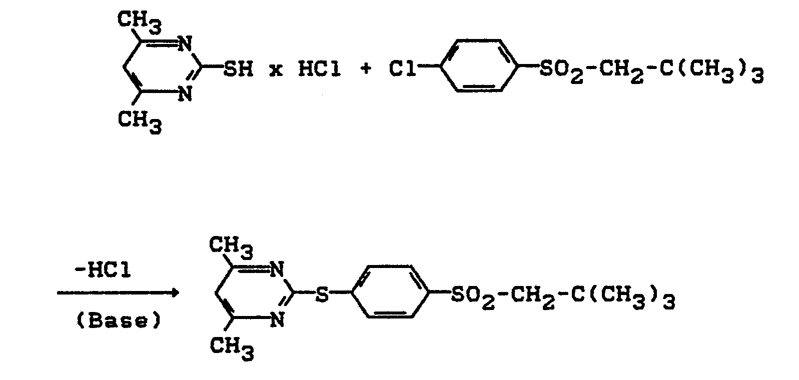

Die zur Durchführung des erfindungsgemäßen Verfahrens (A) als Ausgangsstoffe benötigten Thiophenol-Derivate sind durch die Formel (II) allgemein definiert. In dieser Formel (II) haben B, X, Z und n vorzugsweise diejenigen Bedeutungen, die bereits bei der Beschreibung der erfindungsgemäßen Stoffe der Formel (I) vorzugsweise für dies Substituenten genannt wurden.

Die Thiophenol-Derivate der Formel (II) sind teilweise bekannt (vgl. z.B. Coll. Czech. Chem. Commun, 29, 2161 (1964).

Die Thiophenol-Derivate der Formel (Ila), 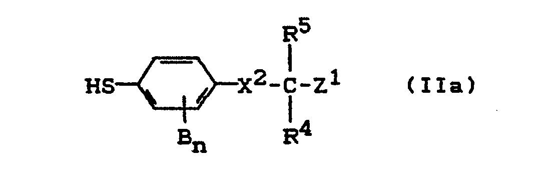 in welcher
R⁴ und R⁵ unabhängig voneinander für Wasserstoff, Alkyl, Alkoxalkyl oder Halogenalkyl stehen, X² für Sauerstoff oder Schwefel steht,
Z¹ für die Gruppe 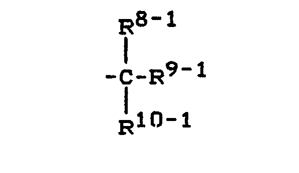 steht, wobei
R⁸⁻¹ für Alkyl steht,
R⁹⁻¹ für Alkyl, Alkoxy, Alkoxyalkyl, Alkylthio oder Halogenalkyl steht und
R¹⁰⁻¹ für Alkyl steht,
B für Halogen, Nitro, Cyano, Amino, Alkyl, Alkoxy, Halogenalkyl oder Halogenalkoxy steht, wobei B gleich oder verschieden sein kann, und
n für eine Zahl 0, 1,2.3 oder 4 steht,
sowie deren optische Isomere, sind neu und ebenfalls Gegenstand der vorliegenden Erfindung.

Bevorzugt sind die Verbindungen der Formel (Ila), in denen
R⁴ und R⁵ unabhängig voneinander jeweils für Wasserstoff, C₁-C₄-Alkyl, C₁-C₄-Alkoxy-C₁-C₄-alkyl oder Halogen-C₁-C₄-alkyl stehen,
X² für Sauerstoff oder Schwefel steht,
Z¹ für die Gruppe 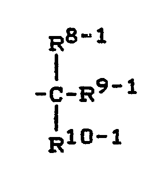 steht, wobei
R⁸⁻¹ für Ci-C₄-Alkyl steht,
R⁹⁻¹ für C₁-C₄-Alkyl, C₁-C₄-Alkoxy, C₁-C₄-Alkoxy-C₁-C₄-alkyl, C₁-C₄-Alkylthio oder Halogen-C₁-C₄-atkyl steht, R¹⁰⁻¹ für C₁-C₄-Alkyl steht,
B für Halogen, Nitro, Cyano, Amino, C₁-C₄-Alkyl, C₁-C₄-Alkoxy, Halogen-C₁-C₄-alkyl oder Halogen-C₁-C₄- alkoxy steht, wobei B gleich oder verschieden sein kann, und
n für eine ganze Zahl 0,1,2,3 oder 4 steht,
sowie deren R-und S-Isomere.

Besonders bevorzugt sind die Verbindungen der Formel (Iia) in denen,
R⁴ und R⁵ unabhängig voneinander jeweils für Wasserstoff, Methyl, Ethyl, Methoxymethyl, Methoxyethyl,
Ethoxymethyl, Ethoxyethyl oder Halogen-C₁-C₂-alkyl stehen,
X² für Sauerstoff oder Schwefel steht,
Z' für die Gruppe 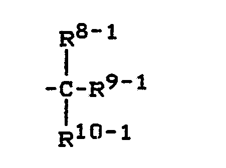 steht, wobei
R⁸⁻¹ für Methyl oder Ethyl steht,
R⁹⁻¹ für Methyl, Ethyl, n-Propyl, i-Propyl, n-, i-, sec-oder tert.-Butyl, Methoxy, Ethoxy, Methoxymethyl, Methoxyethyl, Ethoxymethyl, Ethoxyethyl, Methylthio, Ethylthio oder Halogen-C₁-C₂-alkyl steht, R¹⁰⁻¹für Methyl oder Ethyl steht,
B für Fluor, Chlor, Brom, Methyl, Ethyl, Methoxy, Ethoxy, Halogen-C₁-C₂-alkyl oder Halogen-C₁-C₂-alkoxy steht, wobei B gleich oder verschieden sein kann, und
n für eine ganze Zahl 0,1 oder 2 steht,

Beispielhaft seien die folgenden Verbindungen der Formel (Ila) genannt:
4-Neopentyloxy-thiophenol
2-Methyl-4-neopentyloxy-thiophenol
3-Methyl-4-neopentyloxy-thiophenol
2-Chlor-4-neopentyloxy-thiophenol
3-Chlor-4-neopentyloxy-thiophenol
2-Methoxy-4-neopentyloxy-thiophenol
3-Methoxy-4-neopentyloxy-thiophenol
4-Pinakolyloxy-thiophenol.

Man erhält die Thiophenol-Derivate der Formel (Ila), wenn man D) Amino-(thio)phenol-Derivate der Formel (VIII) 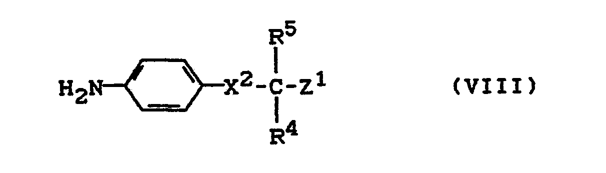 in welcher
R⁴, R⁵, X² und Z¹ die oben angegebene Bedeutung haben,
in einer 1. Stufe mit einem Nitrosierungsmittel, gegebenenfalls in Gegenwart eines Verdünnungsmittels und gegebenenfalls in Gegenwart eines Reaktionshilfsmittels diazotiert, das Diazotierungsprodukt in einer 2. Stufe in Gegenwart eines Verdünnungsmittels und in Gegenwart einer Base mit Alkalixanthogenat, insbesondere mit Kaliumxanthogenat, umsetzt und anschließend das Reaktionsprodukt in einer 3. Stufe in Gegenwart eines Verdünnungsmittels mit Alkalihydroxid erhitzt,
oder wenn man

E) Benzolsulfochloride der Formel (IX) 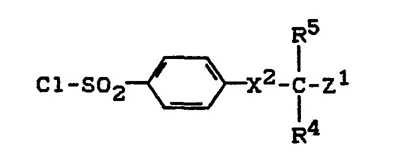 in welcher
_{R}⁴, R⁵, X² und Z¹ die oben angegebene Bedeutung haben,
mit Reduktionsmitteln gegebenenfalls in Gegenwart eines Verdünnungsmittels und gegebenenfalls in
Gegenwart eines Reaktionshilfsmittels reduziert.

Die zur Durchführung des erfindungsgemäßen Verfahrens (D) als Ausgangsstoffe benötigten Amino-(thio)phenol-Derivate sind durch die Formel (VIII) definiert. In dieser Formel stehen R⁴, R⁵, X² und Z¹ vorzugsweise für diejenigen Reste, die bei der Beschreibung der Stoffe der Formel (Ila) vorzugsweise für diese Substituenten genannt wurden.

Man erhält die Amino-(thio)phenol-Derivate der Formel (VIII) beispielsweise, indem man Nitro-(thio)-phenol-Derivate der Formel (X) 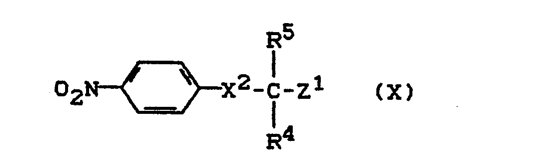 in welcher
R⁴, R⁵, X² und Z¹ die oben angegebene Bedeutung haben,
mit Raney-Nickel in Gegenwart eines Verdünnungsmittels wie beispielsweise Methanol bei Drucken von 60 - 90 bar und bein Temperaturen zwischen 20 und 100° C hydriert.

Die Nitro-(thio)phenole der Formel (X) sind bekannt oder lassen sich nach bekannten Methoden herstellen, indem man beispielsweise 4-Halogennitrobenzole der Formel (XI) 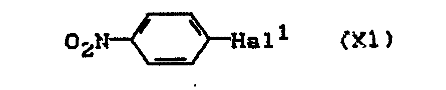 in welcher
Hal¹ für Halogen, insbesondere für Fluor, Chlor oder Brom, steht,
mit (Thio)Alkoholen der Formel (XII) 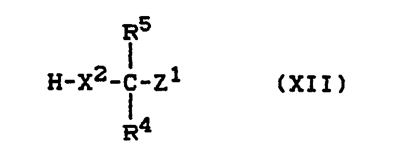 in welcher
R⁴, R⁵, X² und Z¹ die oben angegebene Bedeutung haben,
in Gegenwart eines Verdünnungsmittels, wie beispielsweise Dimethylsulfoxid, N-Methylpyrrolidon oder Sulfolan, und in Gegenwart eines Säurebindemittels, wie beispielsweise Natriumhydrid, Natrium-oder Kaliumhydroxid oder Natrium-oder Kalium-methanolat, bie Temperaturen zwischen +20° C und +200° C umsetzt.

Die 4-Halogennitrobenzole der Formel (XI) und die (Thio)Alkohole der Formel (XIII) sind bekannte Verbindungen oder lassen sich nach bekannten Methoden herstellen.

Die zur Durchführung des erfindungsgemäßen Verfahrens (D) weiterhin benötigten Reaktionsmittel sind bekannte Verbindungen.

Als Nitrosierungsmittel lassen sich alle für derartige Diazotierungen bekannten Verbindungen einsetzen. Vorzugsweise verwendet man Natrium-oder Kaliumnitrit in Gegenwart einer starken Mineralsäure.

In der 1. und 2. Stufe des erfindungsgemäßen Verfahrens (D) verwendet man als Verdünnungsmittel vorzugsweise Wasser. In der 3. Stufe verwendet man vorzugsweise Alkohole wie beispielsweise Methanol oder Ethanol oder deren Gemische mit Wasser, cyclische Ether wie beispielsweise Dioxan oder Tetrahydrofuran oder deren Gemische mit Wasser.

Die Reaktionstemperaturen können bei der Durchführung des erfindungsgemäßen Verfahrens (D) in einem größeren Bereich varriert werden. Im allgemeinen arbeitet man in der 1. Stufe bei Temperaturen zwischen -30° C und +50° C, vorzugsweise zwischen -20° C und +20° C, in der 2. Stufe zwischen +20° C und 130° C, vorzugsweise zwischen +50_{°} C und 100° C und in der 3. Stufe bei Temperaturen zwischen +20_{°} C und 200° C, vorzugsweise zwischen +₅0_{°} C und +150° C.

Bei der Durchführung des erfindungsgemäßen Verfahrens (D) setzt man die Ausgangsstoffe in der 1. und 2. Stufe im allgemeinen in angenähert äquimolaren Mengen um. Es ist jedoch auch möglich die eine oder andere Reaktionskomponente in einem größeren Überschuß zu verwenden. Die Base in der 3. Reaktionsstufe wird im allgemeinen in doppelt äquimolarer Menge umgesetzt.

Die zur Durchführung des erfindungsgemäßen Verfahrens (E) als Ausgangsstoffe benötigten Benzolsulfochloride sind durch die Formel (IX) definert. In dieser Formel stehen R⁴, R5 X² und Z¹ vorzugsweise für diejenigen Reste, die bei der Beschreibung der erfindungsgemäßen Stoffe der Formel (Ila) vorzugsweise für diese Substituenten genannt wurden.

Die Benzolsulfochloride der Formel (IX) sind bekannt oder lassen sich nach bekannten Methoden in einfacher Weise herstellen (vgl. Herstellungsbeispiele).

Als Reduktionsmittel lassen sich bei Berfahren (E) alle für derartige Reduktionen üblichen Stoffe einsetzen. Vorzugsweise verwendet man Zink.

Als Reaktionshilfsmittel verwendet man üblicherweise starke Mineralsäuren oder aliphatische Carbonsäuren. Bevorzugt ist die Verwendung von konzentrierter Salzsäure oder verdünnter Schwefelsäure.

Als Verdünnungsmittel kommen inerte organische Verdünnungsmittel infrage. Vorzugsweise verwendet man Alkohole wie Methanol oder Ethanol, Ether wie Dioxan oder Tetrahydrofuran oder niedere Ketone wie Aceton.

Die Reaktionstemperaturen können bei der Durchführung des erfindungsgemäßen Verfahrens (E) in einem größeren Bereich variiert werden. Im allgemeinen arbeitet man bei Temperaturen zwischen -40 und +₁40_{°} C, vorzugsweise zwischen -20° C und +₁00_{°} C.

Bei der Durchführung des erfindungsgemäßen Verfahrens (E) setzt man im allgemeinen pro Mol an Benzolsulfochlorid der Formel (IX) 2 - 10 Mol, vorzugsweis 2 - 5 Mol, an Reduktionsmittel und 2 bis 10 Mol, vorzugsweise 2 bis 5 Mol an Reaktionshilfsmittel ein.

In einer Verfahrensvariante kann die Herstellung und die Reduktion der Benzolsulfochloride auch als Eintopfreaktion durchgeführt werden.

Die zur Durchführung des erfindungsgemäßen Verfahrens (A) weiterhin als Ausgangsstoffe benötigten Pyri(mi)dylDerivate sind durch die Formel (111) allgemein definiert. In dieser Formel (III) stehen R¹, R², R³ und A vorzugsweise für diejenigen Reste, die bereits bei der Beschreibung der erfindungsgemäßen Stoffe der Formel (I) als bevorzugt für diese Substituenten genannt wurden. R¹⁵ steht vorzugsweise für Fluor, Chlor, Brom, Methylsulfonyl, Ethylsulfonyl, Phenylsulfonat oder Tosylat.

Beispielhaft seien die folgenden Pyri(mi)din-Derivate der Formel (III) genannt:
2-Chlor-6-methyl-pyridin
2-Chlor-4,6-dimethyl-pyridin
2-Methylsulphonyl-4-methyl-pyrimidin
2-Methylsulphonyl-4,5-dimethyl-pyrimidin
2-Methylsulphonyl-4,6-dimethyl-pyrimidin
2-Methylsulphonyl-4-ethyl-5-methyl-pyrimidin
2-Methylsulphonyl-4-methyl-5-ethyl-pyrimidin
2-Methylsulphonyl-4.5.6-trimethyl-pyrimidin
2-Chlor-4-methyl-pyrimidin
2-Chlor-4.5-dimethyl-pyrimidin
2-Chlor-4.6-dimethyl-pyrimidin
2-Chlor-4-ethyl-5-methyl-pyrimidin
2-Chlor-4-methyl-5-ethyl-pyrimidin
2-Chlor-4.5.6-trimethyl-pyrimidin.

Die Pyri(mi)dyl-derivate der Formel (III) sind bekannt oder lassen sich nach im Prinzip bekannten Methoden in einfacher Weise herstellen. So erhält man Pyrimidin-Derivate der Formel (111), in denen R¹⁵ für Halogen steht, zum Beispiel dadurch, daß man 2-Hydroxypyrimidin-Derivate (Dihydro-pyrimidon-2-Derivate) mit anorganischen Säurehalogeniden, wie zum Beispiel Phosphoroxychlorid oder Phosphorpentachlorid, umsetzt, oder auch dadurch, daß man entsprechende 2-Amino-pyrimidin-Derivate mit Salpetriger Säure in Gegenwart von Halogenwasserstoffsäuren umsetzt.

Die zur Durchführung des erfindungsgemäßen Verfahrens (B) als Ausgangsstoffe benötigten Pyri(mi)-dylthio-(thio)phenole sind durch die Formel (IV) definiert. In der Formel (IV) stehen R¹, R², R³, A, B und n vorzugsweise, bzw. besonders bevorzugt für diejenigen Reste, die bereits bei der Beschreibung der erfindungsgemäßen Stoffe der Formel (I) als bevorzugt bzw. besonders bevorzugt für diese Substituenten genannt wurden. X¹ steht für Sauerstoff oder Schwefel.

Die Verbindungen der Formel (IVa) 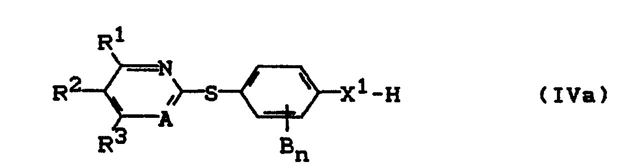 in welcher
R¹, R², R³, A, B, X¹ und n die oben angegebene Bedeutung haben, wobei jedoch R¹, R² und R³ nicht gleichzeitig für Wasserstoff steht, wenn A für die -CH-Gruppe steht und wobei die Verbindung 4-(4,6-Dimethyl-2-pyrimidylthio)-phenol ausgenommen ist,
sind neu und ebenfalls Gegenstand der vorliegenden Erfindung.

In der Formel IVa haben R¹, R², R³, A, B, X¹ und n vorzugsweise bzw. besonders bevorzugt diejenigen Bedeutungen, die bereits im Zusammenhang mit der Beschreibung der erfindungsgemäßen Stoffe der Formel (I) vorzugsweise bzw. besonders bevorzugt Reste genannt wurden, wobei jedoch R¹, R² und R³ nicht gleichzeitig für Wasserstoff steht, wenn A für die -CH-Gruppe steht und wobei die Verbindung 4-(4,6-Dimethyl-2-pyrimidylthio)-phenol ist. X¹ steht für Sauerstoff oder Schwefel.

Als Beispiele für Pyri(mi)dylthio-(thio)phenole der Formel (IVa) seien genannt:
4 (6-Methyl-2-pyridythio)-phenol
4-(4.6-Dimethyl-2-pyridylthio)-phenol
4-(4-Methyl-2-pyrimidylthio)-phenol
4-(4.5-Dimethyl-2-pyrimidylthio)-phenol
4-(4-Ethyl-5-methyl-2-pyrimidylthio)-phenol
4-(5-Ethyl-4-methyl-2-pyrimidylthio)-phenol
4-(4.5.6-Trimethyl-2-pyrimidylthio)-phenol
4-(4.6-Dimethyl-2-pyrimidylthio)-thiophenol

So erhält man beispielsweise Pyri(mi)dylthio-(thio)-phenole der Formel (IVa), wenn man (F) 2-Halogen-Pyri(mi)din-Derivate der Formel (XIII) 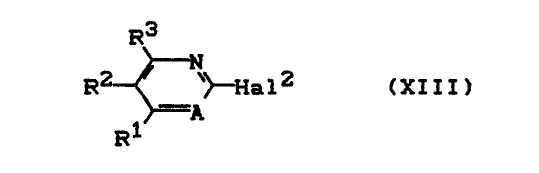 in welcher
R¹, R², R3 und A die oben angegebene Bedeutung haben und
Hal² für Halogen, insbesondere Fluor, Chlor oder Brom, steht,
mit Verbindungen der Formel (XIV) 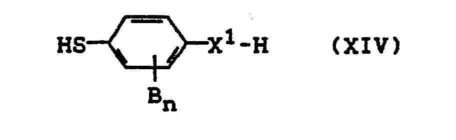 in welcher
X¹, B und n die oben angegebene Bedeutung haben,
in Gegenwart eines Lösungsmittels und in Gegenwart eines Säurebindemittels umsetzt.

Die bekannten Pyrimidylthio-(thio)phenole der Formel (IV) lassen sich analog zu Verfahren (F) herstellen.

Die bei dem obigen Verfahren (F) als Ausgangsstoffe benötigten 2-Halogen-Pyri(mi)din-Derivate sind durch die Formel (XIII) definiert. In dieser Formel haben R¹, R², R³ und A vorzugsweise diejenigen Bedeutungen, die bereits im Zusammenhang mit der Beschreibung der erfindungsgemäßen Stoffe der Formel (I) vorzugsweise für diese Reste genannt wurden. Hal¹ steht vorzugsweise für Fluor, Chlor oder Brom.

Die 2-Halogen-Pyri(mi)din-Derivate der Formel (XIII) sind .bekannt oder lassen sich nach im Prinzip bekannten Methoden in einfacher Weise herstellen (vgl. DE-OS 2,101,359).

Die bei dem Verfahren (F) weiterhin als Ausgangsstoffe benötigten Verbindungen sind durch die Formel (XIV) definiert. In dieser Formel haben X¹, B und n vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden.

Die Verbindungen der Formel (XIV) sind bekannt oder lassen sich nach im Prinzip bekannten Methoden in einfacher Weise herstellen.

Als Säurebindemittel können bei der Durchführung des Verfahrens (F) alle üblicherweise für derartige Umsetzungen verwendbaren Säureakzeptoren verwendet werden. Vorzugsweise verwendbar sind Alkali-und Erdalkalioxide, -hydroxide und -carbonate, wie Natriumhydroxid, Kaliumhydroxid, Calciumoxid, Natriumcarbonat und Kaliumcarbonat, ferner Alkali-alkoholate, -amide und -hydride, wie zum Beispiel Natriummethylat, Natriumethylat, Kalium-tert.-butylat, Natrium-amid und Natrium-hydrid.

Als Verdünnungsmittel können bei der Durchführung des Verfahrens (F) alle üblichen inerten organischen Solventien verwendet werden. Vorzugsweise in Frage kommen Kohlenwasserstoffe, wie Benzin, Toluol und Xylol, ferner Ether, wie Dioxan, Glykoldimethylether und Diglykoldimethylether, außerdem Nitrile, wie Acetonitril, und auch stark polare Solventien, wie Dimethylsulfoxid, Sulfolan, Dimethylformamid und N-Methyl-pyrolidon.

Die Reaktionstemperaturen können bei der Durchführung des Verfahrens (F) innerhalb eines größeren Bereiches variiert werden. Im allgemeinen arbeitet man bei Temperaturen zwischen 0° C und 200° C, vorzugweise zwischen 50° C und 150° C.

Die Umsetzung nach dem Verfahren (F) wird im allgemeinen unter Normaldruck vorgenommen.

Bei der Durchführung des Verfahrens (F) setzt man die Ausgangsstoffe der Formeln (XIII) und (XIV) im allgemeinen in angenähert äquimolaren Mengen um. Es ist je doch auch möglich, die eine oder andere Komponente in einem grösseren Überschuß zu verwenden. Die Aufarbeitung erfolgt nach üblichen Methoden.

Die bei dem Verfahren (B) weiterhin als Ausgangsstoffe benötigten Verbindungen sind durch die Formel (V) definiert. In dieser Formel haben Y und Z vorzugsweise diejenigen Bedeutungen, die bereits bei der Beschreibung der erfindungsgemäßen Stoffe der Formel (I) als bevorzugt für diese Substituenten genannt werden, G steht vorzugsweise für Fluor, Chlor, Brom, Methylsulfonyl, Ethylsulfonyl, Phenylsulfonat, Tosylat oder Mesylat.

Beispielhaft seien die folgenden Verbindungen der Formel (V) genannt:
Isobutyl-tosylat
Neopentyl-tosylat
2.2-Dimethyl-1-butyl-tosylat
2.2-Dimethyl-1-pentyl-tosylat
2.2-Dimethyl-3-methoxy-1-propyl-tosylat
2.2-Dimethyl-3-ethoxy-1-propyl-tosylat
2-Methyl-2-methoxy-1-propyl-tosylat
2.2₋Dimethyl-3-fluor-1-propyl-tosylat
2.2-Dimethyl-3-Chlor-1-propyl-tosylat
2.2-Di-(Chlormethyl)-1 -propyl-tosylat
2.2-Di-(Chlormethyl)-3-chlor-1-propyl-tosylat
2.2-Dimethyl-4-nitrilo-1-butyl-tosylat
3,3-Dimethyl-2-hydroxy-1-butyl-tosylat
Solketal-tosylat
2-Ethoxycarbonyl-2-methyl-1-propyl-tosylat
Hydroxypivalaldehyd-tosylat
1-Chlorpinakolin
Prenylbromid
Methallylchlorid ₑ
2-Fluormethyl-allylchlorid
Trifluorethyl-phenylsulfonat
3-Methyl-3-tosyloxymethyl-oxetan •
3-Ethyl-3-mesyloxyethyl-oxetan
3-Isopropyl-3-tosyloxymethyl-oxetan
2-Methyl-2-tosyloxymethyl-pyran
Dimethyl-tert.-butyl-silylchlorid
Dimethyl-2-(3-Methyl-butyl)-silylchlorid
Chlormethyl-trimethylsilan
3.3-Dimethyl-1-butyl-mesylat.

Die Verbindungen der Formel (V) sind teilweise bekannt und lassen sich nach im Prinzip bekannten Verfahren in einfacher Weise herstellen (vgl. z.B. Helv. Chim. Acta. 63, 1412 (1980)).

Die bei dem erfindungsgemäßen Verfahren (C) als Ausgangsstoffe benötigten Verbindungen sind durch die Formel (VI) definiert. In der Formel (VI) haben R¹, R², R³ und A vorzugweise diejenigen Bedeutungen, die bereits bei der Beschreibung der erfindungsgemäßen Stoffe der Formel (I) als bevorzugt für diese Substituenten genannt wurden.

Die bei dem erfindungsgemäßen Verfahren (C) weiterhin als Ausgangsstoffe benötigten Verbindungen sind durch die Formel (VII) definiert. In dieser Formel haben B, Y, Z und n vorzugsweise die Bedeutung, die bei der Beschreibung der Stoffe der Formel (I) als bevorzugt für diese Substituenten genannt werden. Hal steht vorzugsweise für Fluor, Chlor oder Brom.

Die Verbindungen der Formel (VI) und (VII) sind bekannt oder lassen sich nach im Prinzip bekannten Methoden in einfacher Weise herstellen (vgl. Herstellungsbeispiele).

Als Verdünnungsmittel können bei dem erfindungsgemäßen Verfahren (A) alle inerten Solventien eingesetzt werden. Vorzugsweise verwendbar sind Alkohole bzw. Ether, wie Methanol, Ethanol, Isopropanol, Cyclohexanol, Ethylenglykol, Ethylenglykolmonomethylether, Diethylenglykol, Diethylenglykolmonoethylether und ferner Halogenkohlenwasserstoffe, wie Methylenchlorid, Chloroform, Tetrachlorkohlenstoff, Chlorbenzol und o-Dichlorbenzol und Kohlenwasserstoffe wie Toluol, Xylol und Tetralin, außerdem Ketone, wie Aceton und Methyl-isopropylketon, weiterhin cyclische Ether, wie Tetrahydrofuran und Dioxan, darüberhinaus Carbonsäureester, wie Ethylacetat und Glykolmonomethyletheracetat und auch stark polare Solventien, wie Dimethylsulfoxid, Sulfolan, N-Methylpyrrolidon, Dimethylacetamid und Dimethylformamid.

Als Säurebindemittel kommen bei der Umsetzung nach dem erfindungsgemäßen Verfahren (A) alle üblichen Säureakzeptoren in Betracht. Vorzugsweise verwendbar sind tertiäre Amine, wie Triethylamin, Pyridin und N,N-Dimethyl-anilin, ferner Erdalkalimetalloxide, wie Magnesium-und Calciumoxid, außerdem Alkali-und Erdalkali-metall-carbonate, wie Natriumcarbonat, Kaliumcarbonat und Calciumcarbonat, außerdem Alakali-und Erdalkali-metall-hydroxide, wie Lithiumhydroxid, Natriumhydroxid, Kaliumhydroxid und Calciumhydroxid.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen -20° C und +₁00_{°} C, vorzugsweise zwischen 50° C und 160° C.

Bei der Durchführung des erfindungsgemäßen Verfahrens (A) werden die Ausgangsstoffe der Formeln (11) und (111) und auch gegebenenfalls die Base im allgemeinen in angenähert äquivalenten Mengen verwendet. Es ist jedoch auch möglich die eine oder andere Reaktionskomponente in einem größeren Überschuß einzusetzen. Die Aufarbeitung erfolgt nach üblichen Methoden.

Als Verdünnungsmittel und Säurebindemittel können bei dem erfindungsgemäßen Verfahren (B) vorzugsweise diejenigen Verdünnungsmittel und Säurebindemittel verwendet werden, die auch bei dem Verfahren (A) in Betracht kommen.

Die Reaktionstemperaturen können auch bei dem erfindungsgemäßen Verfahren (B) innerhalb eines größeren Bereiches variiert werden. Im allgemeinen arbeitet man bei Temperaturen zwischen -20° C und +200_{°} C, vorzugsweise zwischen 0 und 160° C.

Bei der Durchführung des erfindungsgemäßen Verfahrens (B) werden die Ausgangsstoffe der Formeln (IV) und (V) und auch gegebenenfalls die Base im allgemeinen in angenähert äquimolaren Mengen verwendet. Es ist jedoch auch möglich die eine oder andere Reaktionskomponente in einem größeren Überschuß einzusetzen.

Bei der Durchführung des erfindungsgemäßen Verfahrens (C) werden als Verdünnungsmittel und Säurebindemittel vorzugsweise diejenigen Verdünnungsmittel und Basen verwendet, die bei dem Verfahren (A) genannt wurden.

Die Reaktionstemperaturen können bei dem erfindungsgemäßen Verfahren (C) innerhalb eines größeren Bereichs varriert werden. Im allgemeinen arbeitet man bei Temperaturen zwischen 0 und 200° C, vorzugsweise zwischen 50 und 150° C.

Bei der Durchführung des erfindungsgemäßen Verfahrens (C) werden die Ausgangsstoffe der Formel (VI) und (VII) und gegebenenfalls die Base in angenähert äquimolaren Mengen umgesetzt. Es ist jedoch auch möglich, die eine oder andere Reaktionskomponente in einem größeren Überschuß zu verwenden.

Die erfindungsgemäßen Verfahren (A), (B) und (C) werden im allgemeinen unter Normaldruck durchgeführt. Es ist jedoch auch möglich unter vermindertem oder erhöhtem Druck zu arbeiten.

Die Reaktionsdurchführung und Aufarbeitung erfolgen bei den Verfahren (A), (B) und (C) nach üblichen Methoden (vgl. Herstellungsbeispiele).

Die erfindungsgemäßen Wirkstoffe können als Defoliants, Desiccants, Krautabtötungsmittel .und insbesondere als Unkrautvernichtungsmitel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao, Beerenfrücht-und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur selektiven Bekämpfung mono-und dikotyler Unkräuter insbesondere in monokotylen und dikotylan Kulturen wie beispielsweise Weizen und Zuckerrüben im Nachauflaufverfahren einsetzen.

Weiterhin läßt sich die Gruppe von Verbindungen der Formel (I), in welcher R¹ und R³ für Methyl stehen, R² für Wasserstoff und Z für 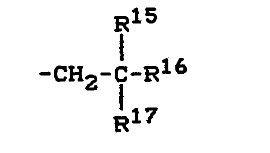 stehen, mit besonders gutem Erfolg zur selektiven Bekämpfung mono-und dikotyler Unkräuter in Rüben, insbesondere im Nachauflaufverfahren, einsetzen.

Bei der Anwendung im Nachauflaufverfahren können die erfindungsgemäßen Wirkstoffe allein oder in Kombination mit emulgierbaren Ölen, oberflächenaktiven Substanzen und anderen Additiven ausgebracht werden.

Darüber hinaus greifen die erfindungsgemäßen Wirkstoffe in den Metabolsimus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoff, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Anwendung als Herbizide können erfindungsgemäßen Wirkstoffe als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1 H,3H)-dion oder N-(2-Benzthiazolyl)-N,N'-dimethyl-harnstoff zur Unkrautbekämpfung in Getreide: 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Als Mischpartner kommen auch in Frage:
Harnstoffe (z.B. Methabenzthiazuron, Chlortuluron, Triazine (z.B. Atrazin, Terbutryne, Cyanazin), Triazinone (z.B. Ethiozin, Metribuzin), Triazindione (z.B. Amethydione), Diphenylether (z.B. Bifenox), Benzonitrile (z.B. loxymil, Bromoxymil), Phenoxyalkancarbonsäuren (z.B. 2,4 D, 2,4 DP, MCPA, MCPP etc.), Aryloxy-oder ,Heteroaryloxyphenoxypropionsäuren (z.B. Haloxyfop, Disclofop, Fenoxaprop).
(R)-2-[4-(3,5-Dichlor-pyridyl-2-oxy)-phenoxyl]-propionsäure-(trimethylsilyl)-methylester, Imidazolinone (z.B. Imazamethabenz), Bentazone, Pyridate, Phenmedipham, Ethofumesate, Chloridazon und Triallate.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfingungsgemäßen Wirkstoffe können sowohl vor als auch nach dem Auflaufen der Pflanzen appliziert werden.

Sie können auch vor der Saat in den Boden eingearbeitet werden.

Die angewendte Wirkstoffmenge kann in einem größeren Bereich schwanken. Sie hängt im wesentlichen von der Art des gewünschten Effektes ab. Im allgemeinen liegen die Aufwendmengen zwischen 0,01 und 10 kg Wirkstoff pro hektar Bodenfläche, vorzugsweise zwischen 0,05 und 5 kg pro ha.

Bei der Anwendung als Wachstumsregulatoren können die erfindungsgemäßen Wirkstoffe in den Formulierungen ebenfalls in Mischung mit anderen bekannten Wirkstoffen vorliegen, wie fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Werkstoffzubereitung oder den Wirkstoff selbst in den Boden zu injizieren. Es kann auch das Saatgut der Pflanzen behandelt werden.

Bei der Anwendung als Wachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

### Herstellungsbeispiele

### Beispiel 1

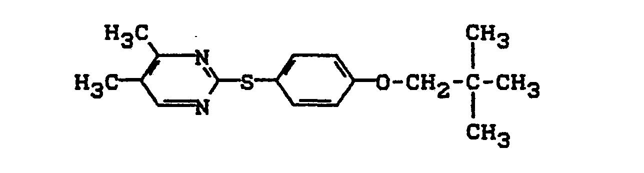

Verfahren (A)

3,43 g (17,5 mmol) 4-Neopentyloxy-thiophenol werden in 20 mi N-Methylpyrrolidon unter Stickstoff mit 1,12 g (20 mmol) gepulvertem Kaliumhydroxid versetzt, 15 Minuten verrührt und nach Zugabe von 2,5 g (17,5 mmol) 4,5-Dimethyl-2-chlor-pyrimidin unter Kontrolle durch Dünnschichtchromatographie (DC) auf 110° C erwärmt. Nach dem Abkühlen wird die Lösung in 100 ml Wasser eingerührt. Der Niederschlag wird abgesaugt und mit Cyclohexan/Essigester 3:1 an Kieselgel chromatographiert. nach Kristallisation aus n-Hexan erhält man 2,85 g (53,9 % der Theorie) 4-(4,5-Dimethyl-2-pyrimidylthio)-phenyIneopentylether vom Schmelzpunkt 69° C.

### Beispiel 2

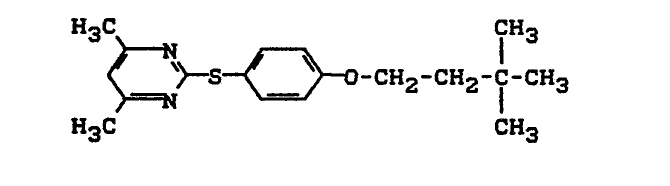

Verfahren (B)

4,64 g (20 mmol) 4-(4,6-Dimethyl-2-pyrimidylthio)-phenol werden in 20 ml N-Methyl-pyrrolidon unter Stickstoffatmosphäre mit 2,73 g (24 mmol) Kalium-tert. -butylat versetzt, 5 Minuten verrührt und nach Zugabe von 3,96 g (22 mmol) 3,3-Dimethyl-1-butyl-mesylat 6 Stunden auf 150° C erhitzt. Die Lösung wird in 100 ml 1N Natriumhydroxid-Lösung eingerührt, 3 x mit Toluol extrahiert und mit Magnesiumsulfat getrocknet. Die organische Phase wird im Vakuum eingedampft. Der Rückstand wird mit Cyclohexan/Essigester 3:1 an Kieselgel chromatographiert. Nach Kristallisation aus n-Hexan bei -20° C erhält man 2,64 g (41,7 % der Theorie) 4-(4,6-Dimethyl-2-pyrimidylthio)-phenyl-3,3-dimethyl-1-butylether vom Schmelzpunkt 57° C.

### Beispiel 3

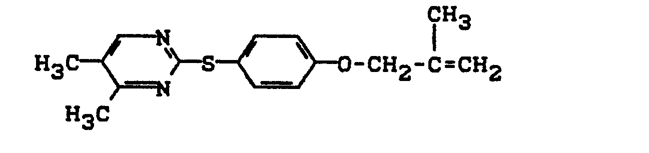

Verfahren (B)

4,64 g (20 mmol) 4-(4,5-Dimethyl-2-pyrimidylthio)-phenol werden in 30 ml absolutem Acetonitril mit 1,24 g (24 mmol) gepulvertem Kaliumhydroxid, 15 Minuten verrührt und nach Zugabe von 2,44 ml (25 mmol) Methallylchlorid unter DC-Kontrolle auf 60° C erhitzt. Die Lösung wird in 150 ml 1N Natriumhydroxid-Lösung eingerührt, 3 x mit Methylenchlorid extrahiert und mit Magnesiumsulfat getrocknet. Die organische Phase wird im Vakuum eingedampft. Man erhält 4,8 g (89,5 % der Theorie) 4-(4,5-Dimethyl-2-pyrimidyl- thio)-phenyl-methallylether als blaßgelbes ÖI. ¹H-NMR (200 MH_{z}, CDCl₃): δ ₌ 1,9 (d,3H, 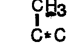 ), 2.15 (s; 3H, Pyrimidin 5-CH₃), 2.38, (s, 6H, Pyrimidin 4-CH₃ )/4.47 (s, 2H, O-CH₂)/ 5.05 ("dm", 2H, CH2- 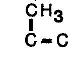 H₂)/6.95, 7.55 (AA'BB'm, 4-H, Phenyl-H), 8.14 (s, 1 H, Pyrimidin 6-H)

### Beispiel 4

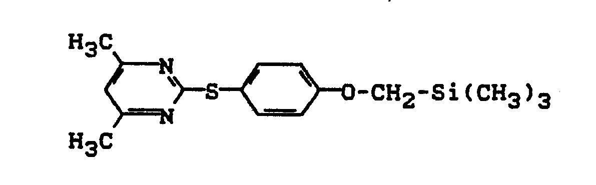

Verfahren (B)

Zu 4,64 g (20 mmol) 4-(4,6-Dimethyl-2-pyrimidylthio)-phenol in 30 ml absolutem Acetonitril gibt man 1,23 g (22 mmol) gepulvertes Kaliumhydroxid und verrührt die Lösung 10 Minuten lang. Nach Zugabe von 2,8 ml (20 mmol) Chlormethyl-trimethylsilan erhitzt man unter DC-Kontrolle auf 60° C. Die Lösung wird filtriert und im Vakuum eingedampft. Der Rückstand wird mit Cyclohexan/Essigester 2:1 chromatographiert und das verbleibende Produkt aus n-Hexan kristallisiert. Man erhält 2,17 g (27,8 % der Theorie) 4-(4,6-Dimethyl-2-pyrimidylthio)-phenylmethylen-trimethylsilylether vom Schmelzpunkt 75° C.

### Beispiel 5

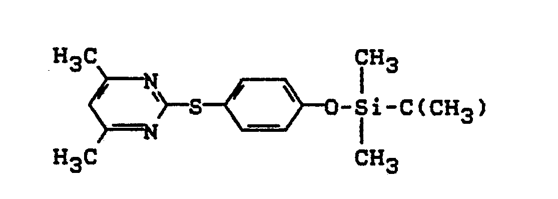

Verfahren (B)

3,31 g (22 mmol) Dimethyl-tert.-butyl-silylchlorid werden in 20 ml absolutem Methylenchlorid gelöst und mit 3,66 ml (24 mmol) Diazabicycloundecen (DBU) versetzt. Dazu gibt man eine Suspension von 4,64 g (20 mmol) 4-(4,6-Dimethyl-2-pyrimidylthio)-phenol in 50 ml absolutem Methylenchlorid. Nach 1 Stunde wird die Lösung in eine gesättigte Ammoniumchlorid-Lösung gegossen, die Methylenchlorid-Phase abgetrennt und mit 1 N Natriumhydroxid-Lösung gewaschen. Nach dem Trocknen mit Natriumsulfat wird die Lösung im Vakuum eingedampft, und der Rückstand aus n-Hexan umkrisallisiert. Man erhält 4,49 g (65 % der Theorie) 4-(4,6-Dimethyl-2-pyrimidylthio)-phenyl-dimethyl-tert.-butyl-silylether vom Schmelzpunkt 61 ⁰ C.

### Beispiel 6

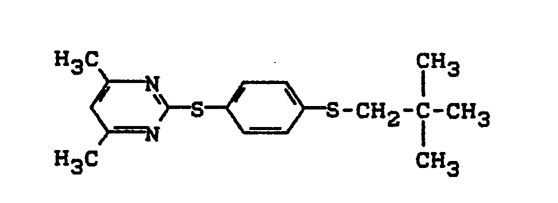

Verfahren (A)

4,03 g (19 mmol) 4-(Neopentylthio)-thiophenol werden in 20 ml N-Methylpyrrolidon unter Stickstoff vorgelegt, mit 1,35 g (24 mmol) gepulvertem Kaliumhydroxid versetzt, 15 Minuten verrührt und nach Zugabe von 2,7 g (19 mmol) 4,6-Dimethyl-2-chlor-pyrimidin unter DC-Kontrolle auf 110° C erhitzt. Die Reaktionsmischung wird in Wasser eingerührt und 5 x mit n-Hexan extrahiert. Die organische Phase wird über Magnesiumsulfat getrocknet und im Vakuum eingedampft. Der Rückstand wird in CyclohexanEssigester 3:1 chromatographiert. Nach Kristallisation aus n-Hexan erhält man 3,08 g (50,9 % der Theorie) 4-(4,6-Dimethyl-2-pyrimidylthio)-neopentylthiophenylether vom Schmelzpunkt 40° C.

### Beispiel 7

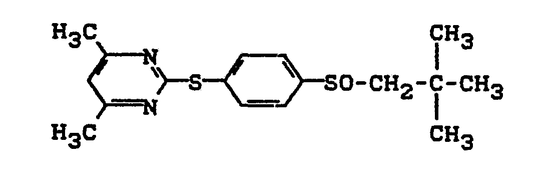

Verfahren (A)

3,88 g (17 mmol) 4-(Neopentylsulfoxy)-thiophenol werden in 20 ml N-Methylpyrrolidon unter Stickstoff mit 1,12 g (20 mmol) gepulvertem Käliumhydroxid versetzt, 15 Minuten verrührt und nach Zugabe von 2,42 g (17 mmol) 4,6-Dimethyl-2-chlor-pyrimidin unter DC-Kontrolle auf 110° C erhitzt. Die Reaktionsmischung wird in 100 ml Waser eingerührt und 3 x mit Toluol extrahiert. Die organische Phase wird über Magnesiumsulfat getrocknet und im Vakuum destilliert. Der Rückstand wird mit Cyclohexan/Essigester 2:1 an Kieselgel chromatographiert. Man erhält 2,75 g (48,4 % der Theorie) 4-(4,6-Dimethyl-2-pyrimidylthio)-neopentylsulfoxy-phenylether als hochviskoses Öl.

¹H-NMR (200 MHz, CDCl₃): = 1.23 (S, 9H, -C(CH₃)₃, 2.36 (S, 6H, Pyrimidin 4-CH₃ und 6-CH₃), 2.58 (AB, 1H, SO-CHH-C(CH₃)₃), 2.85 (AG, 1H, SO-CHH-C(CH₃)₃), 6.75 (S, 1Hm Pyrimidin 5-H), 7.63, 7.78 (AA', BB'm, 4H-Phenyl-H).

### Beispiel 8

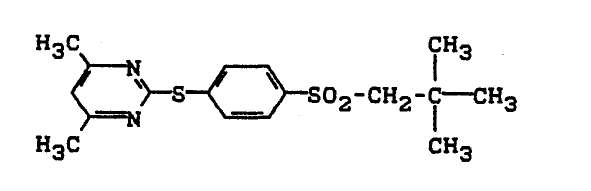

Verfahren (C)

5,36 g (30 mmol) 4,6-Dimethyl-2-mercapto-pyrimidinhydrochlorid werden in 20 ml N-Methylpyrrolidon unter Stickstoff mit 3,7 g (66 mmol) gepulvertem Kaliumhydroxid versetzt, 15 Minuten verrührt und nach Zugabe von 7,4 g (30 mmol) 4-(Neopentylsulfonyl)-chlorbenzol unter DC-Kontrolle auf 150° C erhitzt. Nach dem Abkühlen wird die Reaktionsmischung in 150 ml Wasser eingerührt und 3 x mit Toluol extrahiert. Die organische Phase wird mit Magnesiumsulfat getrocknet und im Vakuum destilliert. Nach Chromatographie mit Cyclohexan/Essigester 2:1 und Kristallisation aus Toluol/n-Hexan erhält man 1,79 g (17 % der Theorie) 4-(4,6-Dimethyl-2-pyrimidylthio)-phenyl-neopentyl-sulfon vom Schmelzpunkt 98° C.

Nach den in den vorausgehenden Beispielen und in der Beschreibung angegebenen Methoden lassen sich die in den folgenden Tabellen formelmäßig aufgeführten Verbindungen der Formel (I) herstellen:

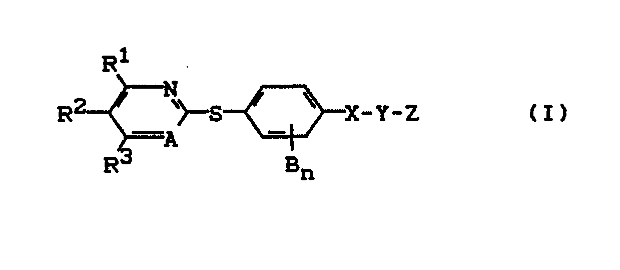

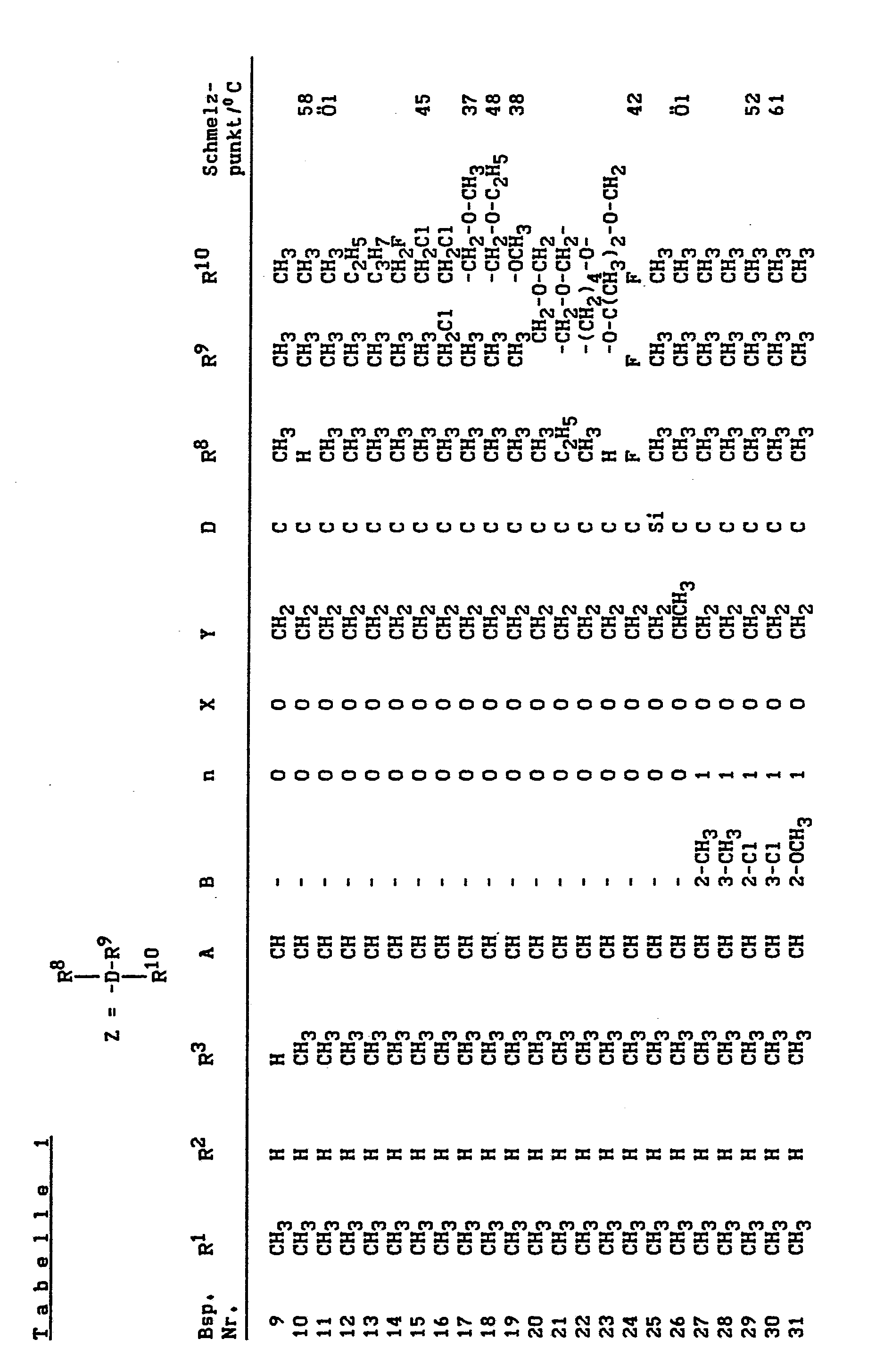

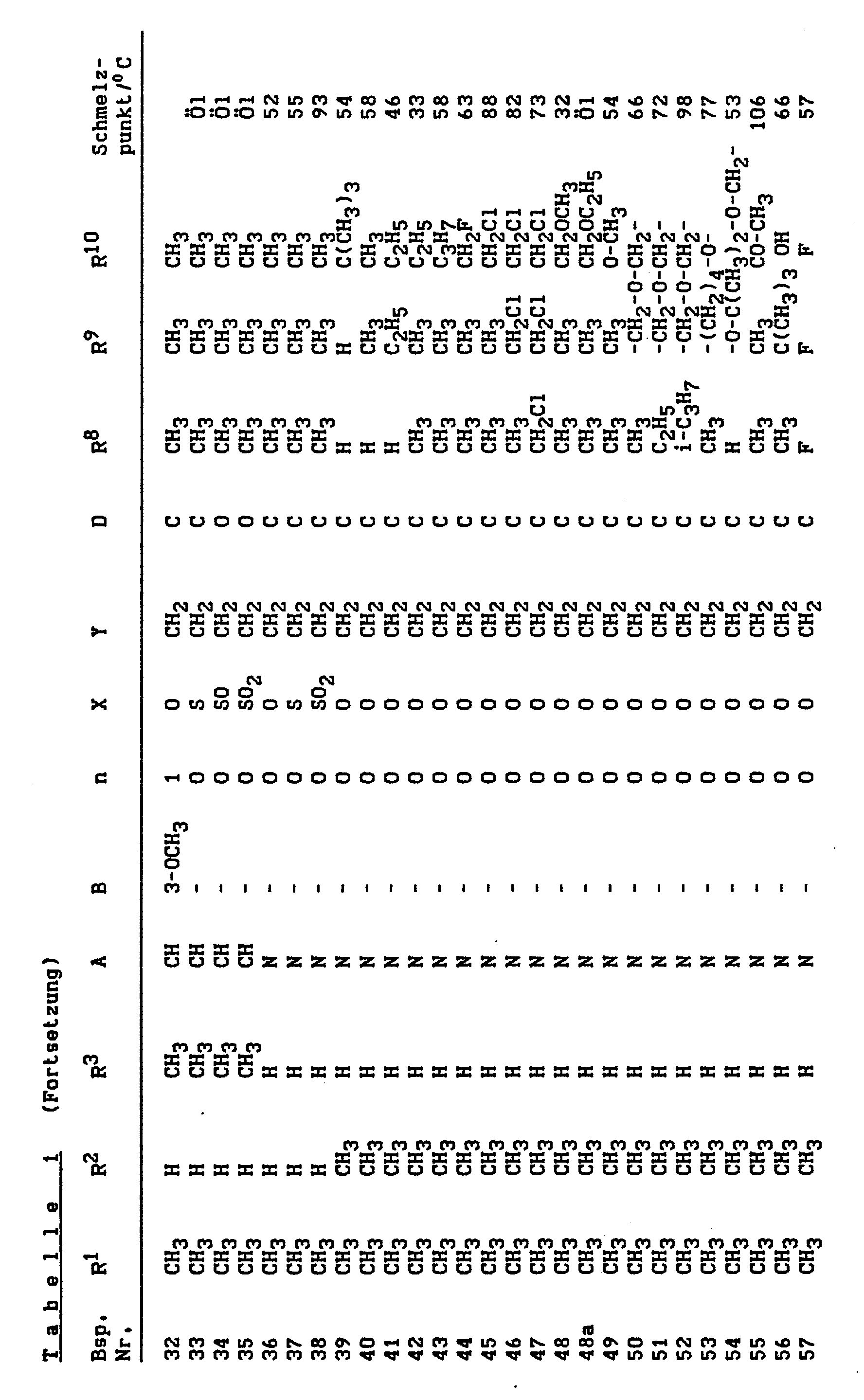

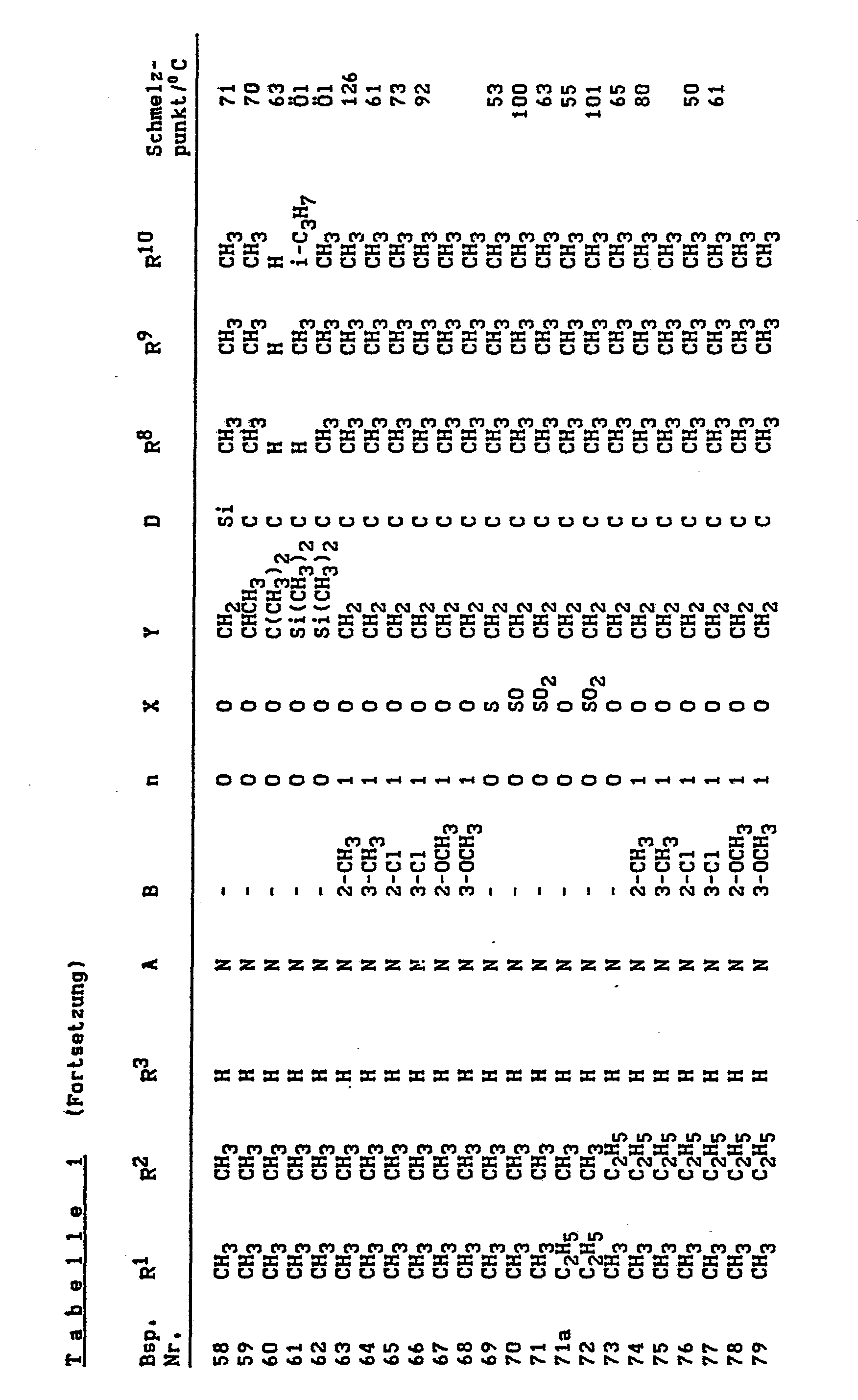

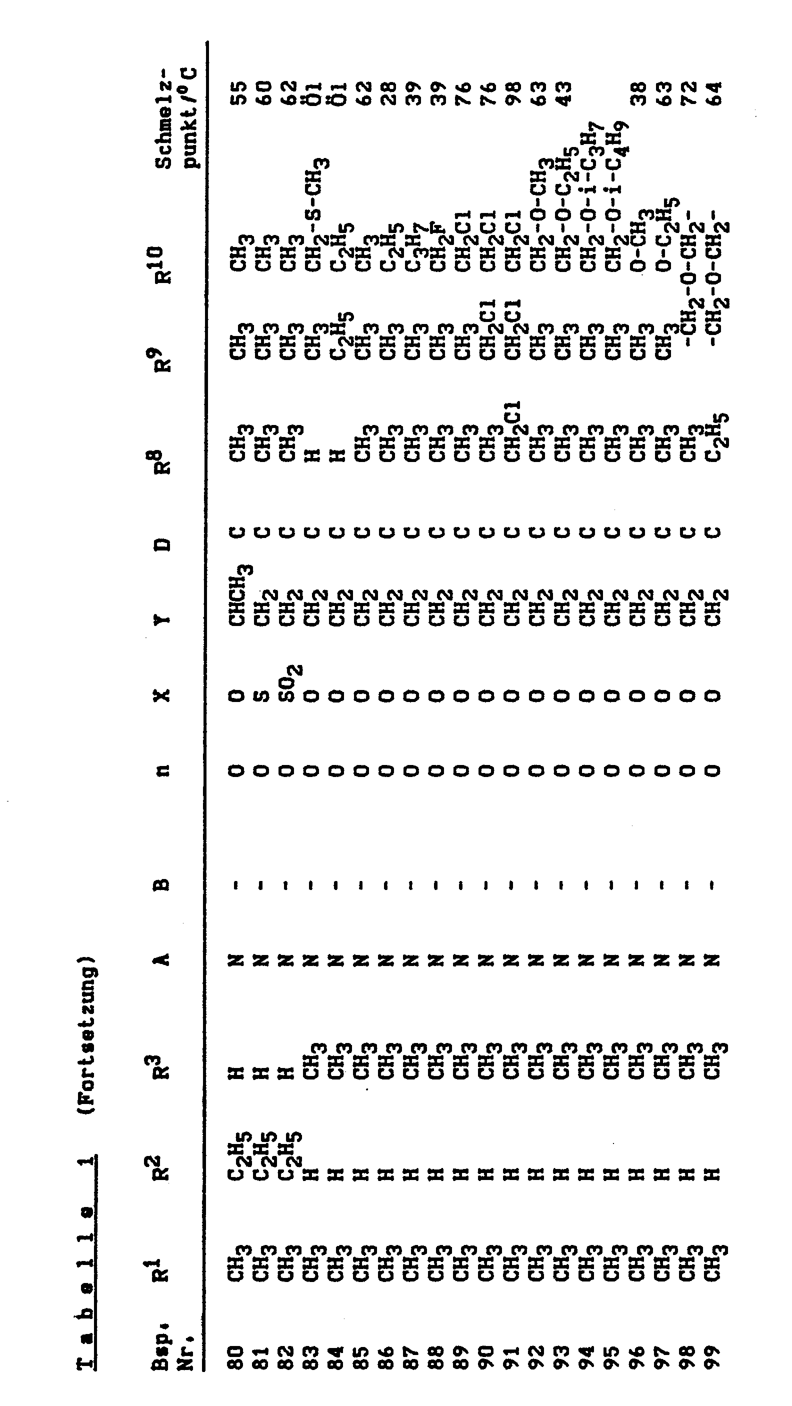

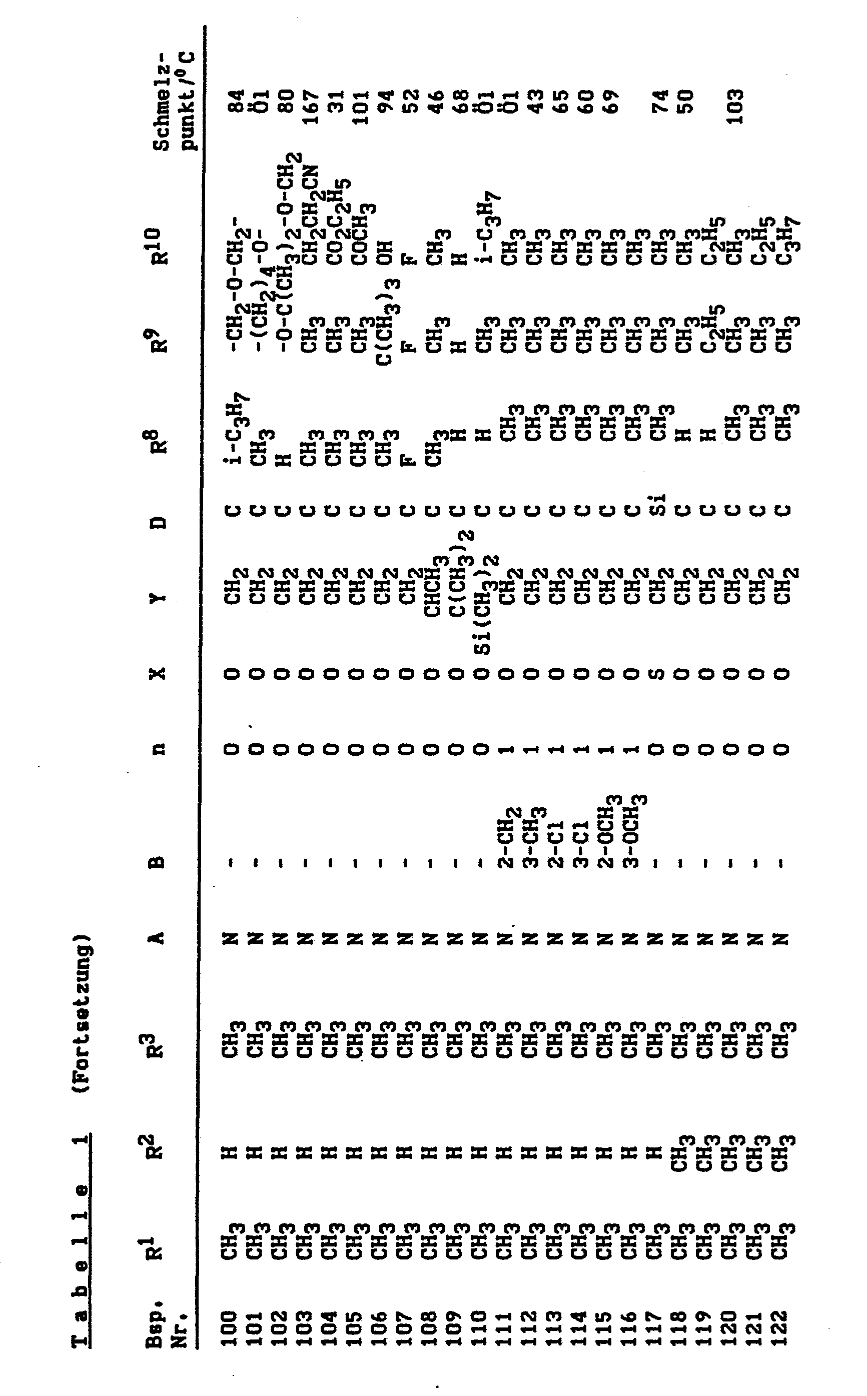

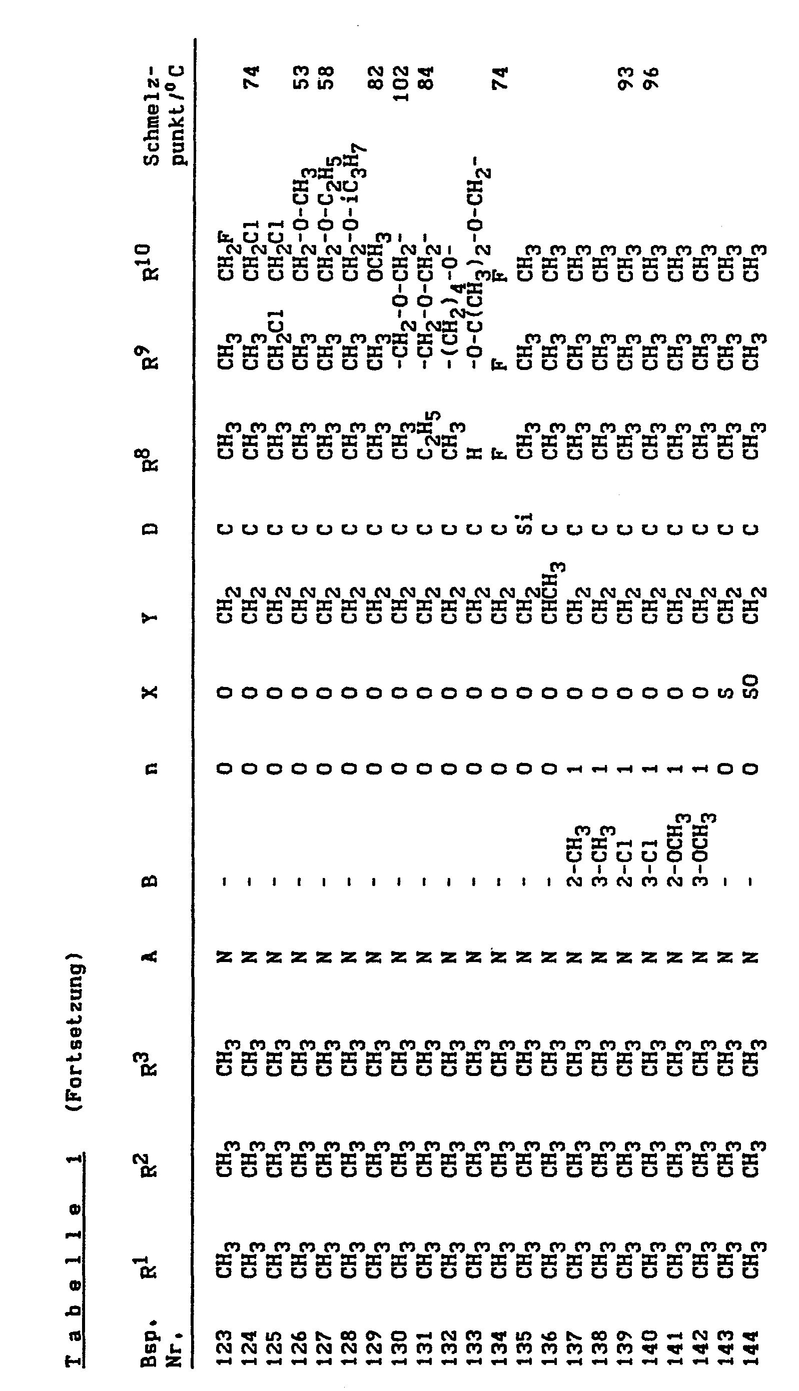

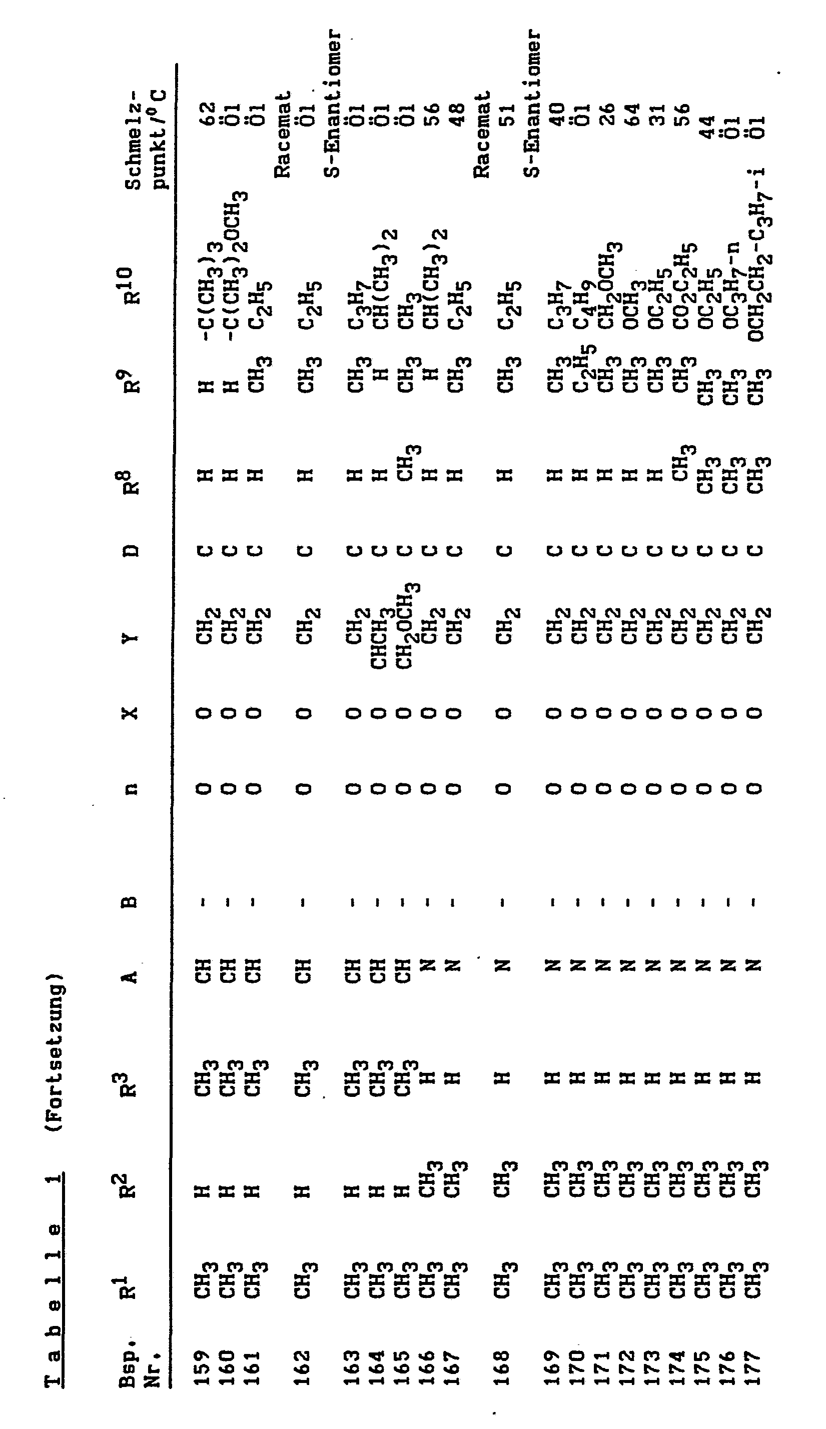

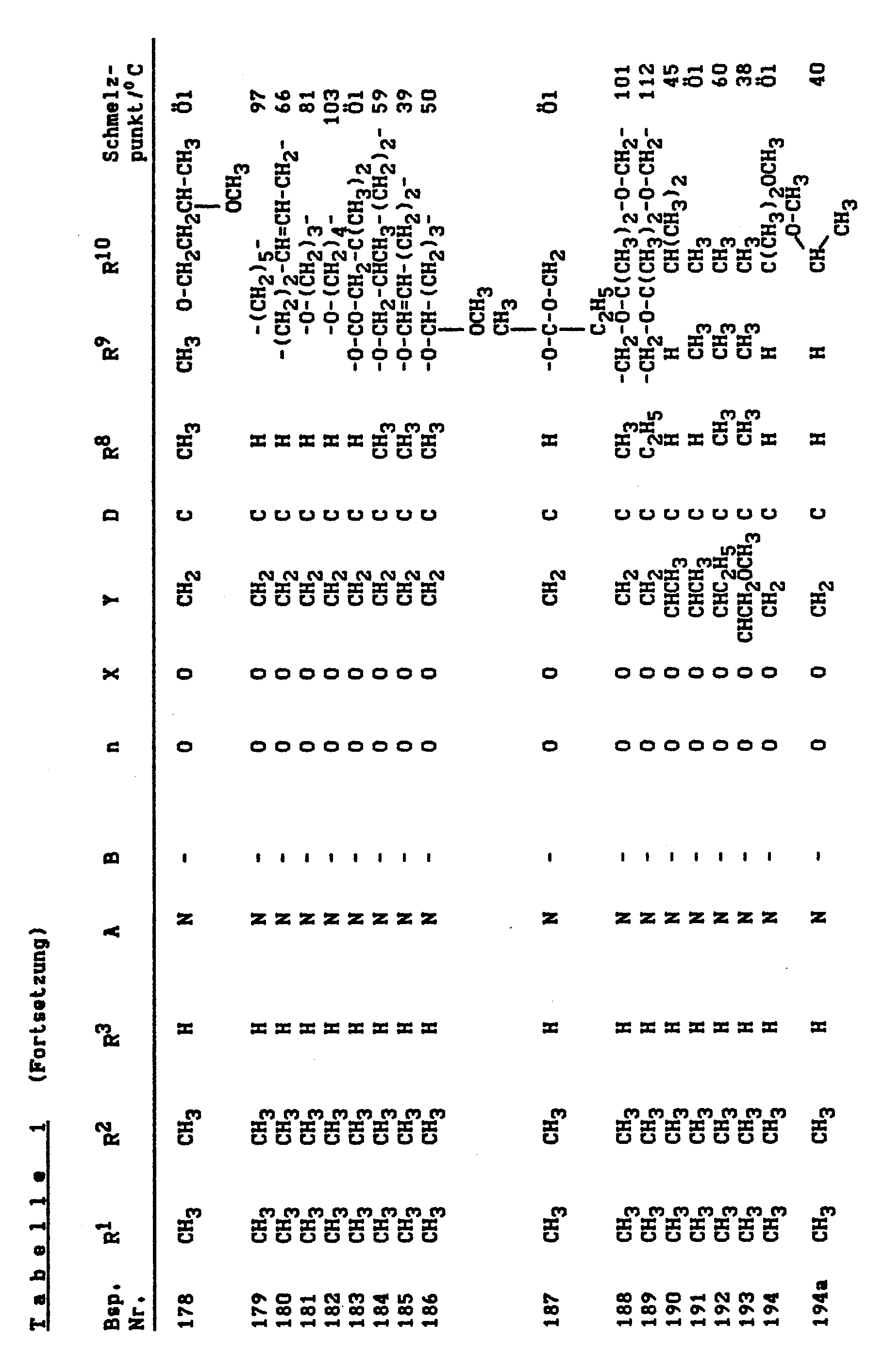

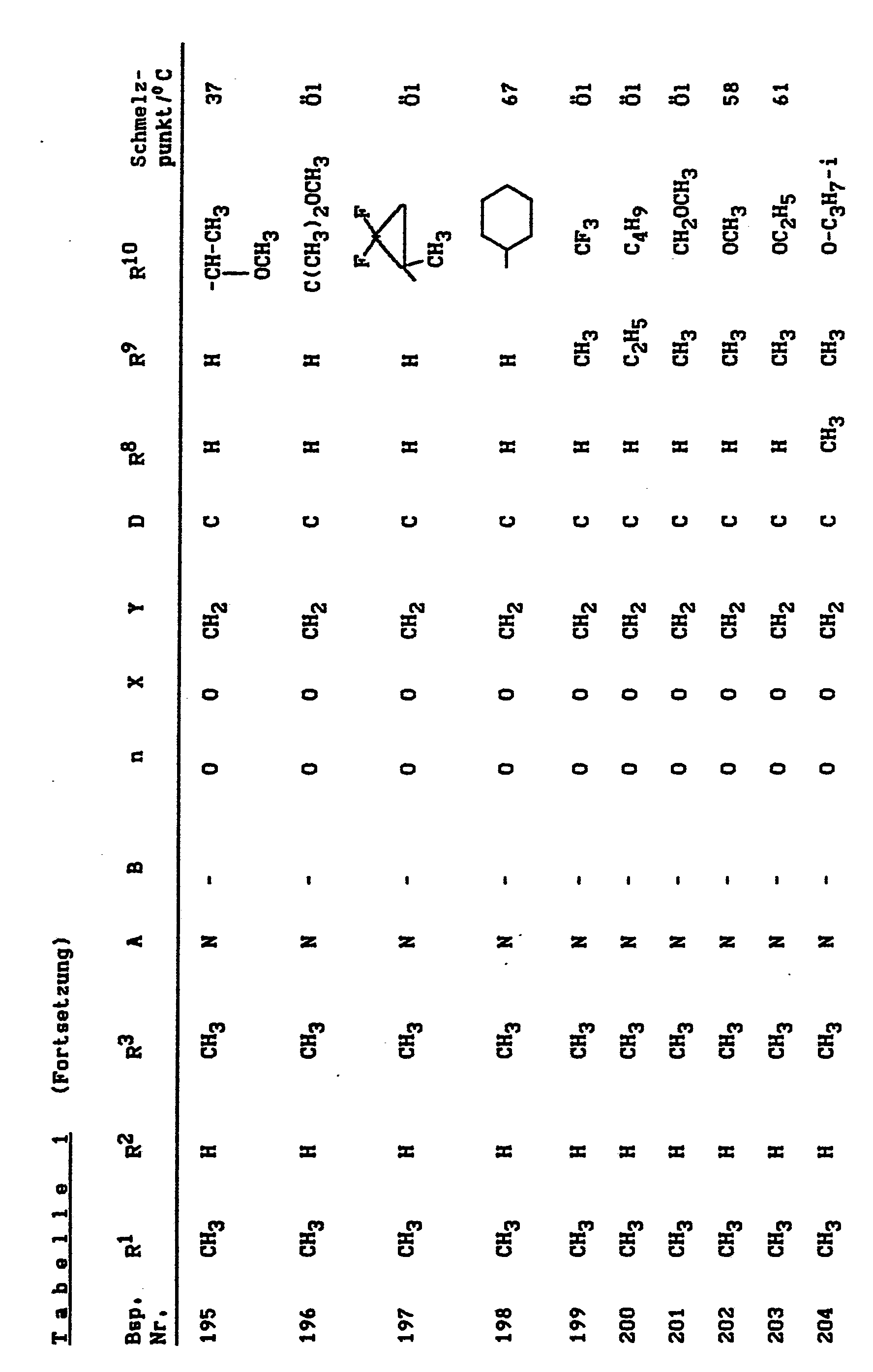

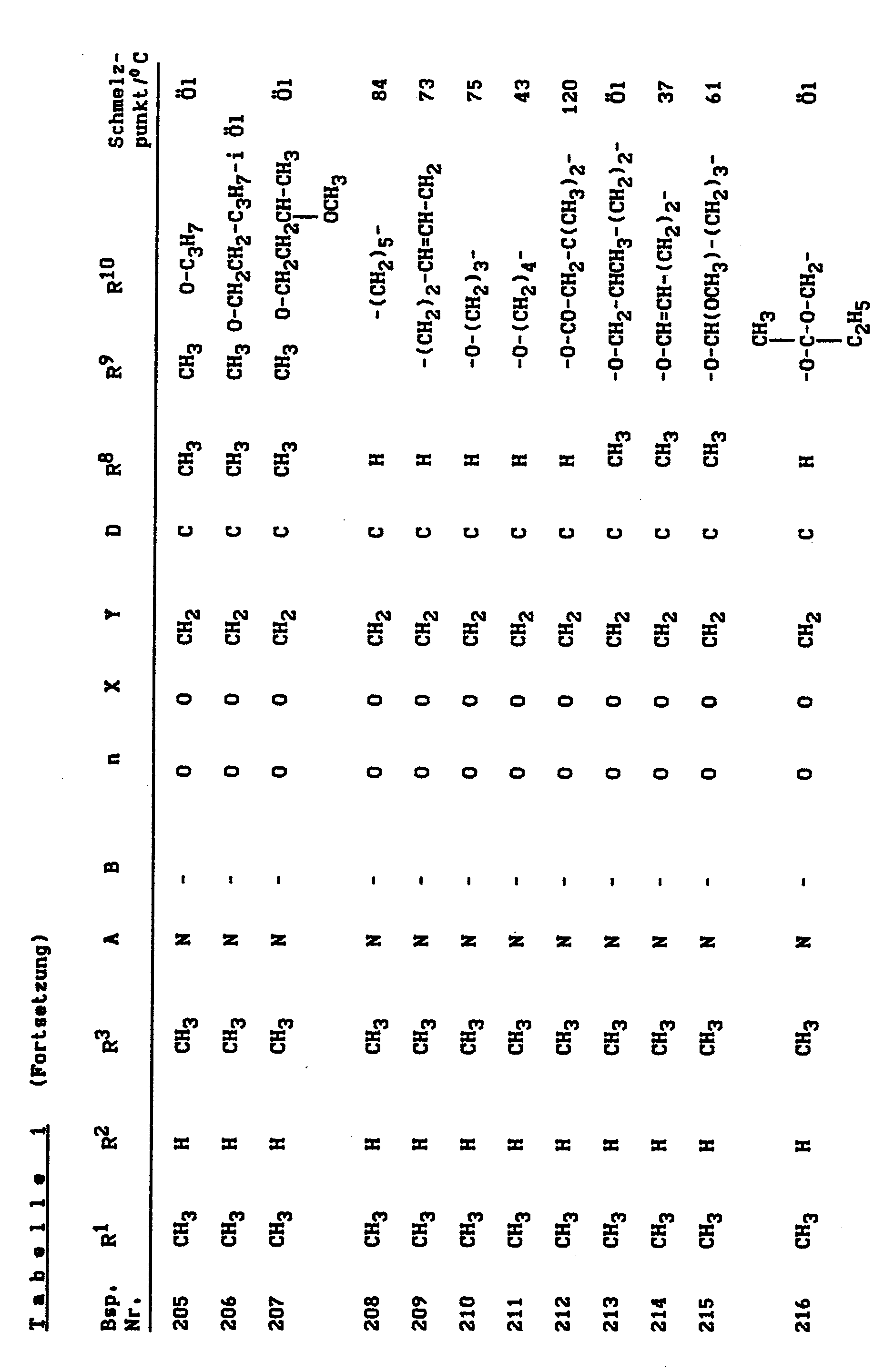

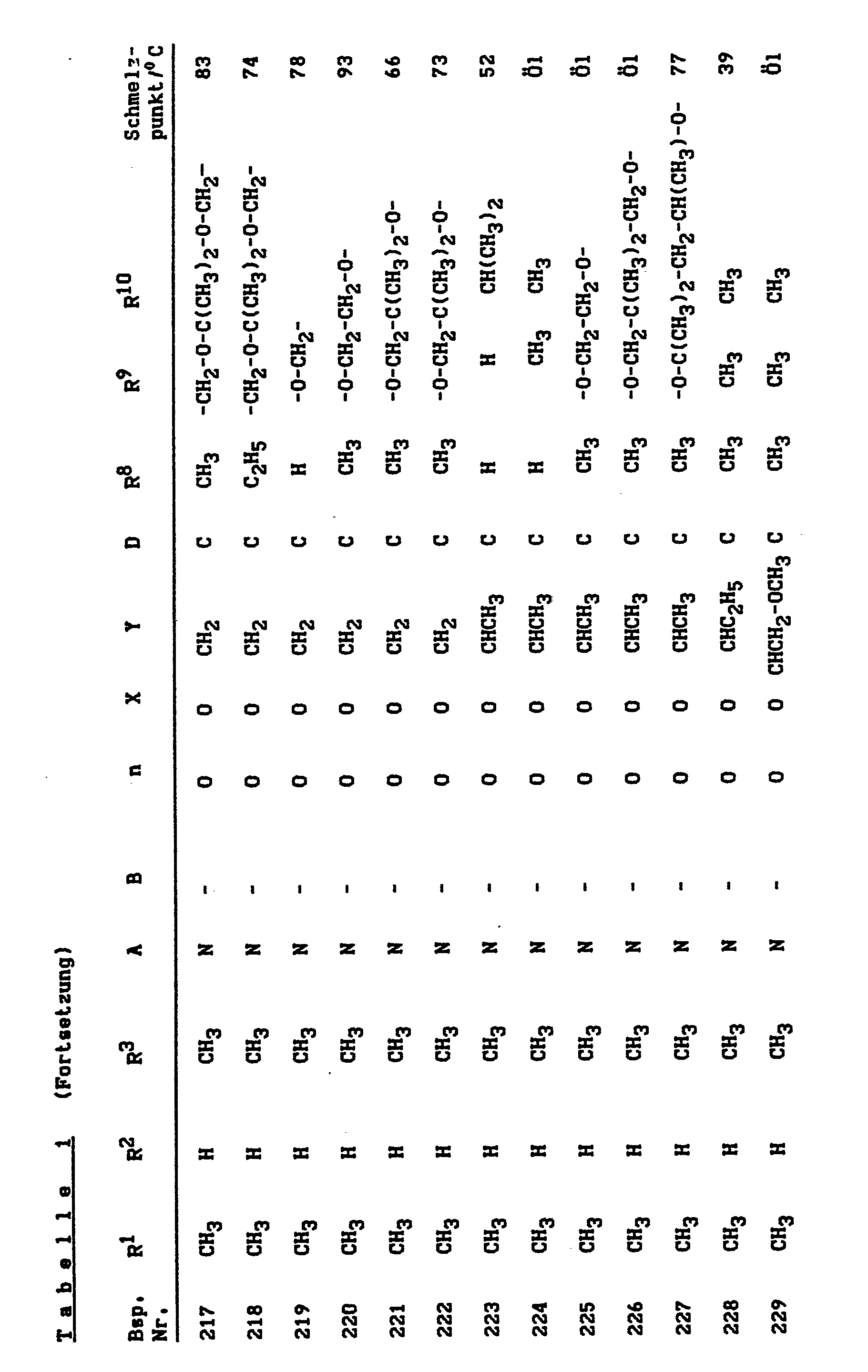

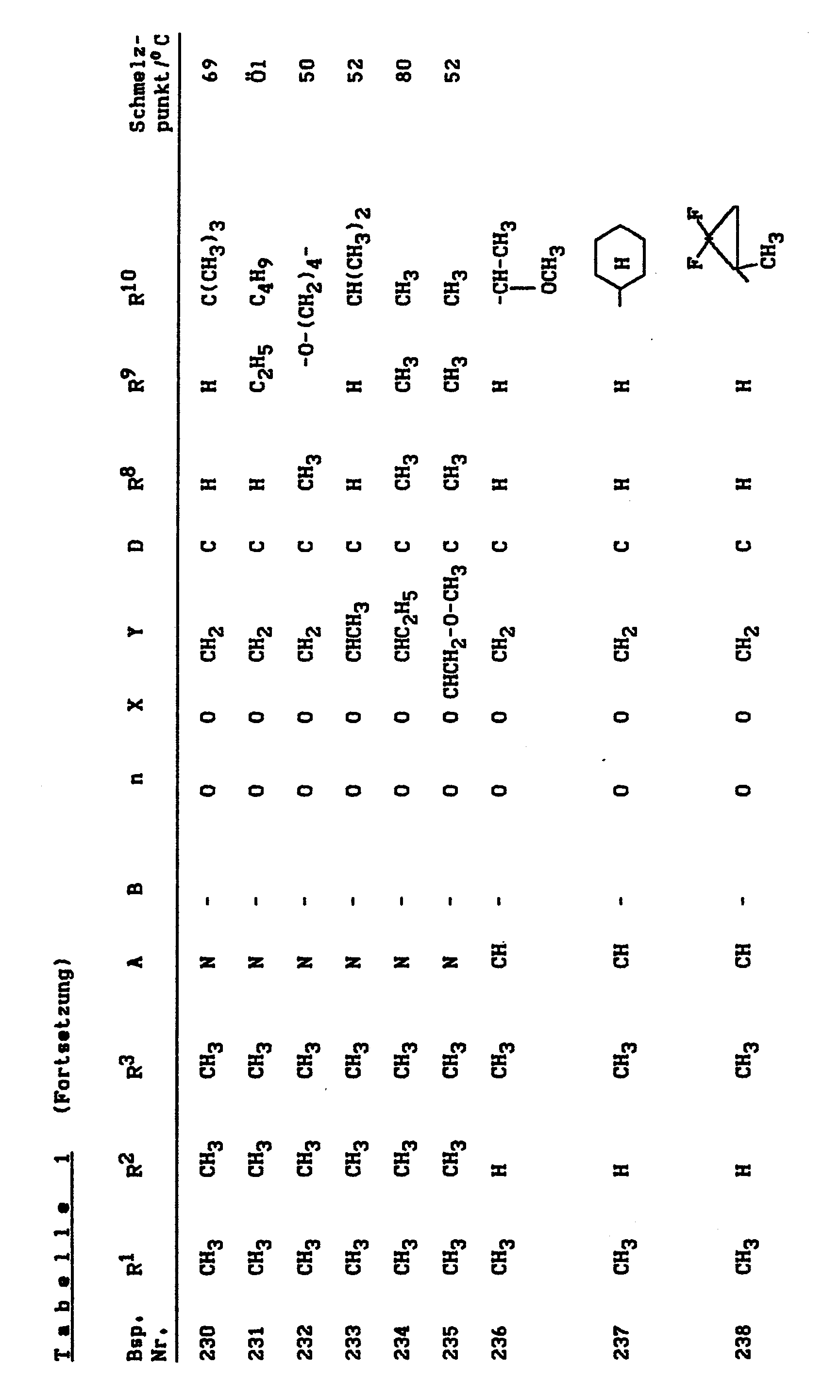

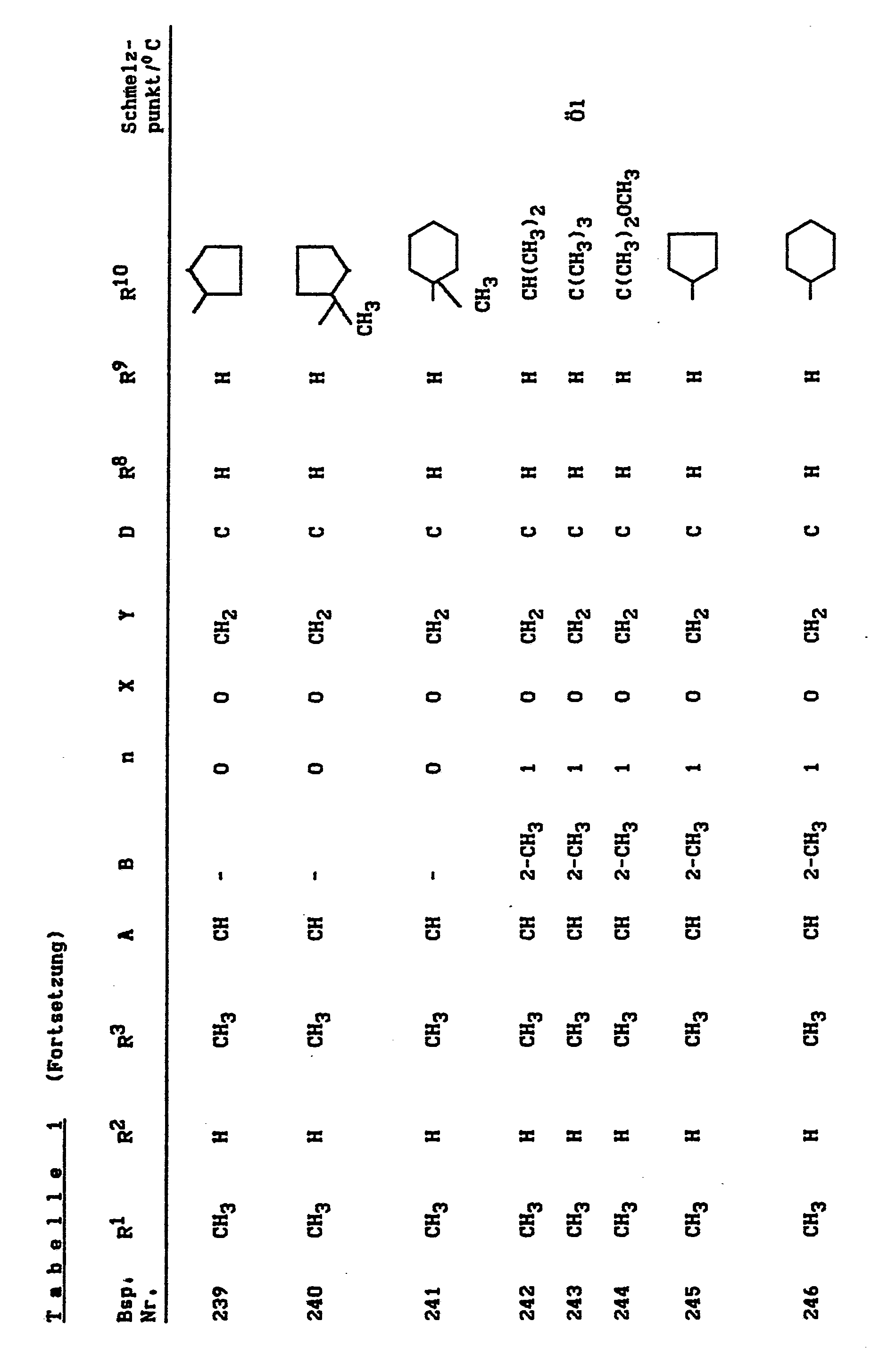

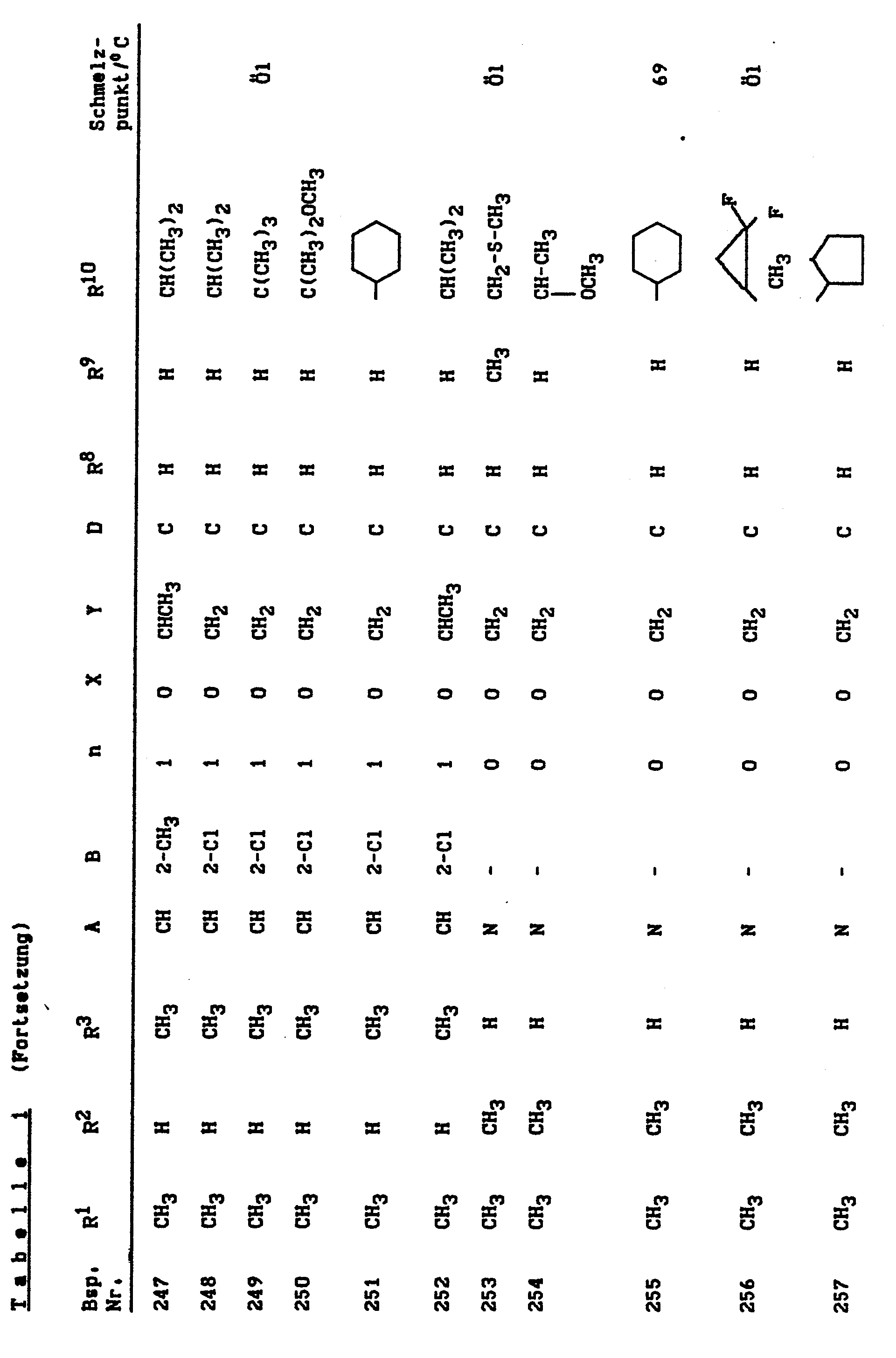

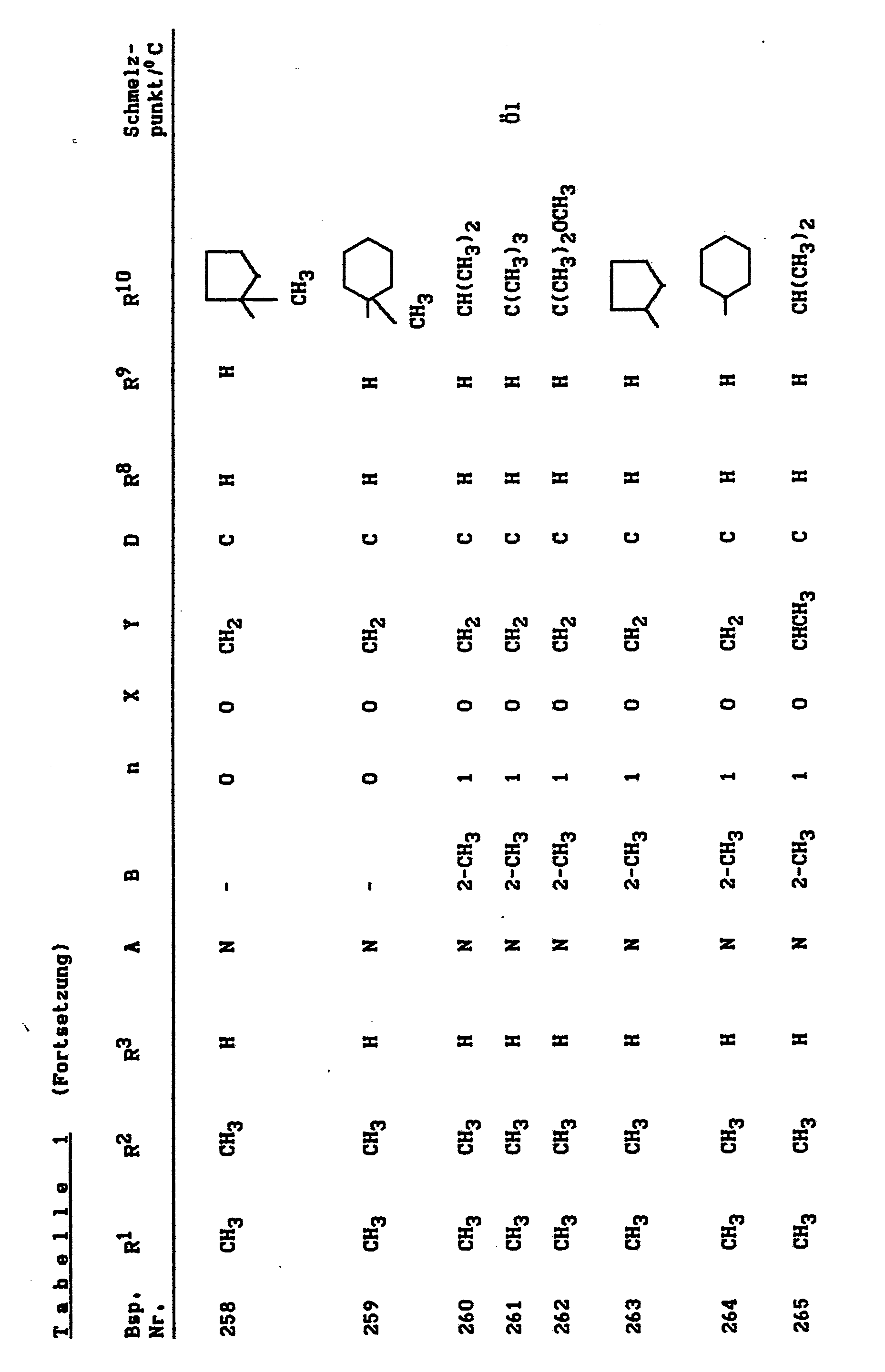

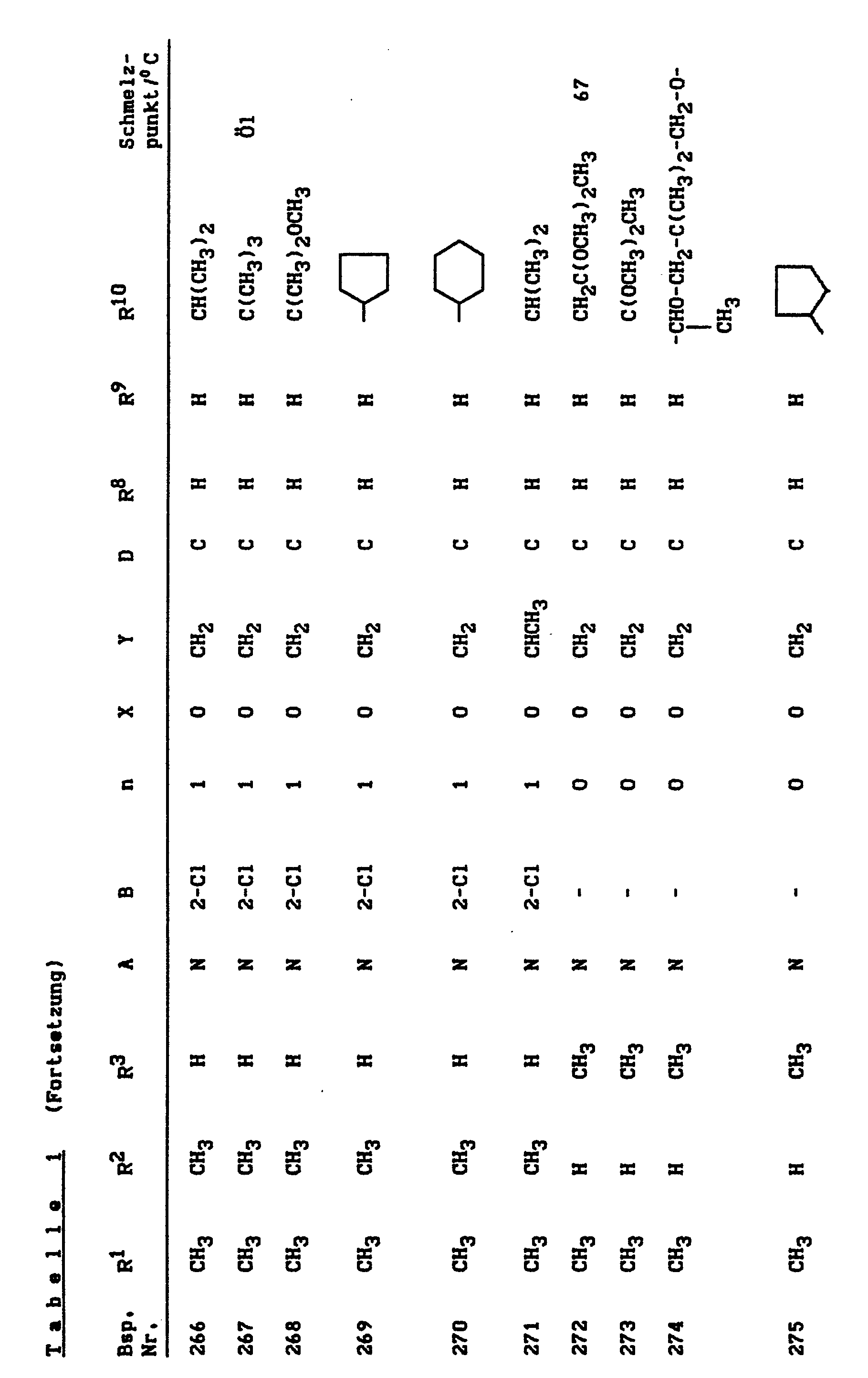

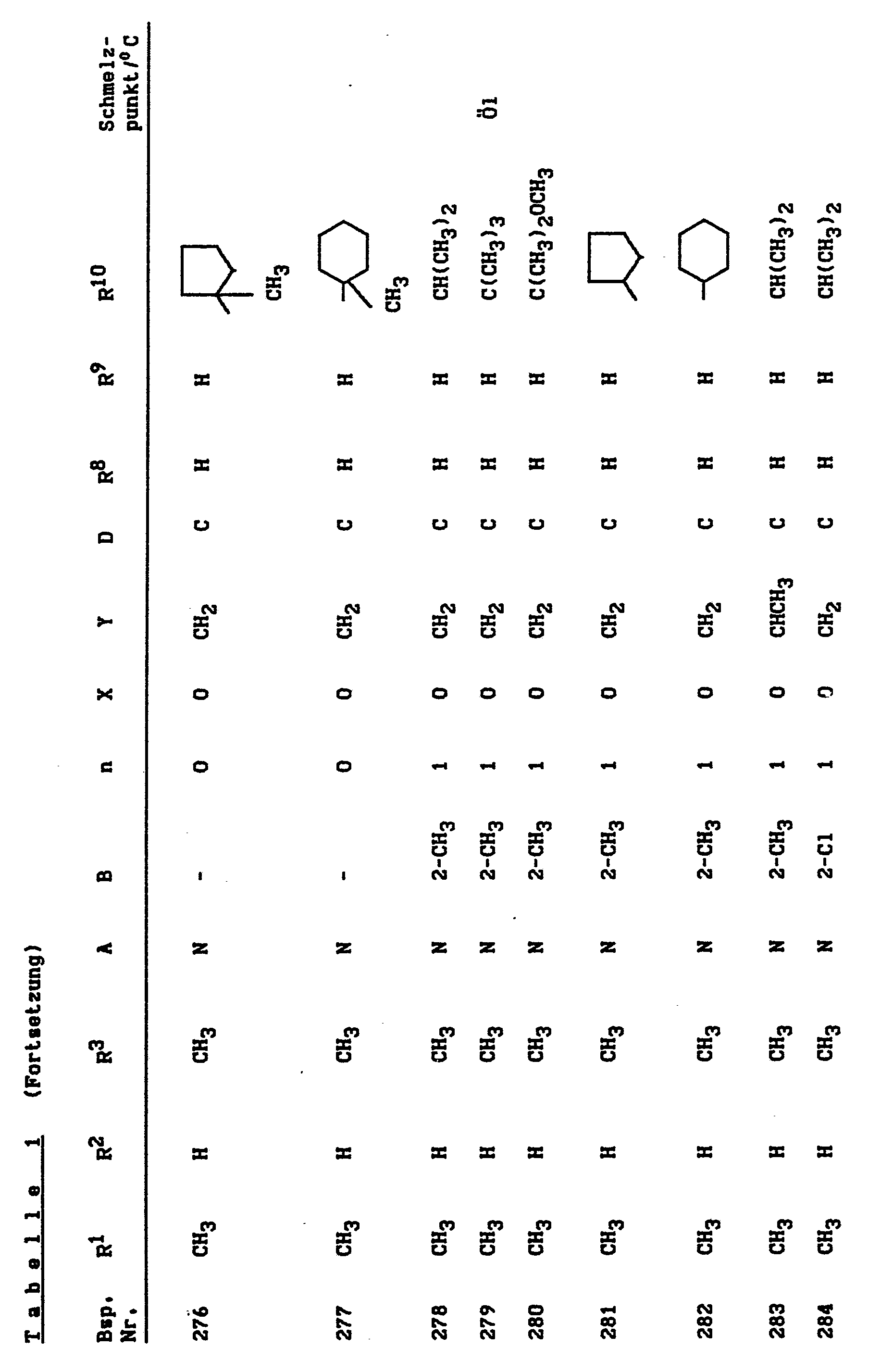

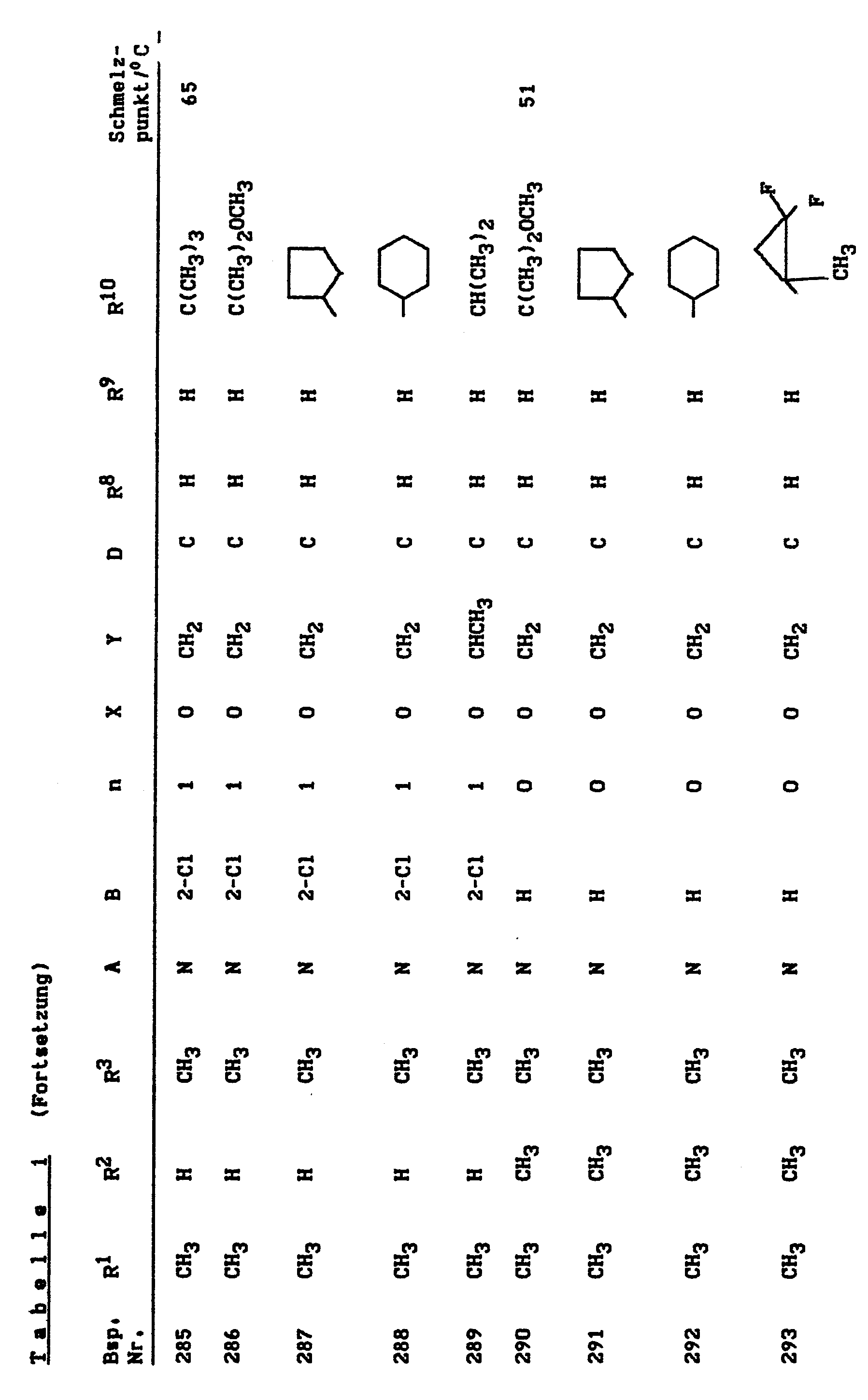

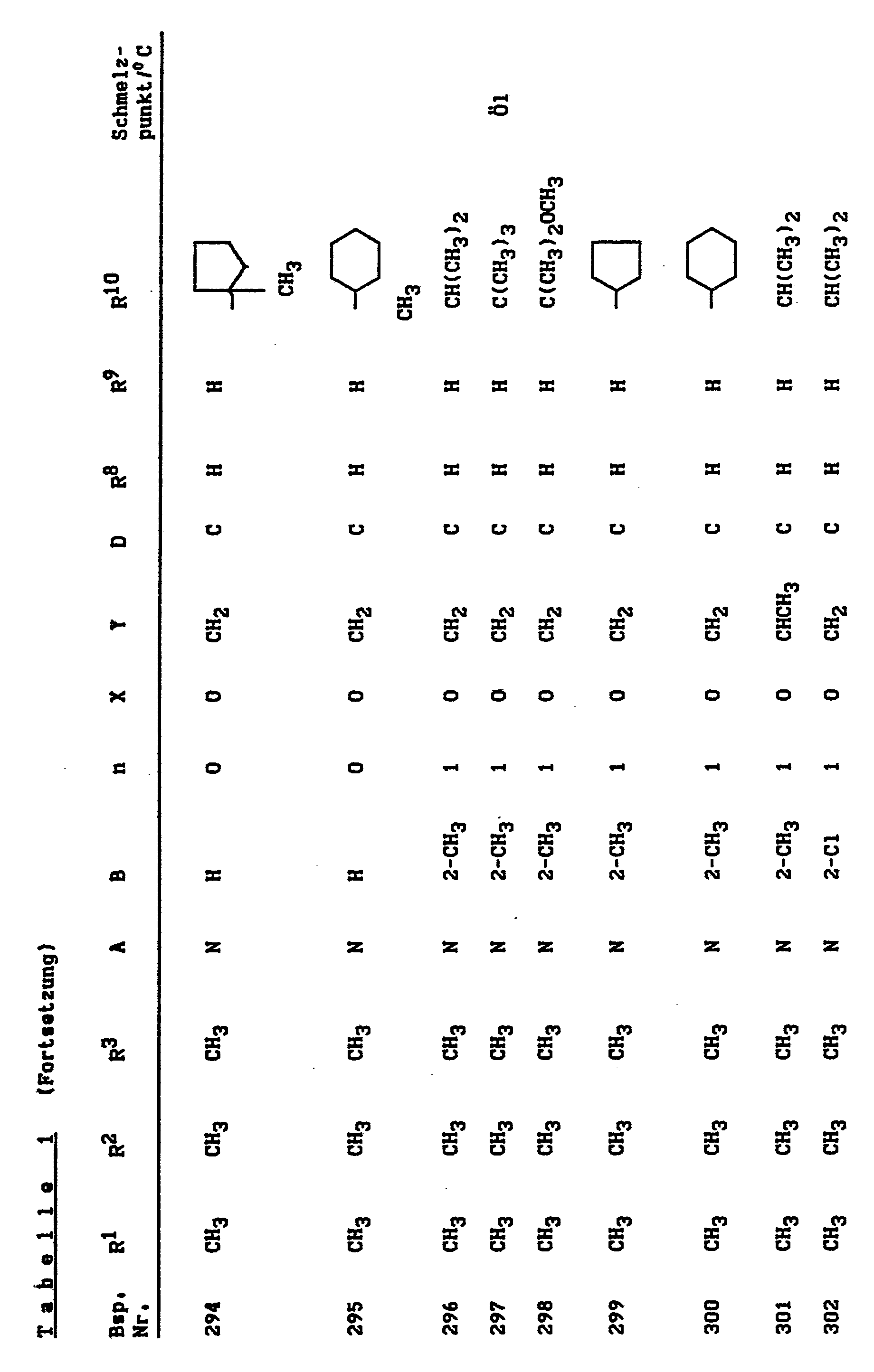

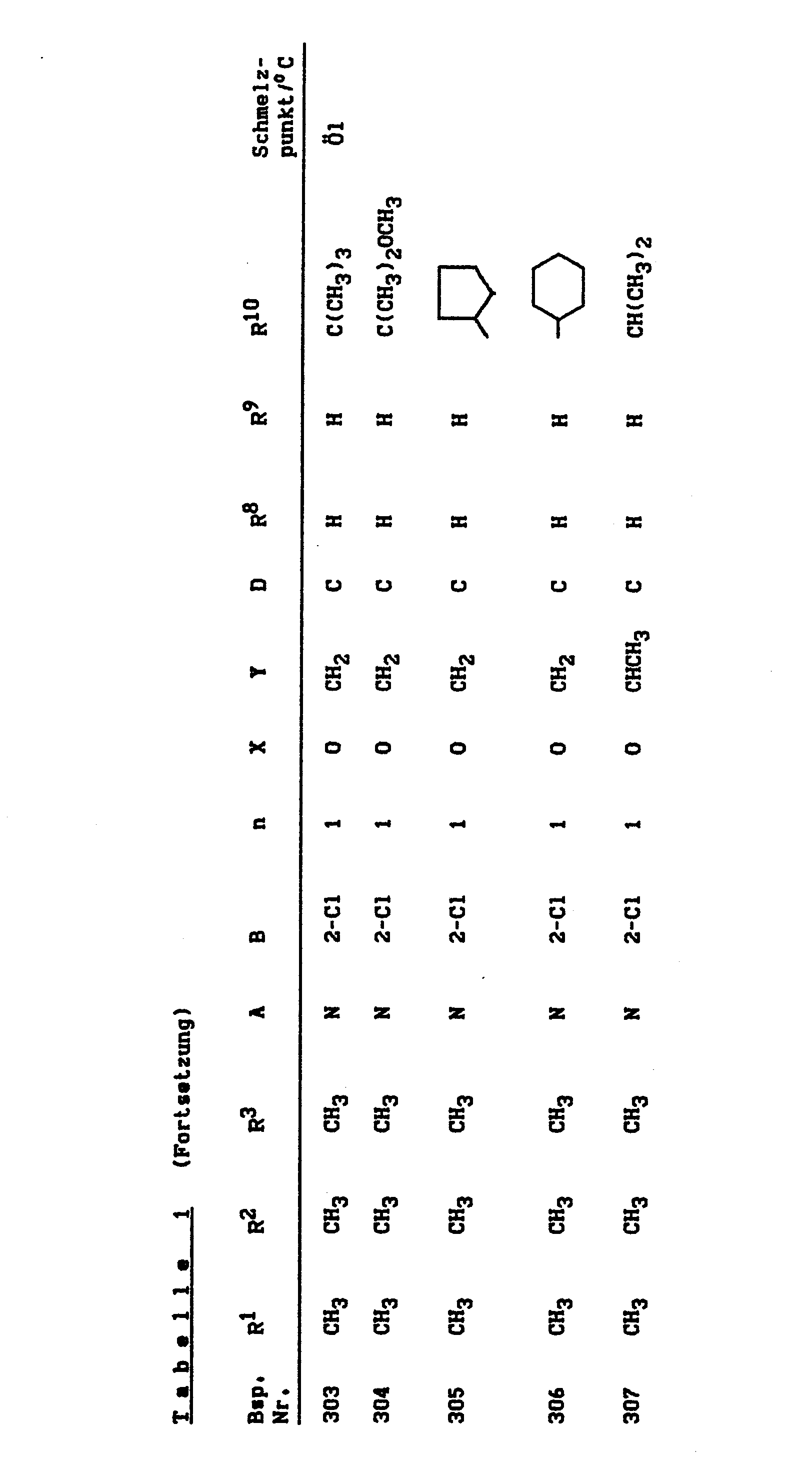

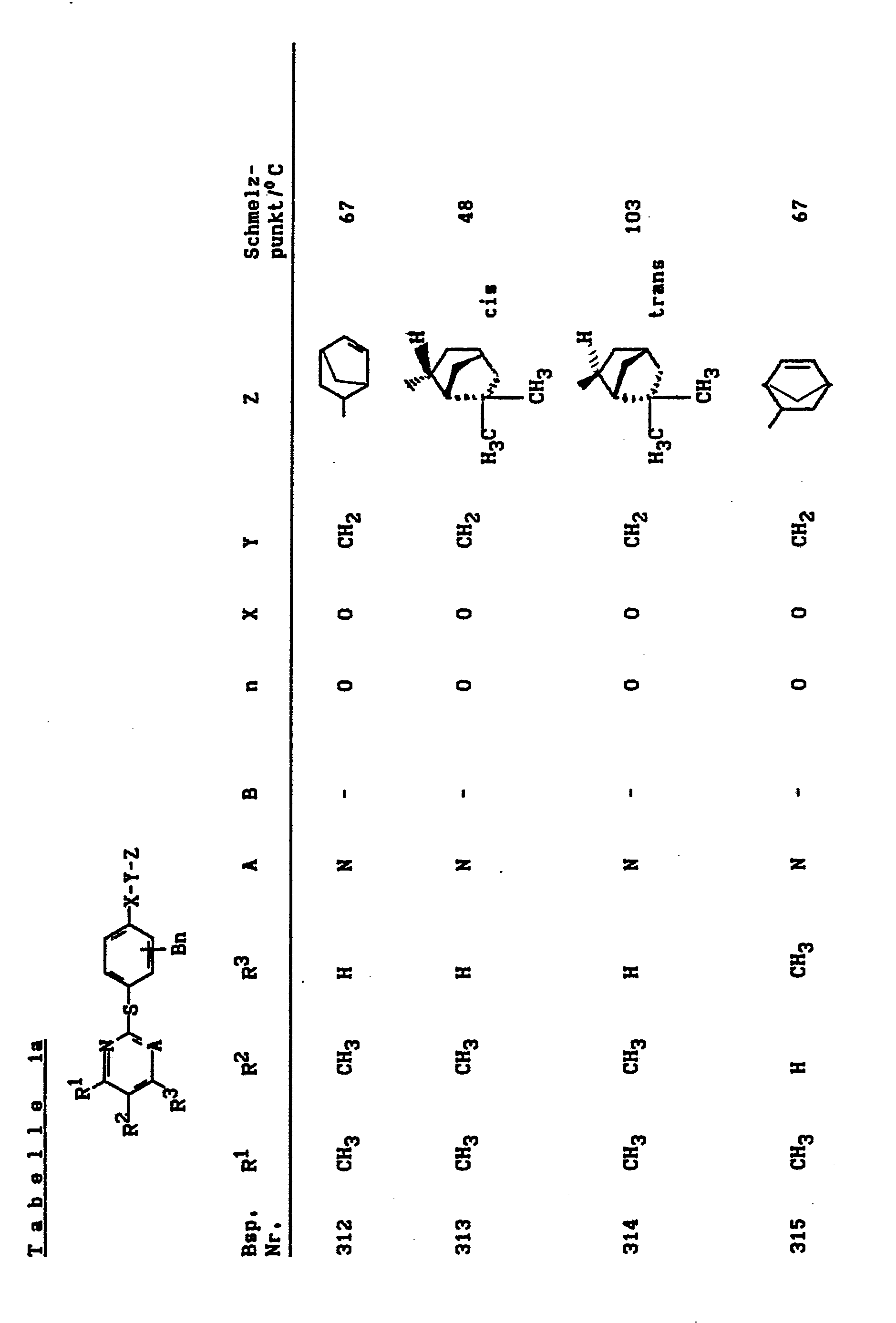

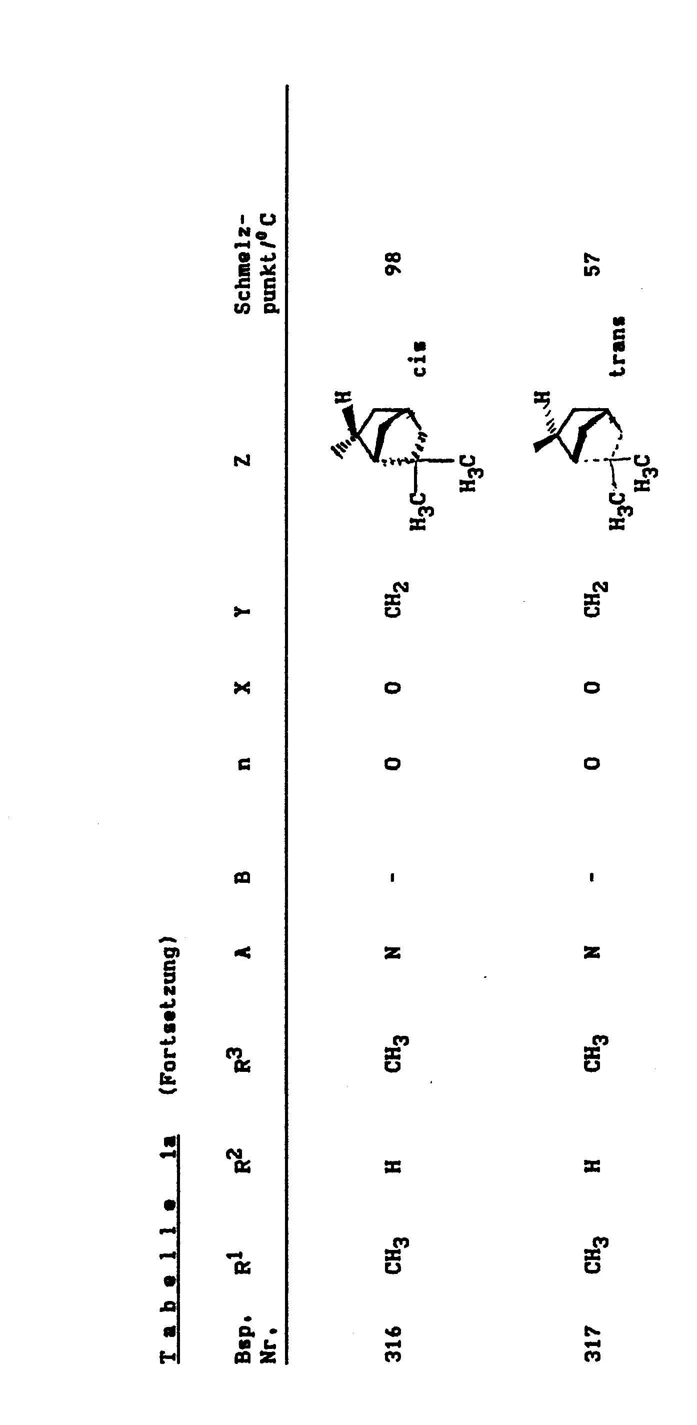

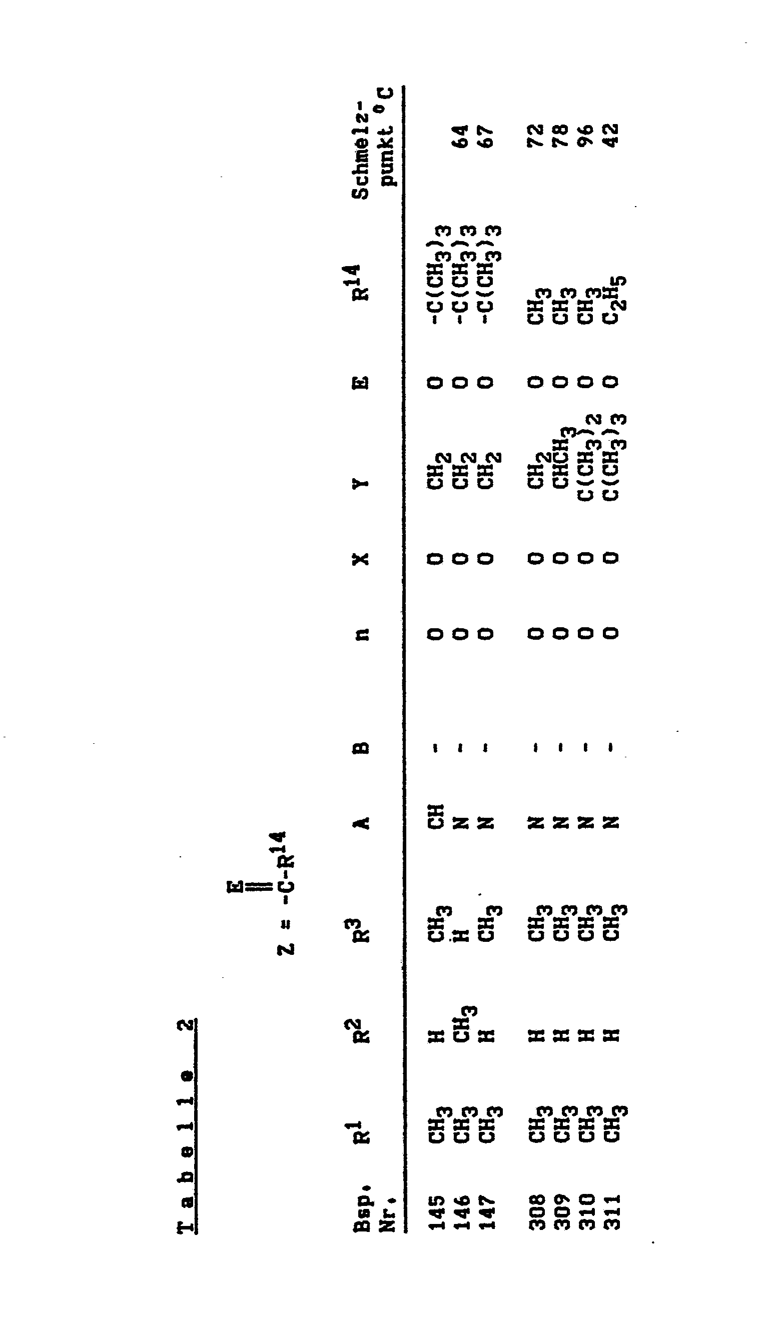

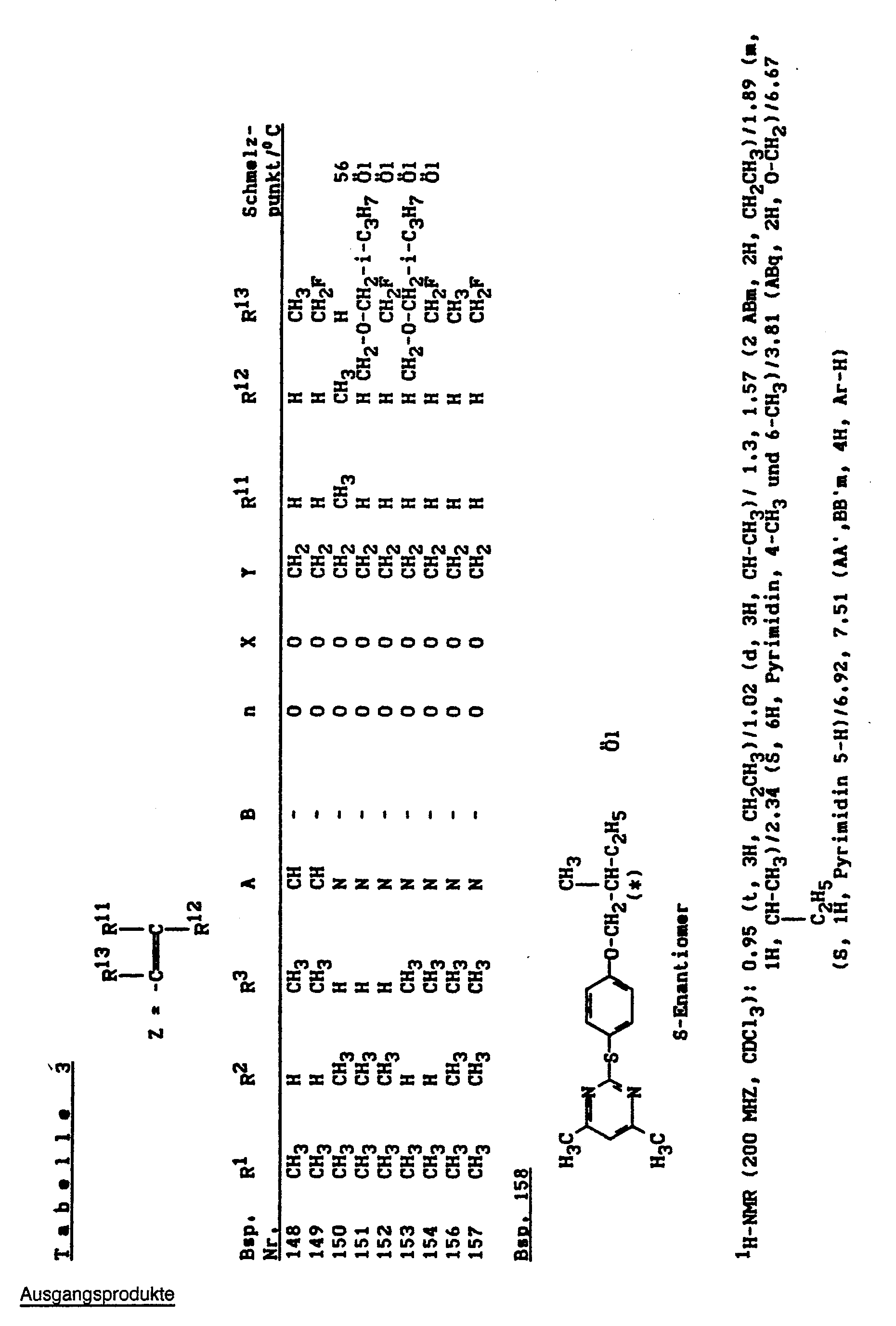

### Beispiel 11₋1

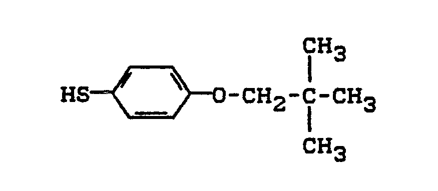

Verfahren (D)

71,4 g (0,4 Mol) p-Neopentyloxy-anilin werden in 150 ml (0,9 Mol) halbkonzentrierter Salzsäure suspendiert und bei 0° C werden 27,6 g (0,4 Mol) Natriumnitrit in 170 ml Wasser zugetropft. Die Diazoniumsalzlösung wird zu einer auf 70° C erwärmten Mischung aus 64 g (0,4 Mol) Kaliumxanthogenat und 53 g (0,5 Mol) Natriumcarbonat in 450 ml Wasser getropft und anschließend 1 Stunde bei 70° C nachgerührt. Das entstandene Öl wird in Ether aufgenommen und eingeengt. Der Rückstand wird mit 56 g (1 Mol) Kaliumhydroxid in 400 ml 80 %igem Ethanol aufgenommen und 2 Stunden unter Stickstoffatmosphäre unter Rückfluß erhitzt. Nach dem Abdestillieren des Lösungsmittels wird der Rückstand unter Eiskühlung mit 600 ml Wasser versetzt und anschließend wird die Reaktionsmischung filtriert. Das Filtrat wird mit 125 ml 20 %iger Schwefelsäure auf pH 2 gebracht. Das entstandene Öl wird in Ether aufgenommen, getrocknet, eingeengt und im Hoch vakuum destilliert. Man erhält 39,2 g (50 % der Theorie) 4- neopentyloxy-thiophenol vom Siedepunkt 73-75° C/0,2 mbar mit einem gaschromatographisch bestimmten Gehalt von 97 %.

### Beispiel 11-1

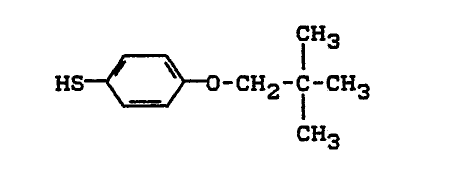

Verfahren (E)

60 g (0,9 g-atom) Zinkstaub werden in 70 ml Ethanol zum Sieden erhitzt und 5 g (0,02 mol) p-Neopentyloxy-benzolsulfochlorid sowie 1 ml konzentrierte Salzsäure zum Starten der Reaktion zugefügt. Dann wird die Reaktionsmischung durch weiteres Eintragen von 55,4 g (0,21 mol) p-Neopentyloxybenzolsulfochorid am Sieden gehalten und noch 10 Minuten am Rückfluß erhitzt. Nach Zugabe von 105 ml konzentrierter Salzsäure wird erneut 3 Stunden unter Rückfluß erhitzt. Die Reaktionsmischung wird mit 200 ml Petrolether verrührt. die organische Phase abgetrennt, über Natriumsulfat getrocknet, eingeengt und destilliert. Man erhält 23,9 g (53 % der Theorie) 4-Neopentyloxy-thiophenol mit einem Siedepunkt von 57° C/0,07 mbar.

Analog Beispiel (11-1) und den allgemeinen Angaben zur Herstellung erhält man die folgenden Verbindungen der Formel (II):

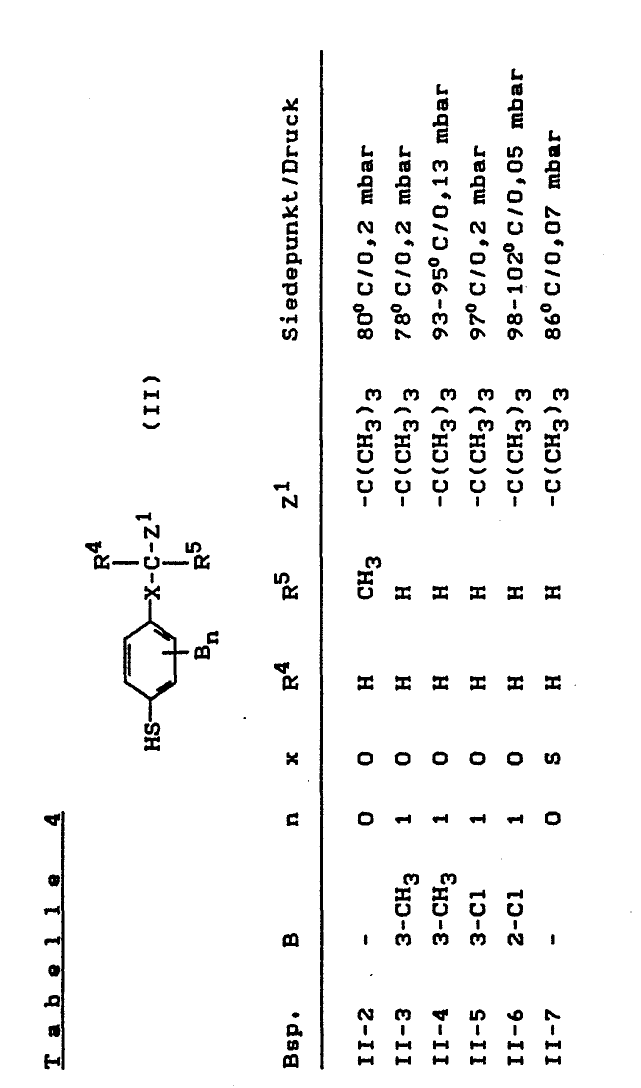

### Beispiel VIII-1

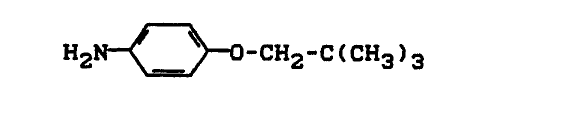

276 g (1,32 mol) 4-Neopentyloxy-nitrobenzol werden in 1300 ml Methanol vorgelegt und mit 30 g Raney-Nickel bei 70° C und 80 bar hydriert. Nach Aufnahme der berechneten Wasserstoffmenge wird die Reaktionsmischung filtriert, das Filtrat eingeengt und der Rückstand im Vakuum destilliert. Man erhält 202 g (85 % der Theorie) 4-Neopentyloxy-anilin mit einem Siedepunkt von 78° C/0,07 mbar une einem Schmelzpunkt von 36-39° C.

Analog Beispiel VIII-1 lassen sich die folgenden Verbindungen erhalten:

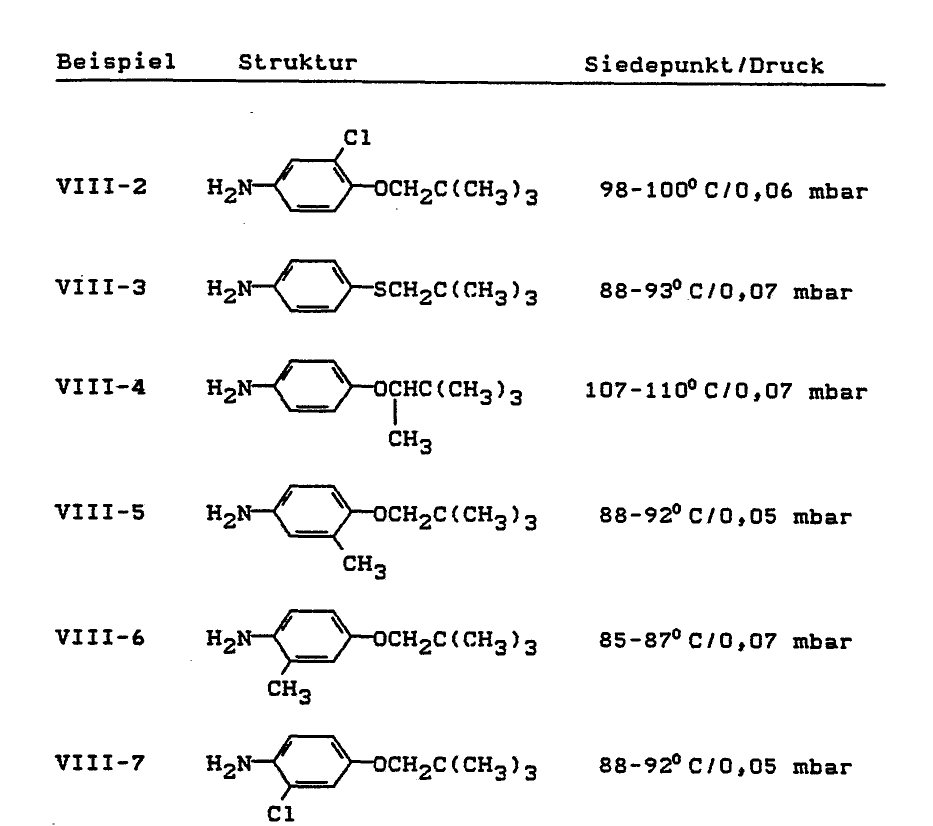

### Beispiel X₋1

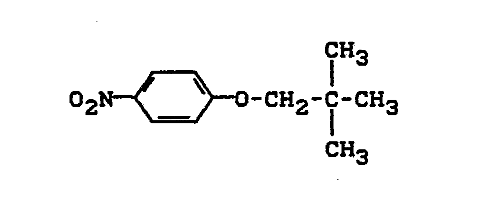

Zu einer Lösung von 92,4 g (1,05 Mol) Neopentanol und 156,5 g (1 Mol) 4-Chlor-nitrobenzol in 800 ml Dimethylsulfoxid werden bei Raumtemperatur 64,5 g (1,15 Mol) gepulvertes Kaliumhydroxid eingetragen. Anschließend wird die Reaktionsmischung 3 Stunden bei 80° C nachgerührt. Dann wird sie auf 31 Eiswasser gegossen. Das Produkt wird mit Methylenchlorid extrahiert. Die organische Phase wird 2 x mit Wasser gewaschen, mit Natriumsulfat getrocknet und eingeengt. Nach Destillation im Vakuum erhält man 127,3 g (59 % der Theorie) 4-Neopentyloxy-nitrobenzol mit einem Siedepunkt von 102-110° C/0,05 mbar und einem Schmelzpunkt von 38-40° C.

Analog Beispiel X-1 erhält man die folgenden Verbindungen:

### Beispiel X₋2

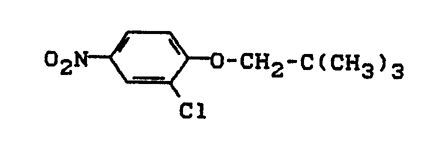

Schmelzpunkt: 73-75° C

### Beispiel X₋3

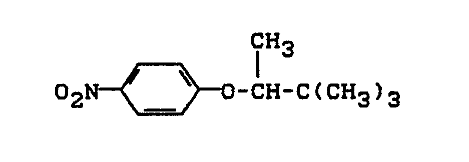

Siedepunkt 110° C/0,26 mbar

### Beisiel IX-1

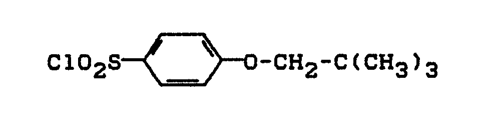

98,6 g (0,6 mol) Neopentyloxybenzol werdenin 200 ml Chloroform gelöst und bei 0-5° C 167,8 g (1,44 mol) Chlorsulfonsäure zugetropft. Nach Abklingen der Gasentwicklung rührt man bei Raumtemperatur, bis diese vollständig beendet ist. Dann gießt man die Reaktionsmischung auf 500 g Eis, trennt die Chloroformphase ab, wäscht diese mit Wasser, trocknet mit Natriumsulfat und engt ein. Es verbleiben 87 g einer violetten Masse, die durch Aufnehmen in 600 ml Petrolether bei 50° C und filtrieren über eine 6 cm Kieselgelsäule gereinigt wird.

Das Filtrat engt man auf ca. 200 ml ein und kühlt auf -20° C ab. Durch Absaugen der Kristalle erhält man 68,3 g (43 % der Theorie) 4-Neopentylox-benzolsulfochlorid vom Schmelzpunkt 80-82° C.

Analog Beispiel IX-1 erhält man:

### Beispiel IX-2

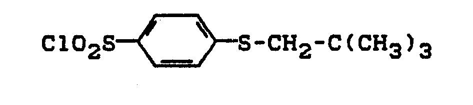

Schmelzpunkt: 88-91 ° C.

Ausgangsprodukt zu Beispiel IX-2:

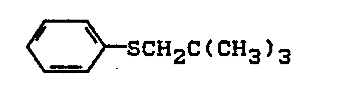

Neopentyl-phenyl-thioether wird in Analogie zur Herstellung des Ausgangsproduktes zu Beispiel VII-1 aus Thiophenol erhalten. Ausbeute 85 %, Siedepunkt 92-96° C/6 mbar. •

### Beispiel IV-1

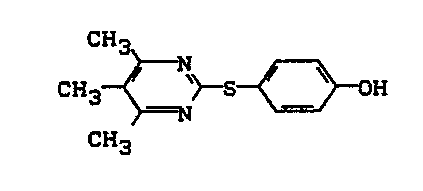

42,2 g (0,64 mol) ca. 85 %iges gepulvertes Kaliumhydroxid werden in 384 ml N-Methylpyrrolidin-2-on vorgelegt und unter Stickstoff bei 60° C 80,6 g (0,64 mol) 4-Mercaptophenol zugetropft. Dann werden bei 100° C 100 g (0,64 mol) 2-Chlor-4,5,6-trimethylpyrimidin in 128 ml N-methyl-pyrrolidin-2-on zugetropft. Die Reaktionsmischung wird 3 Stunden bei 100° C nachgerührt, bis dünnschichtchromatographisch ein vollständiger Umsatz festzustellen ist. Bei 10 mbar werden etwa 2/3 des Lösungsmittels abdestilliert und der Rückstand in 21 Eisenwasser eingerührt. Das Produkt wird abgesaugt, sorgfältig mit Wasser gewaschen, bei 50° C/10 mbar getrocknet und aus Methanol umkristallisiert. Man erhält 128 g (81 % der Theorie) 2-(p-Hydroxphenylthio)-4,5,6-trimethylpyrimidin mit einem Schmelzpunkt von 182-186° C.

Analog zu Beispiel IV-1 erhält man die folgenden Verbindungen:

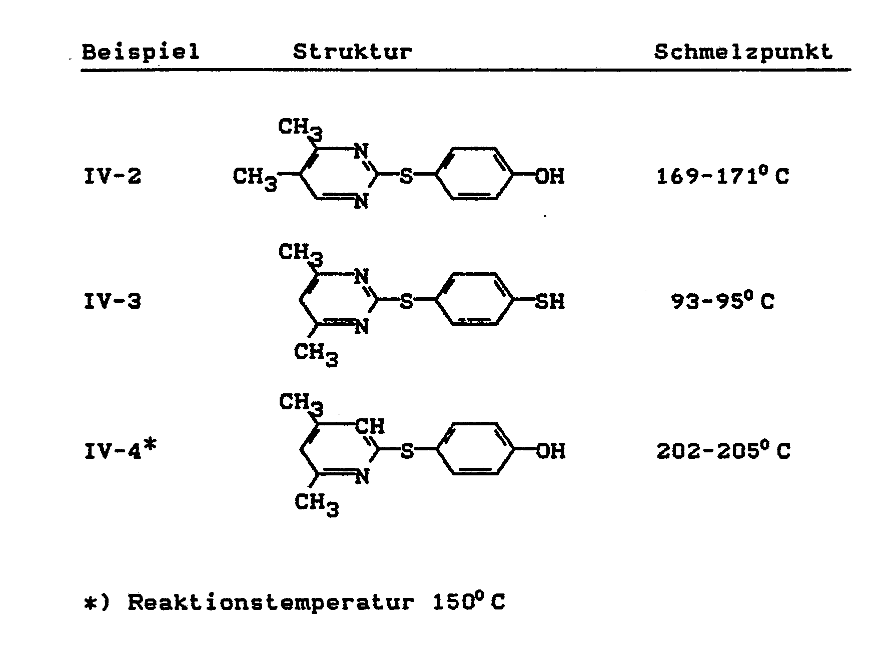

### Beispiel VII-1

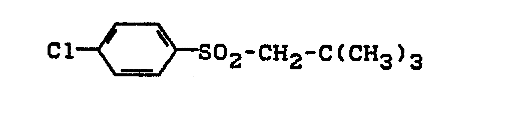

42,9 g (0,2 Mol) p-Neopentylthio-chlorbenzol werden in 100 ml Aceton gelöst, mit 2 g Tetrabutylammoniumchlorid versetzt und 100 mg (NH₄)₂ Mo0₄ in 200 ml Wasser zugefügt. Nach Zugabe von 50 ml (0,44 Mol) ca. 30 %ige H₂0₂-Lösung wird die Reaktionsmischung 24 Stunden unter Rückfluß erhitzt. Anschließend wird das Aceton abdestilliert und der Rückstand abgesaugt. Der Rückstand wird mit Cyclohexan/Essigester 1:1 chromatographiert. Nach Kristallisation aus n-Hexan erhält man 25,2 g (51 % der Theorie) p-Neopentylsulfonyl-chlorbenzol vom Schmelzpunkt 67° C.

### Ausgangsprodukt für Beispiel VII-1

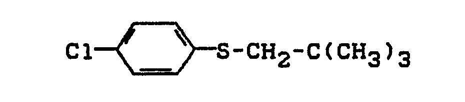

20,1 g (0,31 mol) ca. 85 %iges gepulvertes Kaliumhydroxid werden in 200 ml Glykolmonomethylether vorgelegt und eine Lösung von 43,3 g (0,3 mol) p-Chlorthiophenol in 100 ml Glykolmonomethylether wird unter Stickstoff zugetropft. Es wird 10 Minuten bei Raumtemperatur nachgerührt, dann werden 72,4 g (0.3 mol) p-Toluolsulfonsäureneopentylester als Schmelze zugetropft. Die Reaktionsmischung wird langsam auf Siedetemperatur erhitzt und weitere 5 Stunden unter Rückfluß erhitzt. Dann wird auf 21 Eiswasser gegossen, das Produkt in Petrolether aufgenommen, die organische Phase mit Natriumsulfat getrocknet eingeengt und im Vakuum destilliert. Man erhält 52,3 g (81 % der Theorie) 4-Chlorphenyl-neopentylthioether mit einem Siedepunkt von 62-63° C/ 0,03 mbar.

### Anwendungsbeispiele

### Beispiel A

### Post-emergence-Test

Lösungsmittel: 5 Gewichtsteile Aceton Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung sprizt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewält, daß in 2000 I Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß der Herstellungsbeispiele 1, 4, 6, 11, 39, 45, 48, 57, 58, 69, 71, 73, 85, 89, 93, 96, 99, 107, 109, 120, und 143 eine sehr gute herbizide Wirkung gegen mono-und dikotyle Unkräuter bei sehr guter Nutzpflanzenverträglichkeit, insbesondere für Weizen.

Weiterhin zeigt in diesem Test beispielsweise die Verbindung gemäß Herstellungsbeispiel (2) insbesondere in Zuckerrüben eine selektive Wirkung gegen Unkräuter wie beispielsweise Amaranthus, Galinsoga oder Polygonum.

### Beispiel B

### Entlaubung und Austrocknung der Blätter bei Baumwolle

Lösungsmittel: 30 Gewichtsteile Dimethylformamid Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:
0 kein Austrocknen der Blätter, kein Blattfall
+ leichtes Austrocknen der Blätter, geringer Blattfall
+ + starkes Austrocknen der Blätter, starker Blattfall
+ + + sehr starkes Austrocknen der Blätter, sehr starker Blattfall

Eine deutliche Wirksamkeit im Vergleich zur unbehandelten Kontrolle zeigen in diesem Test beispielsweise die Verbindungen gemäß den Herstellungsbeispielen 4, 6 und 89.

## Patentansprüche

1. 2-Pyri(mi)dylthio-arylether der Formel (I) 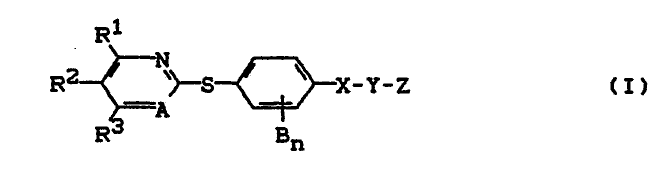 in welcher
R¹, R²⁻ und R³ unabhängig voneinander jeweils für Wasserstoff oder Alkyl stehen,
A für eine -CH-Gruppe oder ein Stickstoffatom steht,
B für Halogen, Nitro, Cyano, Amino, Alkyl, Alkoxy, Halogenalkyl oder Halogenalkoxy steht, wobei B gleich oder verschieden sein kann,
n für eine Zahl 0, 1, 2, 3 oder 4 steht,
X für Sauerstoff, Schwefel, Sulfoxy oder Sulfonyl steht,
Y für eine 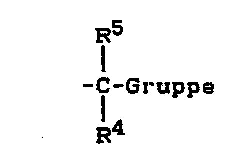 oder eine 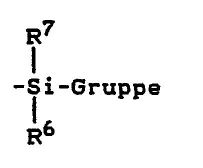 steht wobei
R⁴ und R⁵ unabhängig voneinander jeweils für Wasserstoff, Alkyl, Alkoxyalkyl oder Halogenalkyl steht,
R⁶ und R⁷ jeweils für Alkyl stehen,
Z für einen Rest 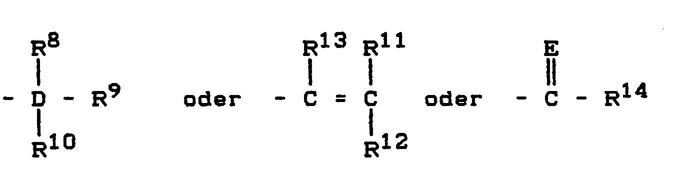 steht, wobei
D für Kohlenstoff oder Silicium steht,
R⁸, R¹3 und R¹⁴ unabhängig voneinander für Wasserstoff, Halogen, Alkyl oder Halogenalkyl stehen,
R⁹ für Wasserstoff, Halogen, Alkyl, Alkoxy, Alkoxyalkyl, Alkylthio oder Halogenalkyl steht,
R¹⁰ für Wasserstoff, Halogen, Hydroxy, Alkyl, Alkoxy, Alkoxyalkyl, Alkylthio, Alkylthioalkyl, Nitriloalkyl, Alkylcarbonyl, Alkoxycarbonyl, Aminocarbonyl, Formyl, Alkenyl, Alkinyl, Halogenalkyl, Halogenalkenyl, Halogenalkinyl, Amino, Alkylamino, Dialkylamino, durch Amino oder Alkylamino substituiertes Alkyl, gegebenenfalls durch Alkyl und/oder Halogen substituiertes Cycloalkyl, offenkettiges oder cyclisches Acetal oder offenkettiges oder cyclisches Thioacetal steht oder
R⁹ und R¹⁰ gemeinsam mit dem Kohlenstoffatom, an das sie gebunden sind, einen 3-8 gliedrigen Ring bilden, der außer Kohlenstoff auch Sauerstoff und/oder Schwefel und/oder Stickstoff als Ringglieder und welcher eine Oxo-Gruppe enthalten kann und der durch Halogen, Alkyl, Alkoxy und/oder Alkoxyalkyl substituiert sein kann und welcher eine oder mehrere Doppelbindungen enthalten kann oder
R⁸, R⁹ und R¹⁰ gemeinsam mit dem Kohlenstoffatom, an das sie gebunden sind, für einen gesättigten oder ungesättigten Rest der Struktur 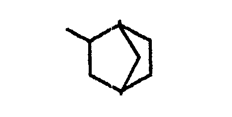 stehen,
E für Sauerstoff oder Schwefel steht und
R¹¹ und R¹² unabhängig voneinander für Wasserstoff, Halogen, Alkyl oder Halogenalkyl stehen,
mit der Maßgabe daß, wenn R¹ und R³ für Methyl, R² für Wasserstoff, A für Stickstoff, X für Sauerstoff und n für 0 stehen, Y und Z zusammen nicht für die Reste:
-C₂H₅, n-C₃H₇, iso-C3H7, n-C₄H₉, iso-C4H9, n-C₅H₁₁, 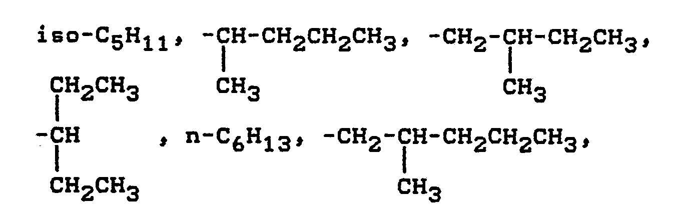 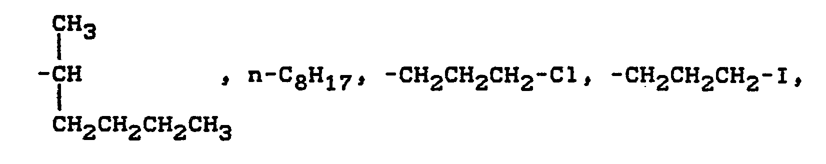 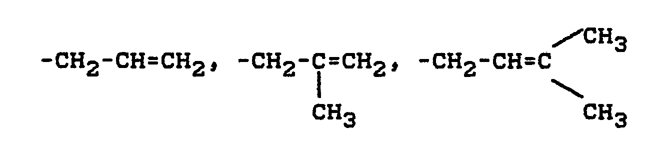 und -(^{C}H₂)₄-CH⁼CH₂ stehen.

2. 2-Pyri(mi)dylthio-arylether der Formel (I) gemäß Anspruch 1, in welcher
R¹, R² und R³ unabhängig voneinander jeweils für Wasserstoff oder C₁-C₄-Alkyl stehen,
A für eine -CH-Gruppe oder ein Stickstoffatom steht,
B für Halogen, Nitro, Cyano, Amino, C₁-C₄-Alkyl, C₁-C₄-Alkoxy, Halogen-C₁-C₄-alkyl oder Fialogen-C₁-C₄- alkoxy steht, wobei B gleich oder verschieden sein kann,
n für eine Zahl 0, 1, 2, 3 oder 4 steht,
X für Sauerstoff, Schwefel, Sulfoxy oder Sulfonyl steht,
Y für eine 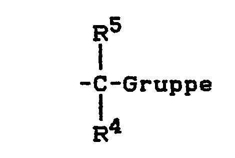 oder eine 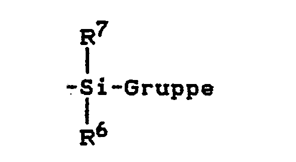 steht, wobei
R⁴ und R⁵ unabhängig voneinander jeweils für Wasserstoff, C₁-C₄-Alkyl, C₁-C₄-Alkoxy-C₁-C₄-alkyl oder Halogen-C₁-C₄-alkyl stehen und
R⁶ und R⁷ unabhängig voneinander jeweils für Methyl oder Ethyl stehen,
Z für einen Rest 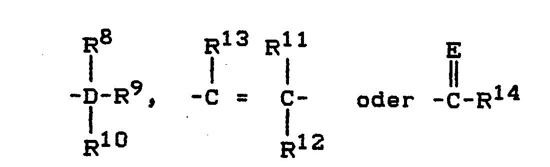 steht, wobei
D für Kohlenstoff oder Silicium steht,
R⁸, R¹3 und R¹⁰ unabhängig voneinander für Wasserstoff, Halogen, C₁-C₆-Alkyl oder Halogen-C₁-C₆-alkyl stehen,
R⁹ für Wasserstoff, Halogen, C₁-C₆-Alkyl, C₁-C₆-Alkoxy, C₁-C₆-Alkoxy-C₁-C₆-alkyl, C₁-C₆-Alkylthio oder Halogen-C₁-C₆-alkyl steht,
R¹⁰ für Wasserstoff, Halogen, Hydroxy, C₁-C₆-Alkyl, C₁-C₆-Alkoxy, C₁-C₆-Alkoxy-C₁-C₆-alkyl, C₁-C₆-Alkylthio, C₁-C₆-Alkylthio-C₁-C₆-alkyl, Nitrilo-C₁-C₆-alkyl, C₁-C₆-Alkylcarbonyl, C₁-C₆-Alkoxycarbonyl, Aminocarbonyl, Formyl, C₂-C₆-Alkenyl, C₂-C₆-Alkinyl, Halogen-C₁-C₆-alkyl, Halogen-C₂-C₆-alkenyl, Halogen-C₂-C₆-alkinyl, Amino, Ci-C₄-Alkylamino, Di-Ci-C₄-alkylamino, durch Amino oder C₁-C₄-Alkylamino substituiertes C₁-C₄-Alkyl, gegebenenfalls durch Methyl, Ethyl und/oder Halogen substituiertes Cycloalkyl mit 3 bis 6 Kohlenstoffatomen, offenkettiges Acetal, cyclisches Acetal mit 5 oder 6 Ringgliedern, offenkettiges Thioacetal oder cyclisches Thioacetal mit 5 oder 6 Ringgliedern steht oder
R⁹ und R¹⁰ gemeinsam mit dem Kohlenstoffatom, an das sie gebunden sind, einen 5 oder 6-gliedrigen Ring bilden, der außer Kohlenstoff auch 1 oder 2 Sauerstoffatome und/oder Schwefelatome und/oder Stickstoffatome als Ringglieder und welcher eine Oxo-Gruppe enthalten kann und der durch Fluor, Chlor, Brom, lod, C₁-C₄-Alkyl, C₁-C₄-Alkoxy und/oder C₁-C₄-Alkoxy-C₁-C₄-alkyl substituiert sein kann und welcher eine oder mehrere, insbesondere eine bis zwei, Doppelbindungen enthalten kann oder
R⁸, R⁹ und R¹⁰ gemeinsam mit dem Kohlenstoffatom, an das sie gebunden sind, für einen gesättigten oder einfach ungesättigten Rest der Struktur 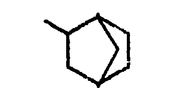 stehen,
E für Sauerstoff oder Schwefel steht und
R¹¹ und R¹² unabhängig voneinander für Wasserstoff, Halogen, C₁-C₆-Alkyl oder Halogen-C₁-C₆-alkyl stehen, mit der Maßgabe daß, wenn R¹ und R³ für Methyl, R² für Wasserstoff, A für Stickstoff, X für Sauerstoff und n für 0 stehen, Y und Z zusammen nicht für die Reste: 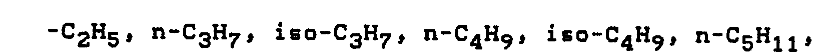 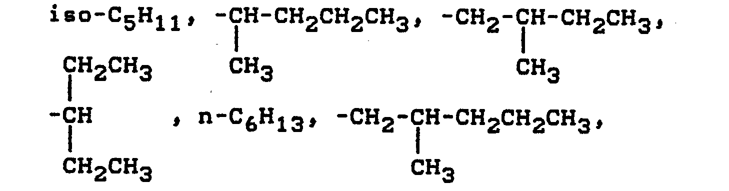 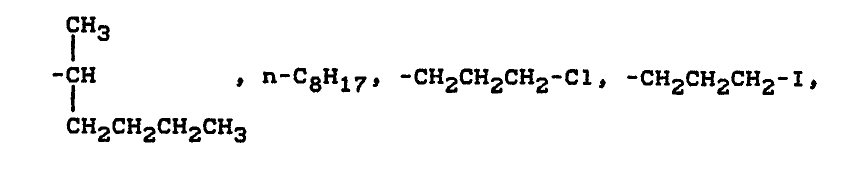 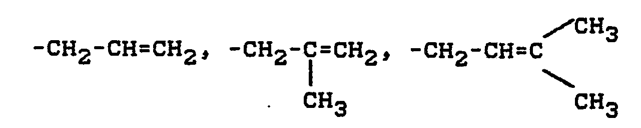 und 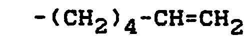 stehen.

3. 2-Pyri(mi)dylthio-arylether der Formel (I) gemäß Anspruch 1, in welcher
R¹ und R³ für Methyl stehen,
R² für Wasserstoff steht,
A für Stickstoff steht,
n für 0 steht,
X für Sauerstoff steht,
Y für die Gruppe. 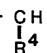 -steht, wobei
R⁴ für Wasserstoff, Methyl, Ethyl, n-oder iso-Propyl, n-, iso-, sec-oder tert.-Butyl, Methoxymethyl, Methoxyethyl, Ethoxymethyl oder Ethoxyethyl steht,
Z für die Gruppe 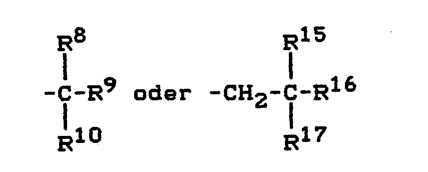 steht, wobei
R⁸ und R⁹ unabhängig voneinander für Methyl, Ethyl, n-oder iso-Propyl, n-, iso-, sec-oder tert.-Butyl stehen,
R¹⁰ für Methyl, Ethyl, n-oder iso-Propyl, n-iso-, sec-oder tert.-Butyl, Methoxy, Ethoxy, n-oder iso-Propoxy, n-, iso-, sec-oder tert.-Butoxy, Halogen-C₁-C₄-alkyl, C₁-C₄-Alkoxy-C₁-C₄-alkyl oder C₁-C₄-Alkylthio-C₁-C₄-alkyl steht und
R¹⁵, R¹⁶ und R¹⁷ unabhängig voneinander für Methyl, Ethyl, n-oder iso-Propyl, n-, iso-, sec-oder tert.-Butyl, Methoxy, Ethoxy, n-Propoxy oder iso-Propoxy stehen oder
R¹⁵ und R¹⁶ unabhängig voneinander für Wasserstoff, Methyl, Ethyl, n-oder iso-Propyl, n-, iso-,sec.-oder tert.-Butyl, Methoxy, Ethoxy, n-oder iso-Propoxy, n-, iso-, sec-oder tert.-Butoxy, Methylthio, Ethylthio, n-oder iso-Propylthio, n-, iso-, sec.-oder tert.-Butylthio und
R¹⁷ für Methoxy, Ethoxy, n-oder iso-Propoxy, n-, iso-, sec-oder tert.-Butoxy, Methylthio, Ethylthio, n-oder iso-Propylthio, n-, iso-, sec-oder tert.-Butylthio steht oder
R¹⁵ für Wasserstoff, Methyl, Ethyl, n-oder iso-Propyl, n-, iso-, sec-oder tert.-Butyl, Methoxy, Ethoxy, n-oder iso-Propoxy, Methylthio, Ethylthio, n-oder iso-Propylthio steht und
R¹⁶ und R¹⁷ zusammen mit dem Kohlenstoffatom, an das sie gebunden sind, für jeweils gegebenenfalls durch Methyl, Fluor und/oder Chlor substituiertes Cyclopropyl, Cyclopentyl oder Cyclohexyl stehen.

4. 2-Pryi(mi)dylthio-arylether der Formel (I) gemäß Anspruch 1, in welcher
R¹, R² und R3 unabhängig voneinander jeweils für Wasserstoff, Methyl oder Ethyl stehen, wobei jedoch R¹ und R³ nicht gleichzeitig für Methyl stehen, wenn R² für Wasserstoff, A für Stickstoff, X für Sauerstoff und n für 0 stehen,
A für eine -CH-Gruppe oder ein Stickstoffatom steht,
B für Halogen, Nitro, Cyano, Amino, Methyl, Ethyl, Methoxy, Ethoxy, Halogen-C₁-C₂-alkyl oder Halogen-C₁-C₂-alkoxy steht, wobei B gleich oder verschieden sein kann,
n für eine Zahl 0, 1 oder 2 steht,
X für Sauerstoff, Schwefel, Sulfoxy oder Sulfonyl steht,
Y für eine 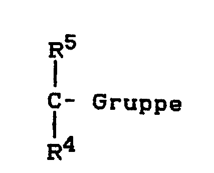 oder 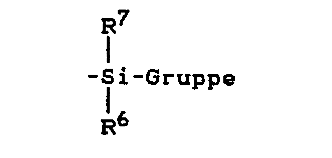 steht, wobei
R⁴ und R⁵ unabhängig voneinander jeweils für Wasserstoff, Methyl, Ethyl, Methoxymethyl, Methoxyethyl, Ethoxymethyl, Ethoxyethyl oder Halogen-C₁-C₂-alkyl stehen,
R⁶ und R⁷ jeweils für Methyl stehen,
Z für einen Rest 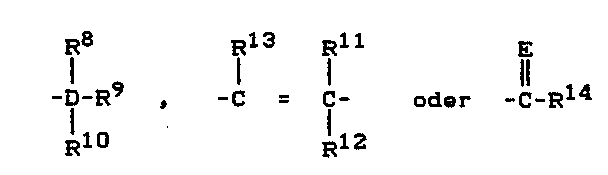 steht, wobei
D für Kohlenstoff oder Silicium steht,
R⁸, R¹³ und R¹⁴ unabängig voneinander für Wasserstoff, Fluor, Chlor, Brom, lod, C₁-C₄-Alkyl oder Halogen-C₁-C₄-alkyl steht,
R⁹ für Wasserstoff, Fluor, Chlor, Brom, lod, C₁-C₄-Alkyl, C₁-C₄-Alkoxy, C₁-C₄-Alkoxy-C₁-C₄-alkyl, C₁-C₄-Alkylthio oder Halogen-C₁-C₄-alkyl steht,
R¹⁰ für Wasserstoff, Fluor, Chlor, Brom, lod, Hydroxy, C₁-C₅-Alkyl, C₁-C₅-Alkoxy, C₁-C₄-Alkoxy-C₁-C₄-alkyl, C₁-C₄-Alkylthio-C₁-C₄-alkyl, Ci-C₄-Alkylthio, Nitrilo-C₁-C₄-alkyl, C₁-C₄-Alkylcarbonyl, Ci-C₄-Alkoxycarbonyl, Aminocarbonyl, Formyl, C₂-C₅-Alkenyl, C₂-C₄-Alkinyl, Halogen-C₁-C₄-alkyl, Halogen-C₂-C₄-alkenyl, Halogen-C₂-C₄- alkinyl, Amino, C₁-C₂-Alkylamino, Di-C₁-C₂-alkylamino, jeweils. gegebenenfalls durch Methyl, Fluor und/oder Chlor substituiertes Cyclopropyl, Cyclopentyl oder Cyclohexyl, offenkettiges Acetal, cyclisches Acetal mit 5 oder 6 Ringgliedern, offenkettiges Thioacetal oder cyclisches Thioacetal mit 5 oder 6 Ringgliedern steht oder
R⁹ und R¹⁰ gemeinsam mit dem Kohlenstoffatom, an das sie gebunden sind, einen 5 oder 6-gliedrigen Ring bilden, der außer Kohlenstoff auch 1 oder 2 Sauerstoffatome und/oder Schwefelatome und/oder Stickstoffatome als Ringglieder und der eine Oxo-Gruppe enthalten kann und der durch Fluor, Chlor, Brom, lod, Methyl, Ethyl, Methoxy, Ethoxy, Methoxymethyl, Methoxyethyl, Ethoxymethyl oder Ethoxyethyl substituiert sein kann und welcher eine oder zwei Doppelbindungen enthalten kann oder
R⁸, R⁹ und R¹⁰ gemeinsam mit dem Kohlenstoffatom, an das sie gebunden sind, für einen Rest der Struktur 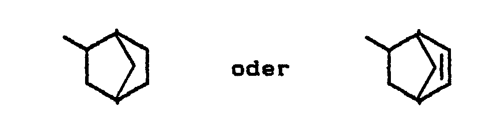 stehen,
E für Sauerstoff oder Schwefel steht und
R¹¹ und R¹² unabhängig voneinander für Wasserstoff, Halogen, C₁-C₂-Alkyl oder Halogen-C₁-C₂-alkyl stehen.

5. Verfahren zur Herstellung von 2-Pyri(mi)dylthioarylether der Formel (I), 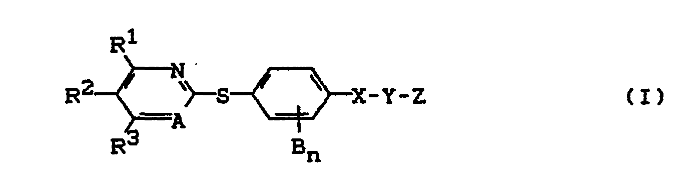 in welcher
R¹, R² und R³ unabhängig voneinander jeweils für Wasserstoff oder Alkyl stehen,
A für eine -CH-Gruppe oder ein Stickstoffatom steht,
B für Halogen, Nitro, Cyano, Amino, Alkyl, Alkoxy, Halogenalkyl oder Halogenalkoxy steht, wobei B gleich oder verschieden sein kann,
n für eine Zahl 0, 1, 2, 3 oder 4 steht,
X für Sauerstoff, Schwefel, Sulfoxy oder Sulfonyl steht,
Y für eine 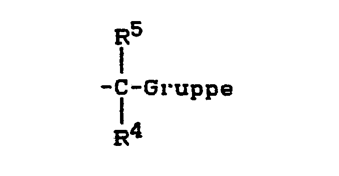 oder eine 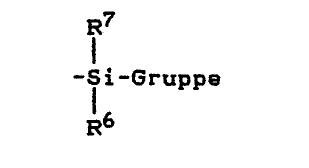 steht, wobei
R⁴ und R⁵ unabhängig voneinander jeweils für Wasserstoff, Alkyl, Alkoxyalkyl oder Halogenalkyl steht, R⁶ und R⁷ jeweils für Alkyl stehen,
Z für einen Rest 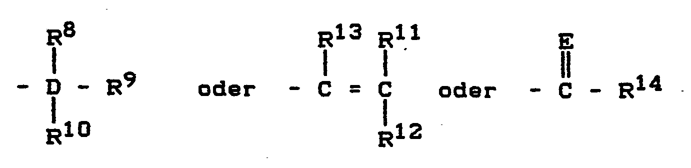 steht, wobei
D für Kohlenstoff oder Silicium steht,
R⁸, R¹3 und R¹⁴ unabhängig voneinander für Wasserstoff, Halogen, Alkyl oder Halogenalkyl stehen,
R⁹ für Wasserstoff, Halogen, Alkyl, Alkoxy, Alkoxyalkyl, Alkylthio oder Halogenalkyl steht,
R¹⁰ für Wasserstoff, Halogen, Hydroxy, Alkyl, Alkoxy, Alkoxyalkyl, Alkylthio, Alkylthioalkyl, Nitriloalkyl, Alkylcarbonyl, Alkoxycarbonyl, Aminocarbonyl, Formyl, Alkenyl, Alkinyl, Halogenalkyl, Halogenalkenyl, Halogenalkinyl, Amino, Alkylamino, Dialkylamino, durch Amino oder Alkylamino substituiertes Alkyl, gegebenenfalls durch Alkyl und/oder Halogen substituiertes Cycloalkyl, offenkettiges oder cyclisches Acetal oder offenkettiges oder cyclisches Thioacetal steht oder
R⁹ und R¹⁰ gemeinsam mit dem Kohlenstoffatom, an das sie gebunden sind, einen 3-8 gliedrigen Ring bilden, der außer Kohlenstoff auch Sauerstoff und/oder Schwefel und/oder Stickstoff als Ringglieder und welcher eine Oxo-Gruppe enthalten kann und der durch Halogen, Alkyl, Alkoxy und/oder Alkoxyalkyl substituiert sein kann und welcher eine oder mehrere Doppelbindungen enthalten kann oder
R⁸, R⁹ und R¹⁰ gemeinsam mit dem Kohlenstoffatom, an das sie gebunden sind, für einen gesättigten oder ungesättigten Rest der Struktur 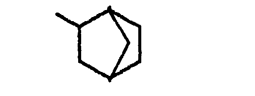 stehen,
E für Sauerstoff oder Schwefel steht und
R" und R¹² unabhängig voneinander für Wasserstoff, Halogen, Alkyl oder Halogenalkyl stehen,
mit der Maßgabe daß, wenn R¹ und R³ für Methyl, R² für Wasserstoff, A für Stickstoff, X für Sauerstoff und n für 0 stehen, Y und Z zusammen nicht für die Reste:
-C₂H₅, n-C₃H₇, iso-C₃H₇, n-C₄H₉, iso-C₄Hg, n-C₅H₁₁, 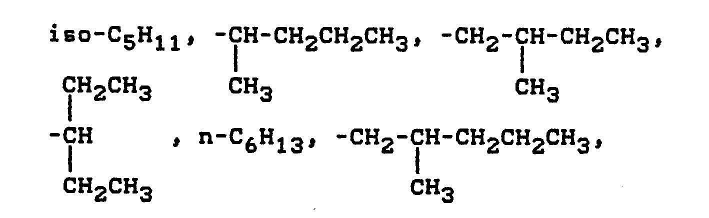 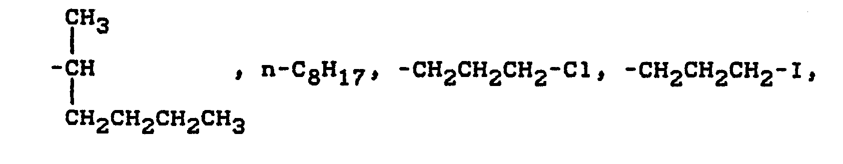 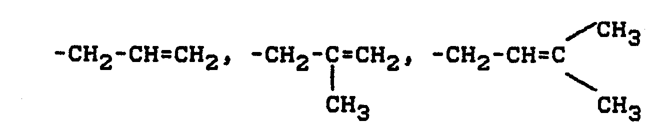 und 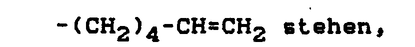 dadurch gekennzeichnet, daß man
A) Thiophenol-Derivate der Formel (II), 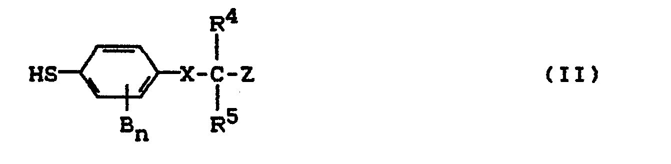 in welcher
R⁴, R⁵, B, X, Z und n die oben angegebene Bedeutung haben,
mit Pyri(mi)dyl-Derivaten der Formel (III), 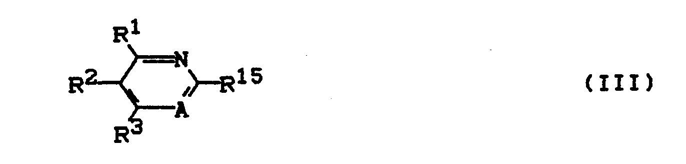 in welcher
R¹, R², R³ und A die oben angegebene Bedeutung haben und
R¹⁵ für Halogen, C₁-C₄-Alkylsulfonyl, Phenylsulfonyl oder substituiertes Phenylsulfonyl steht,
in Gegenwart eines Verdünnungsmittels und gegebenenfalls im Gegenwart eines Säurebindemittels umsetzt,
oder daß man
B) Pyri(mi)dylthio-(thio)phenole der Formel (IV), 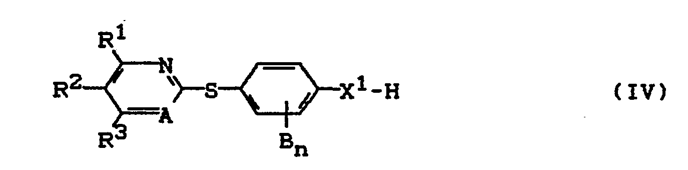 in welcher
A, B, R¹, R², R³ und n die oben angegebene Bedeutung haben und
X¹ für Sauerstoff oder Schwefel steht,
mit Verbindungen der Formel (V),
G-Y-Z (V)
in welcher
Y und Z die oben angegebene Bedeutung haben und
G für Halogen, Ci-C₄-Alkylsulfonyloxy, Haiogen-C₁-C₄-alkylsulfonyloxy, Phenylsulfonyloxy oder substituiertes Phenylsulfonyloxy steht,
in Gegenwart eines Verdünnungsmittels und gegebenenfalls in Gegenwart eines Säurebindemittels umsetzt, oder daß man
C) Verbindungen der Formel (VI) 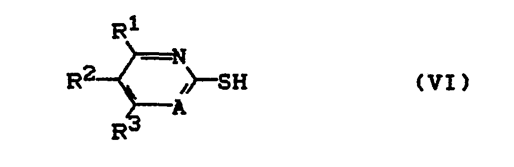 in welcher
R¹, R², R³ und A die oben angegebene Bedeutung haben,
mit Verbindungen der Formel (VII) 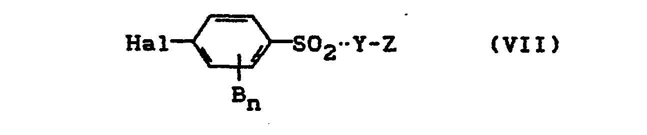 in welcher
B, Y, Z und n die oben angegebene Bedeutung haben,
Hal für Halogen steht,
in Gegenwart eines Verdünnungsmittels und gegebenenfalls in Gegenwart eines Säurebindemittels umsetzt.

6. Herbizide und pflanzenwuchsregulierende Mittel, gekennzeichnet durch einen Gehalt an mindestens einem 2-Pyri(mi)dylthio-arylether der Formel (I) gemäß den Ansprüchen 1 bis 5.

7. Verfahren zur Bekämpfung von Unkräutern, dadurch gekennzeichnet, daß man 2-Pyri(mi)dylthio- arylether der Formel (I) gemäß den Ansprüchen 1 bis 5 auf die Unkräuter und/oder ihren Lebensraum einwirken läßt.

8. Verwendung der 2-Pyri(mi)dylthio-arylether der Formel (I) gemäß den Ansprüchen 1 bis 5 zur Bekämpfung von Unkräutern und/oder als Pflanzenwachstumsregulatoren.

9. Thiophenol-Derivate der Formel (Ila), 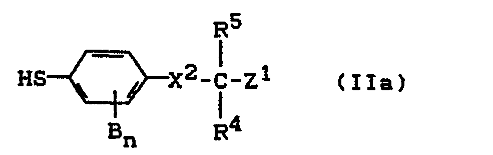
in welcher
R⁴ und R⁵ unabhängig voneinander für Wasserstoff, Alkyl, Alkoxyalkyl oder Halogenalkyl stehen, X² für Sauerstoff oder Schwefel steht,
Z¹ für die Gruppe 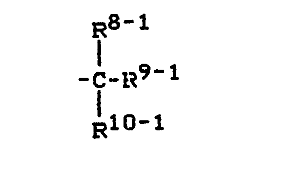 steht, wobei
R⁸⁻¹ für Alkyl steht,
R⁹⁻¹ für Alkyl, Alkoxy, Alkoxyalkyl, Alkylthio oder Halogenalkyl steht und
R¹⁰⁻¹ für Alkyl steht,
B für Halogen, Nitro, Cyano, Amino, Alkyl, Alkoxy, Halogenalkyl oder Halogenalkoxy steht, wobei B gleich oder verschieden sein kann, und
n für eine Zahl 0. 1, 2, 3 oder 4 steht.

10. Verbindungen der Formel (IVa) 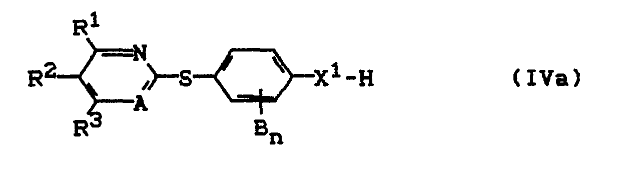 in welcher
R¹, R² und R³ unabhängig voneinander jeweils für Wasserstoff oder Alkyl stehen,
A für eine -CH-Gruppe oder ein Stickstoffatom steht,
B für Halogen, Nitro, Cyano, Amino, Alkyl, Alkoxy, Halogenalkyl oder Halogenalkoxy steht, wobei B gleich oder verschieden sein kann, und
n für eine Zahl 0, 1, 2, 3 oder 4 steht,
X¹ für Sauerstoff oder Schwefel steht,
wobei jedoch R¹, R² und R³ nicht gleichzeitig für Wasserstoff steht, wenn A für die -CH-Gruppe steht und wobei die Verbindung 4-(4,6-Dimethyl-2-pyrimidylthio)-phenol ausgenommen ist.

11. Die Verbindung der Formel
